# FASCICULE DE BREVET EUROPEEN

(11) **EP 0 962 220 B1**
(45) Date de publication et mention de la délivrance du brevet: **08.03.2006**
(21) Numéro de dépôt: 99401100.5
(22) Date de dépôt: 05.05.1999
(51) Int. Cl.: A61K 8/34, A61K 8/49, A61Q 5/10

(54) **Composition de teinture pour fibres kératiniques avec un colorant direct cationique et un polyol ou ether de polyol**
Färbezusammensetzung für keratinische Fäser mit einem direktziehenden kationischen Farbstoff und einem Polyol oder einem Polyolether
Dying composition for keratinous fibres with a direct cationic dye and a polyol or a polyolether

(30) Priorité: 28.05.1998 FR 9806752
(43) Date de publication de la demande: 08.12.1999
(73) Titulaire: L'OREAL, 75008 Paris (FR)
(72) Inventeur: Lang, Gérard, 95390 Saint Prix (FR); Cotteret, Jean, 78480 Verneuil/Seine (FR)
(74) Mandataire: Miszputen, Laurent

(56) Documents cités:
- US-A- 3 985 499

## Description

L'invention concerne une composition de teinture pour fibres kératiniques, en particulier pour fibres kératiniques humaines telles que les cheveux, comprenant, dans un milieu approprié pour la teinture, au moins un colorant direct cationique de formule donnée, et au moins de la glycérine et/ou un polyol particulier et/ou un éther de polyol particulier.

L'invention a également pour objets les procédés et dispositifs de teinture mettant en oeuvre ladite composition.

Dans le domaine capillaire, on peut distinguer deux types de coloration.

Le premier est la coloration semi-permanente ou temporaire, ou coloration directe, qui fait appel à des colorants capables d'apporter à la coloration naturelle des cheveux, une modification de couleur plus ou moins marquée résistant éventuellement à plusieurs shampooings. Ces colorants sont appelés colorants directs; ils peuvent être mis en oeuvre avec ou sans agent oxydant. En présence d'oxydant, le but est d'obtenir une coloration éclaircissante. La coloration éclaircissante est mise en oeuvre en appliquant sur les cheveux le mélange extemporané d'un colorant direct et d'un oxydant et permet notamment d'obtenir, par éclaircissement de la mélanine des cheveux, un effet avantageux tel qu'une couleur unie dans le cas des cheveux gris ou de faire ressortir la couleur dans le cas de cheveux naturellement pigmentés.
Le deuxième est la coloration permanente ou coloration d'oxydation. Celle-ci est réalisée avec des colorants dits "d'oxydation" comprenant les précurseurs de coloration d'oxydation et les coupleurs. Les précurseurs de coloration d'oxydation, appelés couramment "bases d'oxydation", sont des composés initialement incolores ou faiblement colorés qui développent leur pouvoir tinctorial au sein du cheveu en présence d'agents oxydants ajoutés au moment de l'emploi, en conduisant à la formation de composés colorés et colorants. La formation de ces composés colorés et colorants résulte, soit d'une condensation oxydative des "bases d'oxydation" sur elles-mêmes, soit d'une condensation oxydative des "bases d'oxydation" sur des composés modificateurs de coloration appelés couramment "coupleurs" et généralement présents dans les compositions tinctoriales utilisées en teinture d'oxydation.
Pour varier les nuances obtenues avec lesdits colorants d'oxydation, ou les enrichir de reflets, Il arrive qu'on leur ajoute des colorants directs.

Parmi les colorants directs cationiques disponibles dans le domaine de la teinture des fibres kératiniques notamment humaines, on connaît déjà les composés dont la structure est développée dans le texte qui va suivre; néanmoins, ces colorants conduisent à des colorations qui présentent des caractéristiques encore insuffisantes, à la fois sur le plan de l'homogénéité de la couleur répartie le long de la fibre (« unisson »), on dit alors que la coloration est trop sélective, que sur le plan de la tenacité, en terme de résistance aux diverses agressions que peuvent subir les cheveux (lumière, intempéries,shampooings).

Or, après d'importantes recherches menées sur la question, la demanderesse vient maintenant de découvrir qu'il est possible d'obtenir de nouvelles compositions pour la teinture des fibres kératiniques capables de conduire à des colorations résistant mieux aux diverses agressions que peuvent subir les cheveux, en associant au moins de la glycérine et/ou un polyol particulier et/ou un éther de polyol particulier à au moins un colorant direct cationique connu de l'art antérieur et de formule (I) définie ci-après.

Cette découverte est à la base de la présente invention.

La présente invention a donc pour premier objet une composition pour la teinture des fibres kératiniques et en particulier des fibres kératiniques humaines telles que les cheveux, renfermant dans un milieu approprié pour la teinture, (i)au moins un colorant direct cationique dont la structure répond à la formule (I) suivante, caractérisée par le fait qu'elle contient en outre (ii)au moins de la glycérine et/ou un polyol particulier et/ou un éther de polyol particulier.
(i) Le colorant direct cationique utilisable selon la présente invention est un composé de formule (I) suivante :

A―N=N―B (I)

dans laquelle :
**le symbole A** représente un groupement choisi parmi les structures A1 à A3 suivantes : 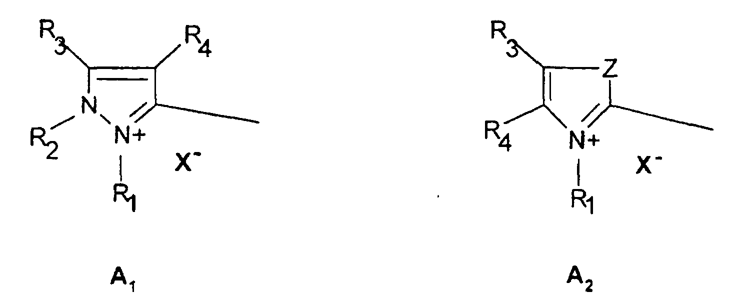 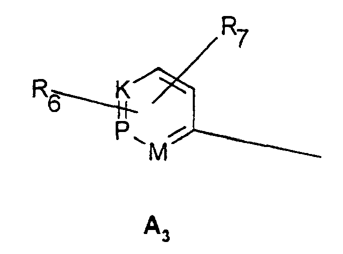
   structures A1 à A3 dans lesquelles,
   R₁ désigne un radical alkyle en C₁-C₄, un radical phényle pouvant être substitué par un radical alkyle en C₁-C₄ ou un atome d'halogène choisi parmi le chlore, le brome, l'iode et le fluor ;
   R₂ désigne un radical alkyle en C₁-C₄ ou un radical phényle;
   R₃ et R₄, identiques ou différents, représentent un radical alkyle en C₁-C₄, un radical phényle, ou bien, dans le cas de la structure A1, peuvent former ensemble un cycle benzénique substitué, et dans le cas de la structure A2, peuvent former ensemble un cycle benzénique éventuellement substitué par un ou plusieurs radicaux alkyle en C₁-C₄, alcoxy en C₁-C₄, ou NO₂;
   R₃ peut en outre désigner un atome d'hydrogène;
   Z désigne un atome d'oxygène, de soufre ou un groupement -NR₂;
   M représente un groupement -CH, -CR (R désignant alkyle en C₁-C₄),
   ou -NR₅(X⁻)ᵣ;
   K représente un groupement -CH, -CR (R désignant alkyle en C₁-C₄),
   ou -NR₅(X⁻)r;
   P représente un groupement -CH, -CR (R désignant alkyle en C₁-C₄),
   ou -NR₅(X⁻)ᵣ; r désigne zéro ou 1;
   R₅ représente un atome O⁻, un radical alcoxy en C₁-C₄, ou un radical alkyle en C₁-C₄;
   R₆ et R₇, identiques ou différents, représentent un atome d'hydrogène ou d'halogène choisi parmi le chlore, le brome, l'iode et le fluor, un radical alkyle en C₁-C₄, alcoxy en C₁-C₄, un radical -NO₂;
   X⁻ représente un anion de préférence choisi parmi le chlorure, l'iodure, le méthyl sulfate, l'éthyl sulfate, l'acétate et le perchlorate;
   sous réserve que,
   si R₄ désigne un radical alkyle en C₁-C₄ et Z désigne un atome de soufre, R₃ ne désigne pas un atome d'hydrogène;
   si R₅ désigne O⁻, alors r désigne zéro;
   si K ou P ou M désignent -N-alkyle C₁-C₄ X⁻ , alors R₆ ou R₇ est différent d'un atome d'hydrogène;
   si K désigne -NR₅(X⁻)ᵣ, alors M= P= -CH; -CR;
   si M désigne -NR₅(X⁻)ᵣ, alors K= P= -CH; -CR;
   si P désigne -NR₅(X⁻)ᵣ, alors K= M et désignent -CH ou -CR;
   si Z désigne -NR₂ et R₂ désigne un radical alkyle en C₁-C₄, alors au moins un des radicaux R₁, R₃ ou R₄ de **A**_{**2**} est différent d'un radical alkyle en C₁-C₄;
**le symbole B** représente :
   - **(a)** un groupement de structure B1 suivante : 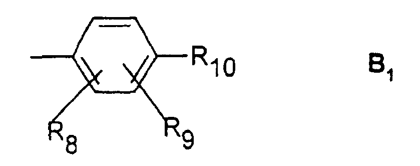 structure B1 dans laquelle,
      R₈ représente un atome d'hydrogène, un atome d'halogène choisi parmi le chlore, le brome, l'iode et le fluor, un radical alkyle en C₁-C₄, alcoxy en C₁-C₄, un radical - OH, -NO₂, -NHR₁₁, -NR₁₂R₁₃, -NHCOalkyle en C₁-C₄, ou forme avec R₉ un cycle à 5 ou 6 chaînons contenant ou non un ou plusieurs hétéroatomes choisis parmi l'azote, l'oxygène ou le soufre;
      R₉ représente un atome d'hydrogène, un atome d'halogène choisi parmi le chlore, le brome, l'iode et le fluor, un radical alkyle en C₁-C₄, alcoxy en C₁-C₄,

   ou forme avec R₁₀ ou R₁₁ un cycle à 5 ou 6 chaînons contenant ou non un ou plusieurs hétéroatomes choisis parmi l'azote, l'oxygène ou le soufre;
   R₁₀ représente un atome d'hydrogène, un radical -OH, un radical -NHR₁₁, un radical -NR₁₂R₁₃;
   R₁₁ représente un atome d'hydrogène, un radical alkyle en C₁-C₄, un radical monohydroxyalkyle en C₁-C₄, polyhydroxyalkyle en C₂-C₄, un radical phényle;
   R₁₂ et R₁₃, identiques ou différents, représentent un radical alkyle en C₁-C₄ , un radical monohydroxyalkyle en C₁-C₄, polyhydroxyalkyle en C₂-C₄;
   - **(b)** un groupement hétérocyclique azoté à 5 ou 6 chaînons susceptible de renfermer d'autres hétéroatomes et/ou des groupements carbonylés et pouvant être substitué par un ou plusieurs radicaux alkyle en C₁-C₄, amino ou phényle,
      et notamment un groupement de structure B2 suivante : 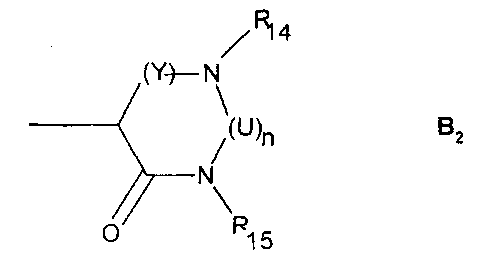
      structure B2 dans laquelle,
      R₁₄ et R₁₅, identiques ou différents, représentent un atome d'hydrogéne, un radical alkyle en C₁-C₄ un radical phényle;
      Y désigne le radical -CO- ou le radical 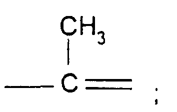
      n = 0 ou 1, avec, lorsque n désigne 1, U désigne le radical -CO- .

Dans les structures définies ci-dessus le groupement alkyle ou alcoxy en C₁-C₄ désigne de préférence méthyle, éthyle, butyle, méthoxy, éthoxy.

Les colorants directs cationiques de formule (I) utilisables dans les compositions tinctoriales conformes à l'invention, sont des composés connus et sont décrits par exemple dans les demandes de brevets FR-2189006, FR-2285851 et FR-2140205 et ses certificats d'addition.

Parmi les colorants directs cationiques de formule (I) utilisables dans les compositions tinctoriales conformes à l'invention, ceux de formule (I) dans lesquelles le symbole A désigne la structure A3 tandis que le symbole B désigne la structure B1 ou B2 sont particulièrement préférés.
Parmi ces composés, on peut notamment citer plus particulièrement les composés de structures (I)₁ à (I)₇₇ suivantes :

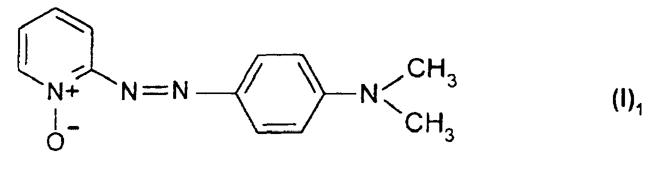

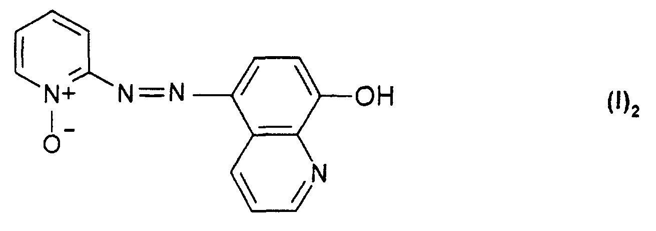

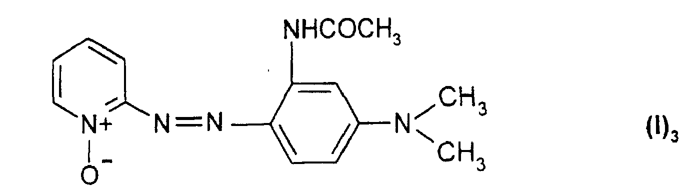

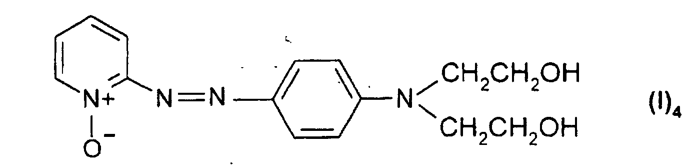

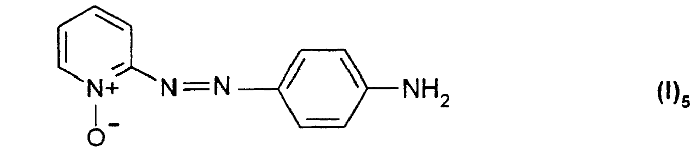

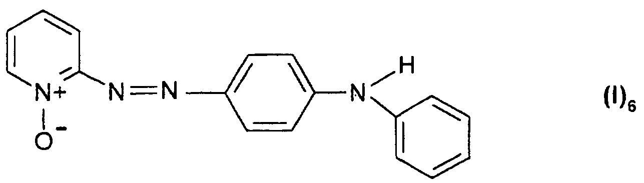

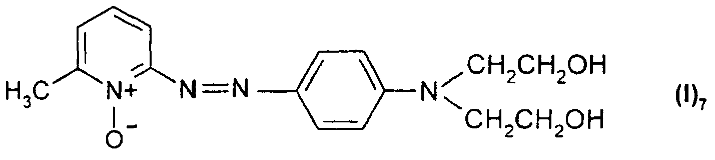

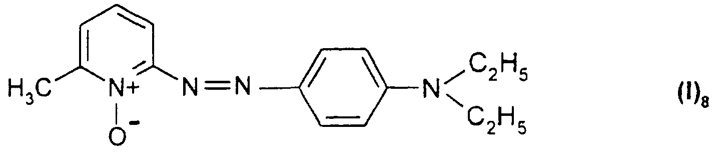

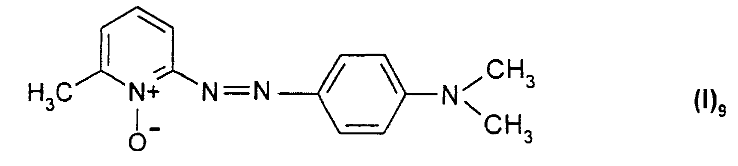

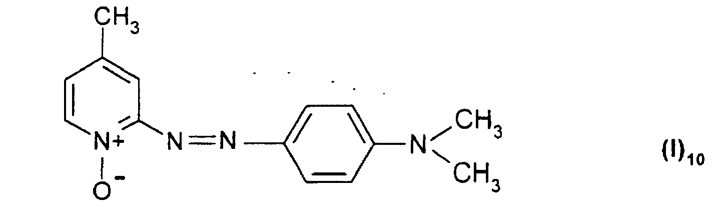

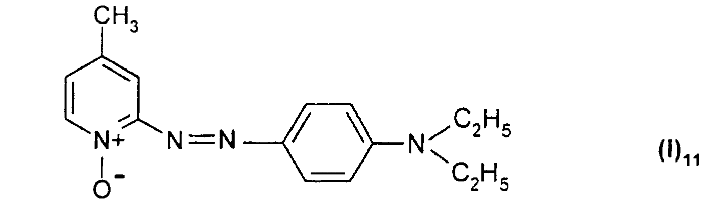

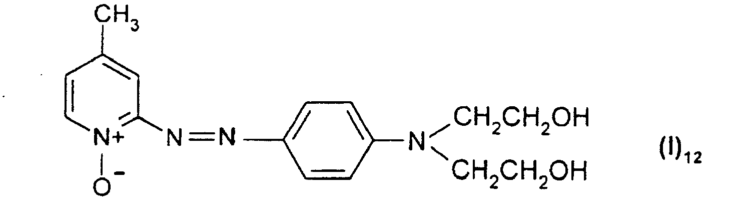

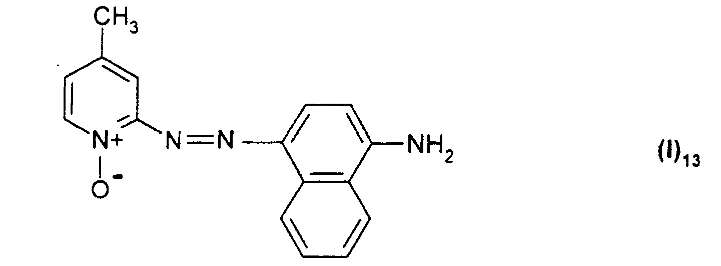

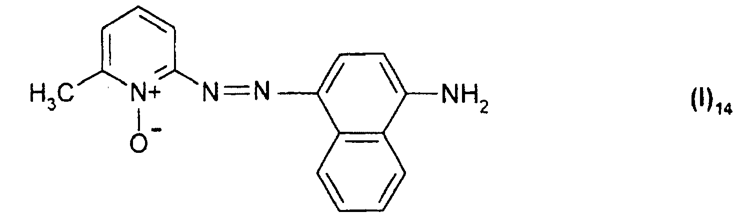

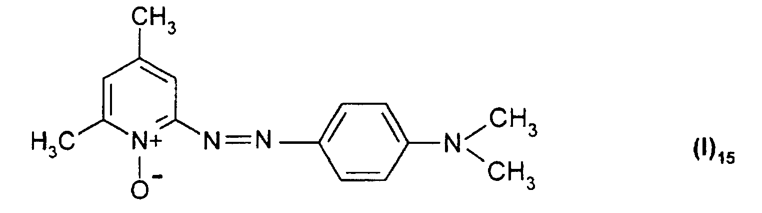

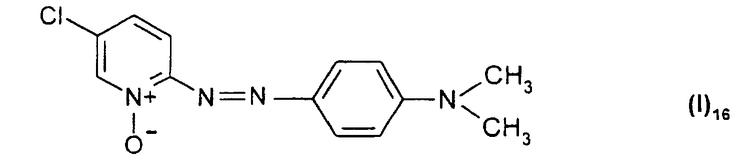

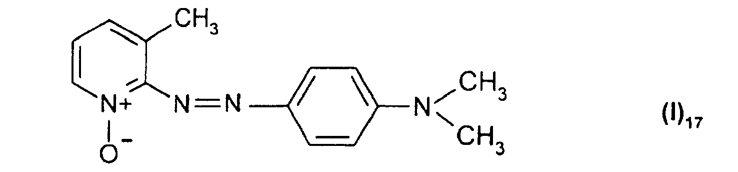

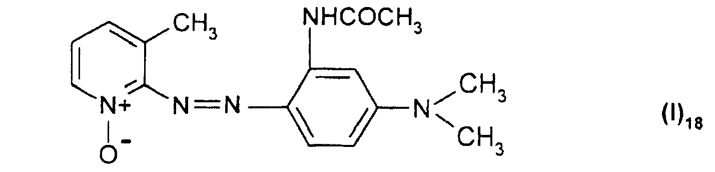

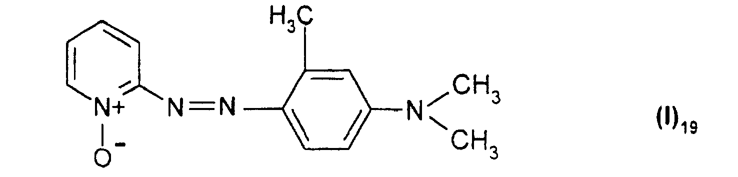

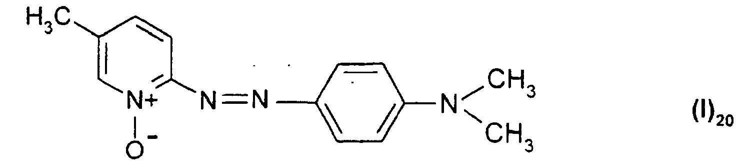

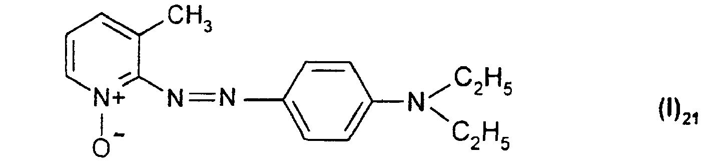

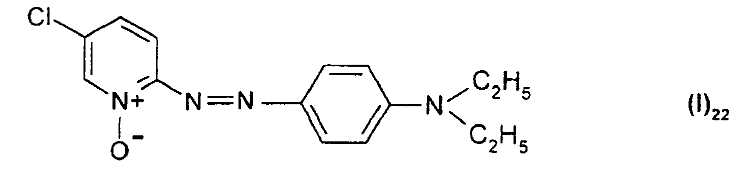

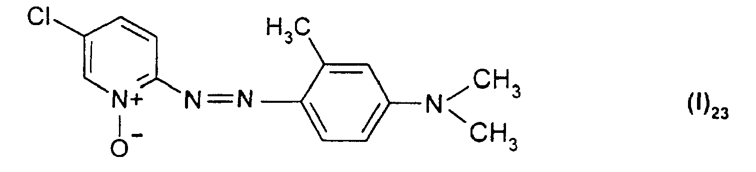

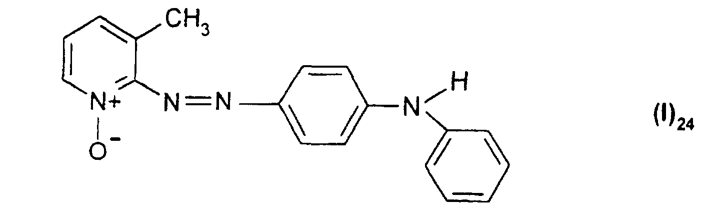

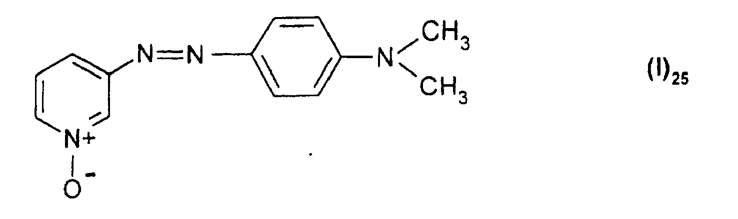

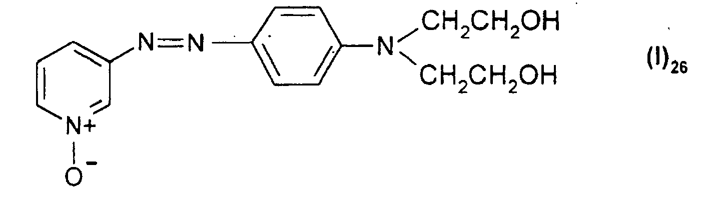

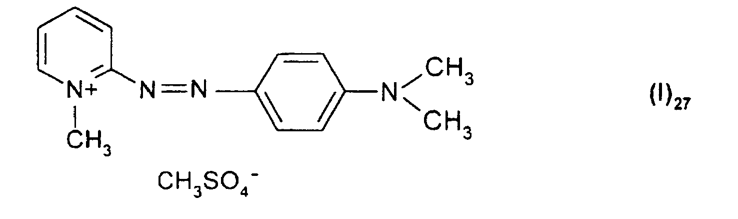

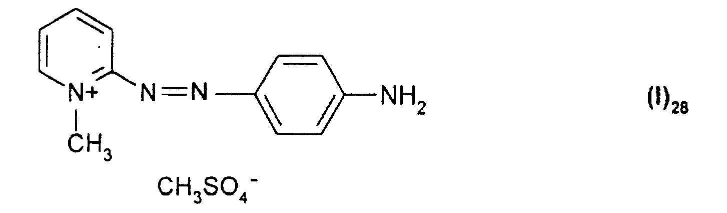

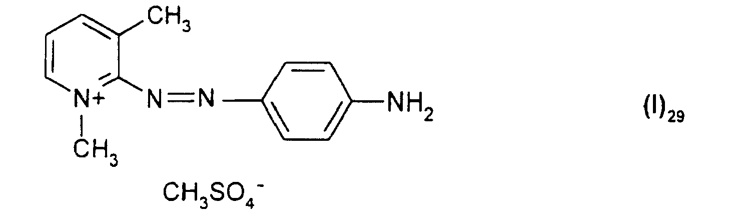

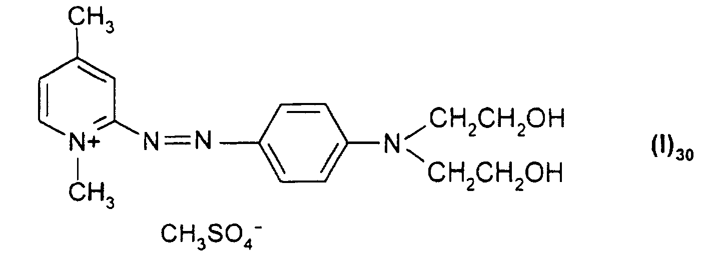

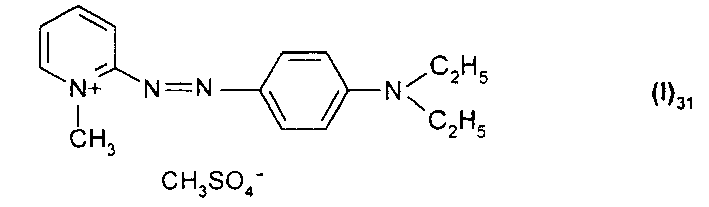

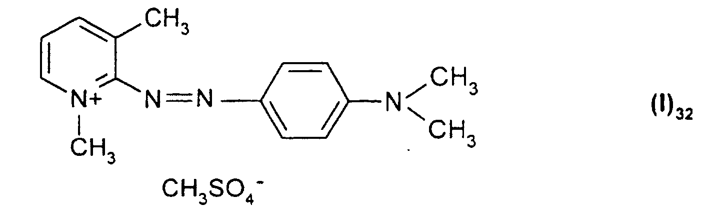

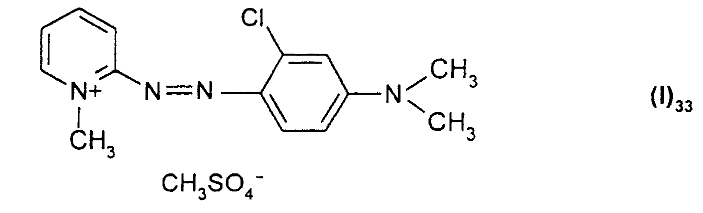

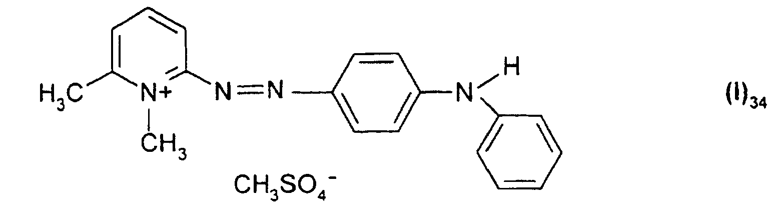

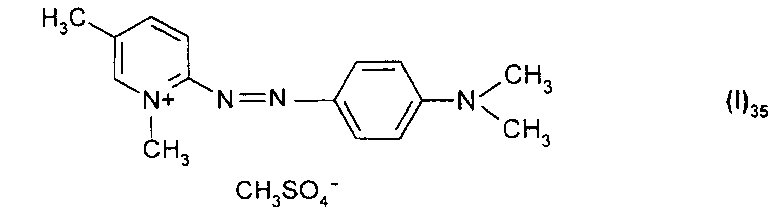

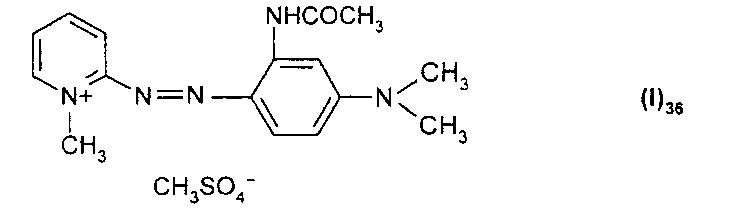

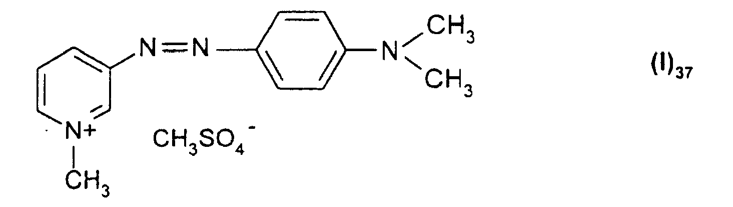

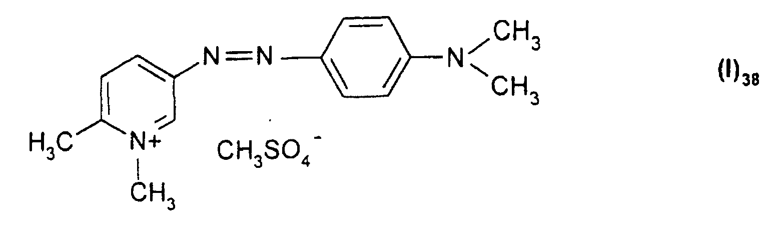

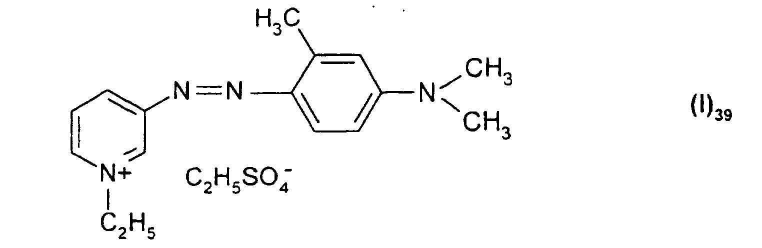

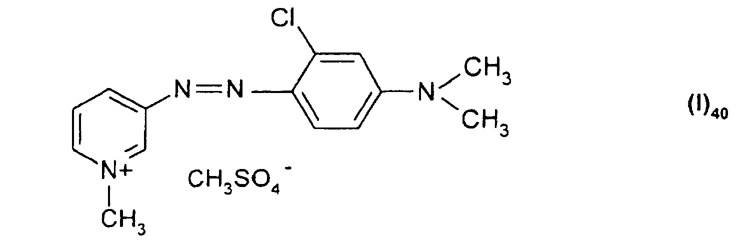

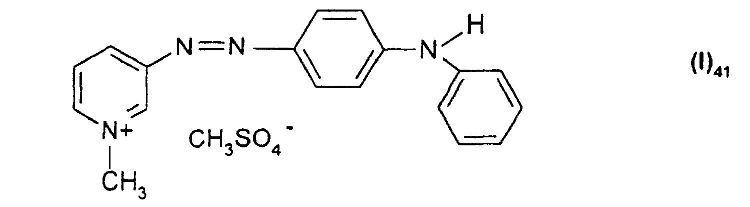

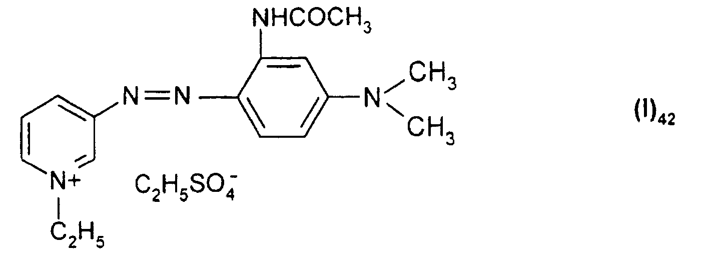

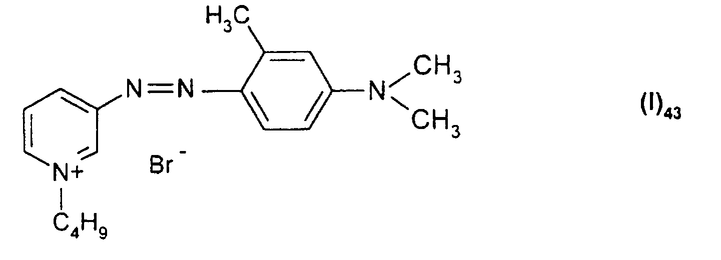

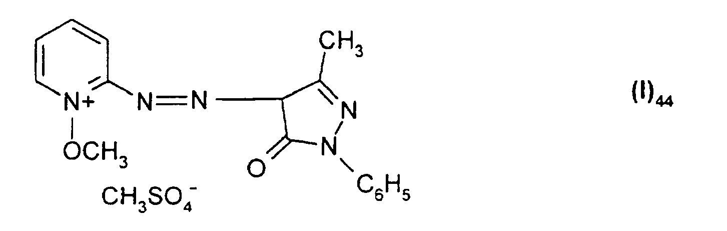

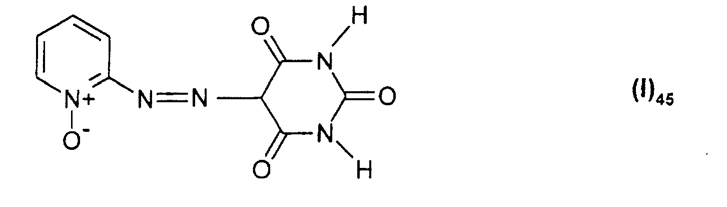

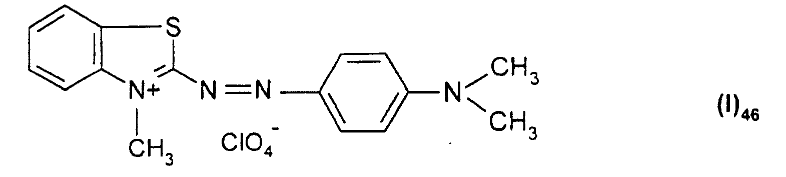

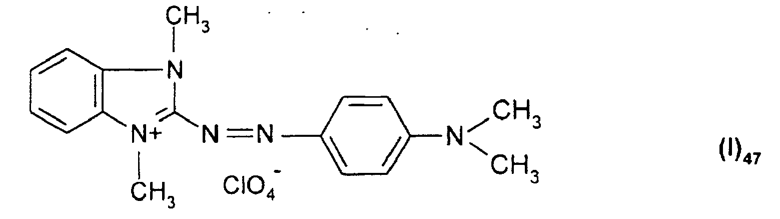

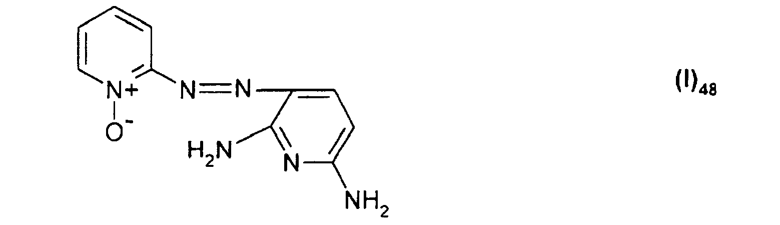

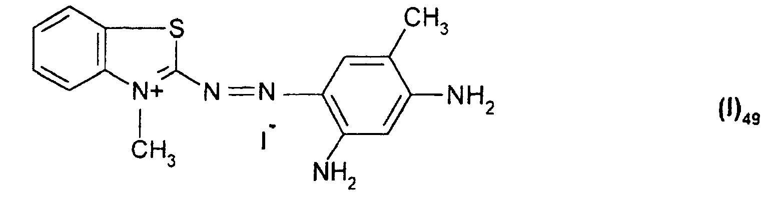

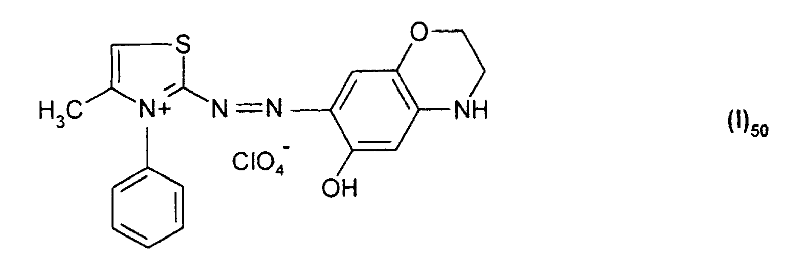

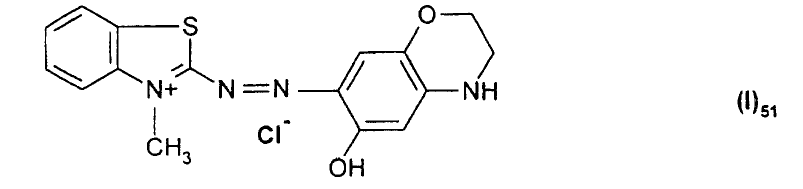

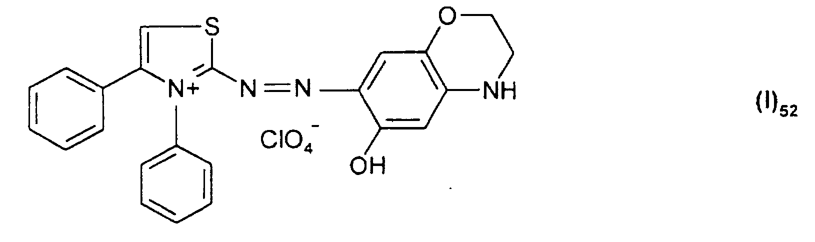

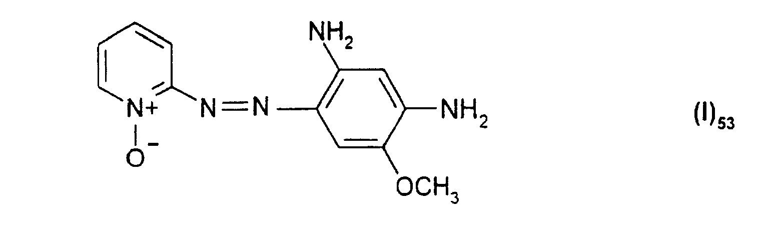

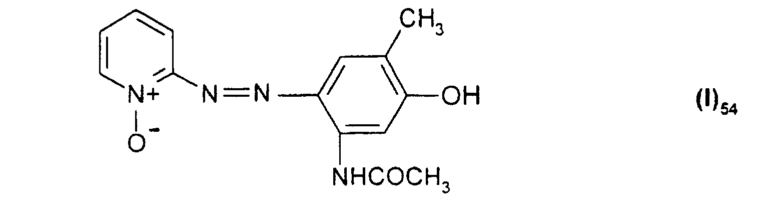

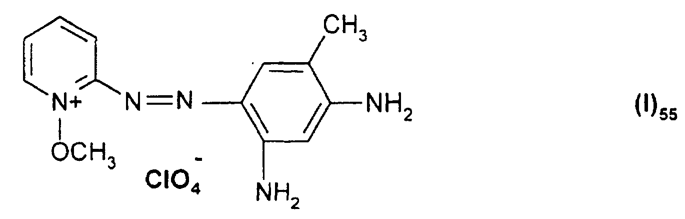

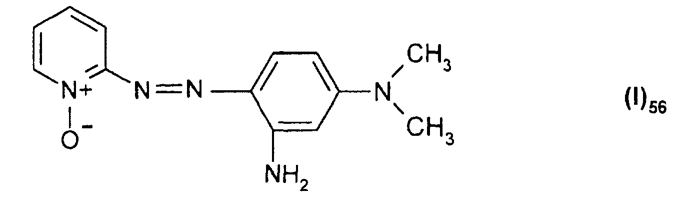

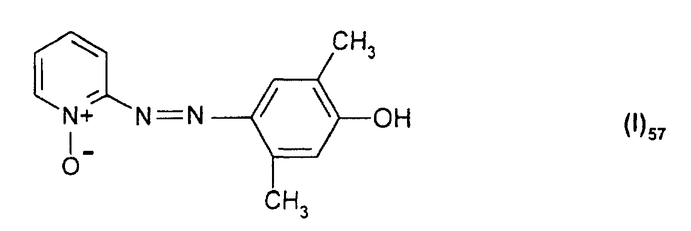

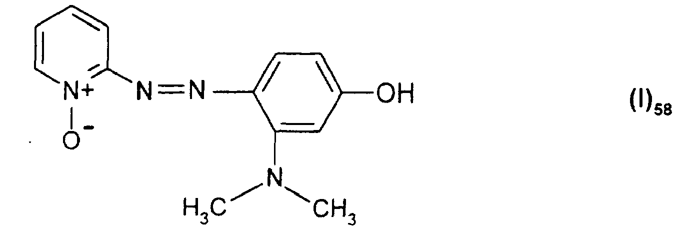

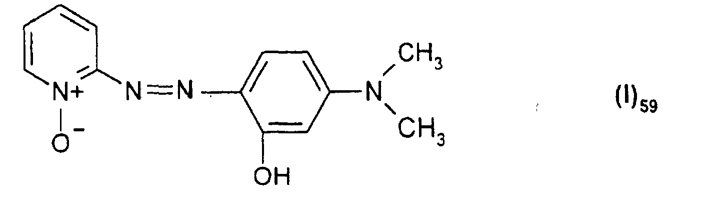

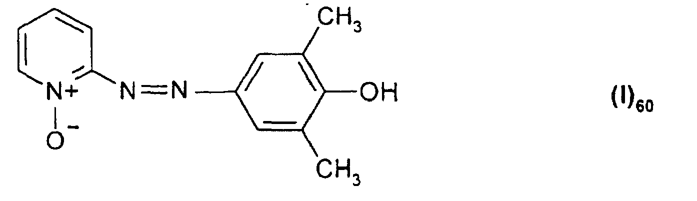

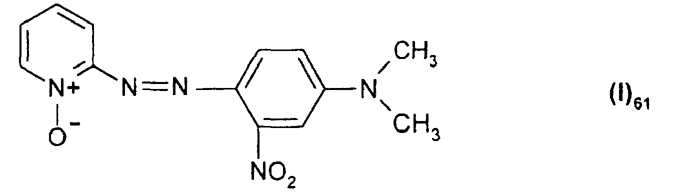

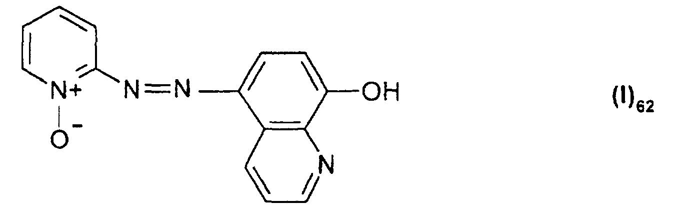

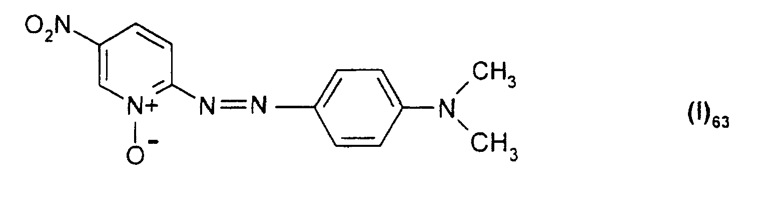

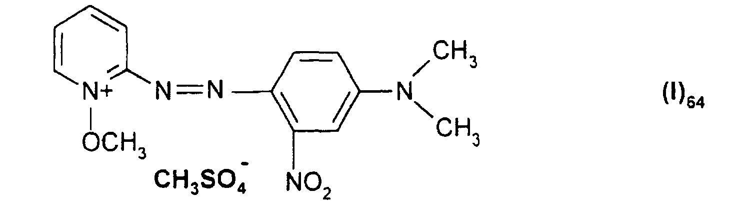

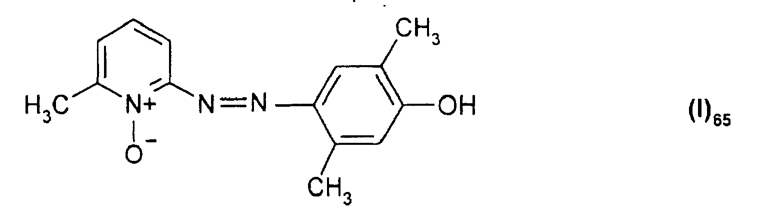

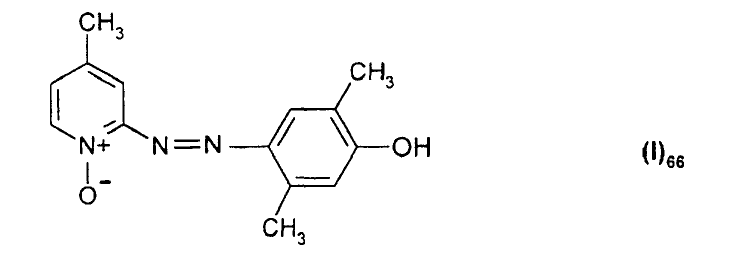

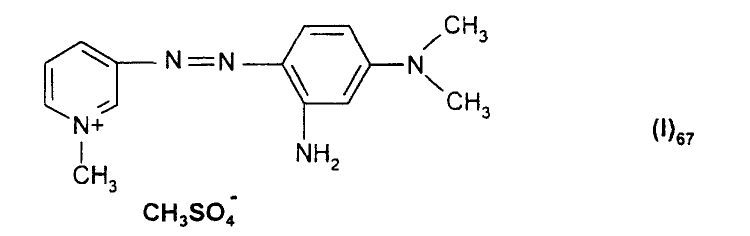

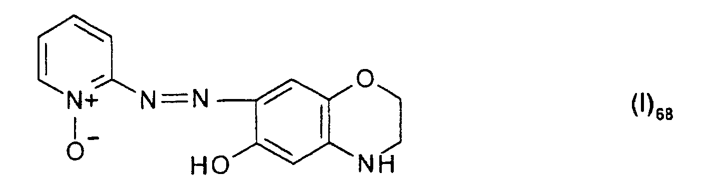

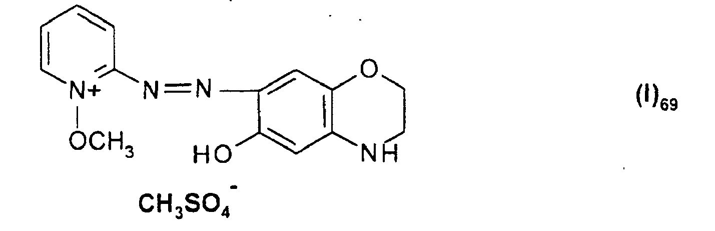

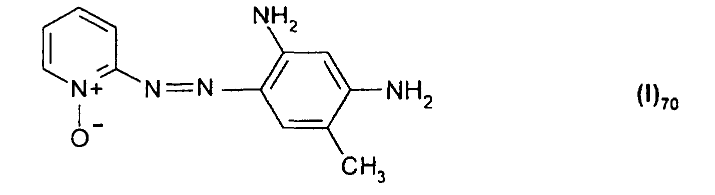

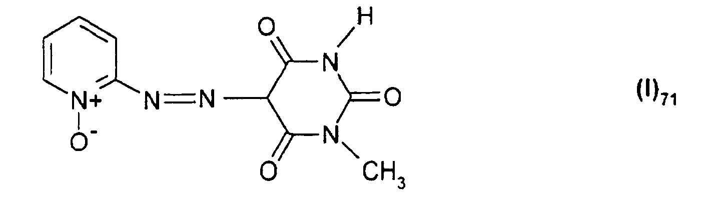

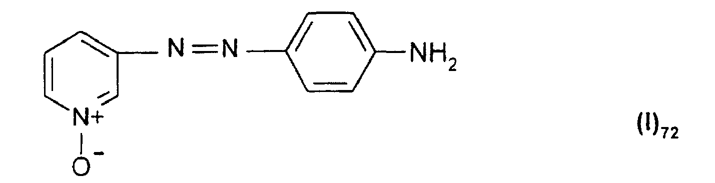

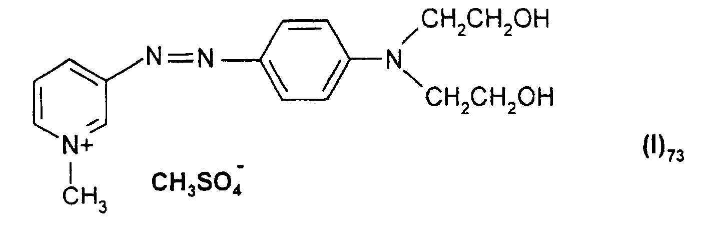

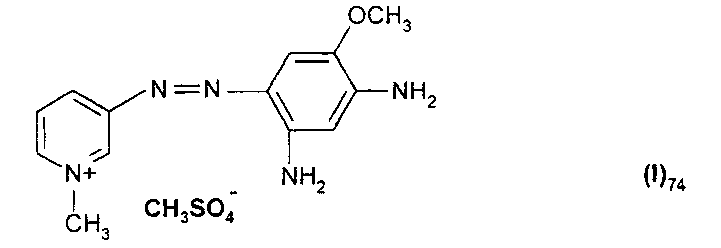

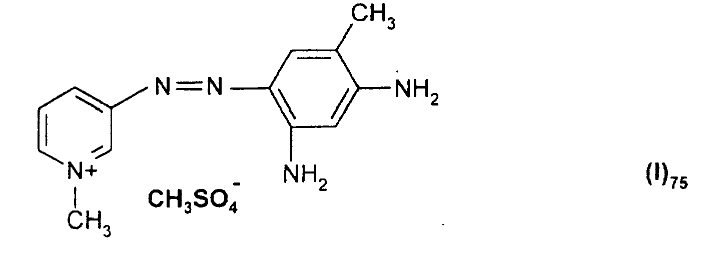

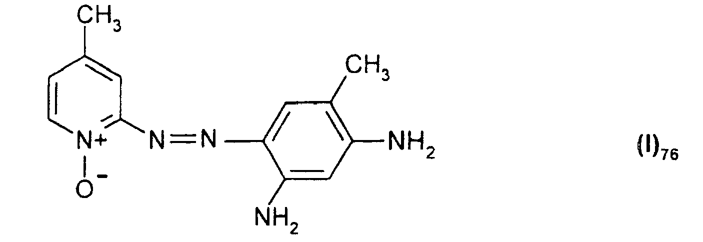

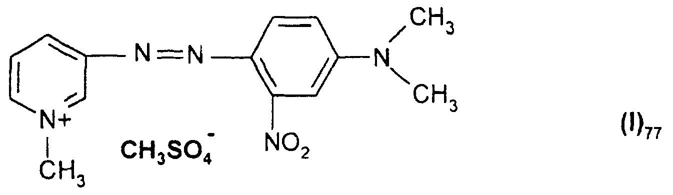

Le ou les colorants directs cationiques utilisés selon l'invention, représentent de préférence de 0,001 à 10 % en poids environ du poids total de la composition tinctoriale et encore plus préférentiellement de 0,005 à 5 % en poids environ de ce poids.

(ii) Par polyol, on désigne au sens de l'invention, un composé de type alkyle, linéaire, ramifié ou cyclique, saturé ou insaturé, portant au moins deux fonctions - OH sur la chaîne alkyle, ainsi que les polymères (polyéthers) de ces composés alkyle polyhydroxylés.

Les polyols particuliers utilisés selon l'invention contiennent au moins 4 atomes de carbone et peuvent être choisis notamment parmi les polyols en C₄-C₉ ainsi que les polyalkylèneglycols tels que plus particulièrement les polyéthylèneglycols et les polypropylèneglycols.
Parmi les polyols en C₄-C₉, on peut citer notamment le 2-butène-1,4-diol, le pentane-1,5-diol, le 2,2-diméthyl-propane-1,3-diol, le 3-méthyl-pentane-1,5-diol, le pentane-1,2-diol, le 2,2,4-triméthyl-pentane-1,3-diol, le 2-méthyl-propane-1,3-diol, l'hexylèneglycol, le 1,3-butylèneglycol, le dipropylèneglycol, le diéthylèneglycol, le triéthylèneglycol.

Les éthers de polyols particuliers selon l'invention sont choisis parmi les éthers aliphatiques en C₁-C₈ de polyols en C₃-C₉ et les éthers aromatiques en C₆-C₈ de polyols en C₂-C₉.
Parmi les éthers aliphatiques en C₁-C₈ de polyols en C₃-C₉, on peut citer notamment le monométhyléther de propylèneglycol, le monoéthyléther de propylèneglycol, le diméthyléther d'isopropylèneglycol, le monométhyléther et le monoéthyléther du diéthylèneglycol, le monométhyléther de dipropylèneglycol, le monométhyléther de tripropylèneglycol et le diméthyléther de diéthylèneglycol ; parmi les éthers aromatiques en C₆-C₈ de polyols en C₂-C₉, on peut citer notamment le monophényléther d'éthylèneglycol ou le monobenzyléther d'éthylèneglycol, le monophényléther de propylèneglycol, le monobenzyléther de propylèneglycol, le monophényléther de diéthylèneglycol et le monobenzyléther de diéthylèneglycol.

La glycérine et/ou le ou les polyols et/ou éthers de polyols décrits au sens de l'invention sont présents dans la composition tinctoriale conforme à l'invention dans des proportions généralement comprises entre 0,1 et 40% en poids, et encore plus particulièrement de 0,5 à 20% en poids parrapport au poids total de la composition.

Le milieu approprié pour la teinture (ou support) est généralement constitué par un mélange d'eau et d'au moins de la glycérine et/ou un polyol et/ou un éther de polyol tels que définis ci-dessus. Il peut contenir en outre un ou des solvants organiques différents de la glycérine et/ou du ou des polyols et/ou du ou des éthers de polyols utilisés conformément à l'invention, pour solubiliser les composés qui ne seraient pas suffisamment solubles dans le milieu. A titre de solvant organique, on peut par exemple citer les alcanols en C₁-C₄, tels que l'éthanol et l'isopropanol ainsi que les alcools aromatiques comme l'alcool benzylique, les produits analogues et leurs mélanges.

Lesdits solvants organiques additionnels peuvent être présents dans des proportions de préférence comprises entre 0,5 et 40 % en poids environ par rapport au poids total de la composition tinctoriale, et encore plus préférentiellement entre 1 et 20 % en poids environ.

Le pH de la composition tinctoriale conforme à l'invention est généralement compris entre 2 et 11 environ, et de préférence entre 5 et 10 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 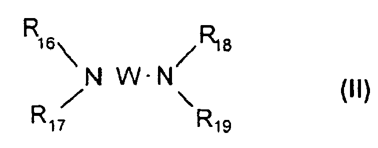
dans laquelle W est un reste propylène éventuellement substitué par un groupement hydroxyle ou un radical alkyle en C₁-C₆ ; R₁₆, R₁₇, R₁₈ et R₁₉, identiques ou différents, représentent un atome d'hydrogène, un radical alkyle en C₁-C₆ ou hydroxyalkyle en C₁-C₆.

La composition tinctoriale conforme à l'invention peut, en plus du ou des colorants directs cationiques définis précédemment, contenir un ou plusieurs colorants directs additionnels qui peuvent par exemple être choisis parmi les colorants benzéniques nitrés, les colorants anthraquinoniques, les colorants naphtoquinoniques, les colorants triarylméthaniques, les colorants xanthéniques, les colorants azoïques non cationiques.

Lorsqu'elle est destinée à la teinture d'oxydation, la composition tinctoriale conforme à l'invention contient, en plus du ou des colorants directs cationiques et de la glycérine et/ou du polyol particulier et/ou de l'éther de polyol définis ci-dessus, une ou plusieurs bases d'oxydation choisie parmi les bases d'oxydation classiquement utilisées pour la teinture d'oxydation et parmi lesquelles on peut notamment citer les paraphénylènediamines, les bis-phénylalkylènediamines, les para-aminophénols, les ortho-aminophénols et les bases hétérocycliques. Lorsqu'elles sont utilisées, la ou les bases d'oxydation représentent de préférence de 0,0005 à 12 % en poids environ du poids total de la composition tinctoriale, et encore plus préférentiellement de 0,005 à 6 % en poids environ de ce poids.

Lorsqu'elle est destinée à la teinture d'oxydation, la composition tinctoriale conforme à l'invention peut également renfermer, en plus du colorant direct cationique et de la glycérine et/ou du polyol particulier et/ou de l'éther de polyol définis ci-dessus ainsi que des bases d'oxydation, un ou plusieurs coupleurs de façon à modifier ou à enrichir en reflets les nuances obtenues en mettant en oeuvre le ou les colorants direct(s) cationique(s) et la ou les bases d'oxydation.

Les coupleurs utilisables dans la composition tinctoriale conforme à l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques.
Lorsqu'ils sont présents, le ou les coupleurs représentent de préférence de 0,0001 à 10 % en poids environ du poids total de la composition tinctoriale et encore plus préférentiellement de 0,005 à 5 % en poids environ de ce poids.

La composition tinctoriale conforme à l'invention peut également renfermer divers adjuvants utilisés classiquement dans les compositions pour la teinture des cheveux, tels que des agents antioxydants, des agents de pénétration, des agents séquestrants, des parfums, des tampons, des agents dispersants, des agents tensioactifs, des agents filmogènes, des céramides, des agents conservateurs, des agents filtrants, des agents opacifiants.

Bien entendu, l'homme de l'art veillera à choisir ce ou ces éventuels composés complémentaires de manière telle que les propriétés avantageuses attachées intrinsèquement à la composition tinctoriale conforme à l'invention ne soient pas, ou substantiellement pas, altérées par la ou les adjonctions envisagées.

La composition tinctoriale selon l'invention peut se présenter sous des formes diverses, telles que sous forme de liquides, de shampooings, de crèmes, de gels, ou sous toute autre forme appropriée pour réaliser une teinture des fibres kératiniques, et notamment des cheveux humains. Elle peut être obtenue par mélange extemporané d'une composition, éventuellement pulvérulente, contenant le ou les colorants directs cationiques avec une composition contenant la glycérine et/ou le polyol particulier et/ou l'éther de polyol particulier.

Lorsque l'association du colorant direct cationique et de la glycérine et/ou du polyol particulier et/ou de l'éther de polyol particulier définis selon l'invention, est utilisée dans une composition destinée à la teinture d'oxydation (une ou plusieurs bases d'oxydation sont alors utilisées, éventuellement en présence d'un ou plusieurs coupleurs) ou lorsqu'elle est utilisée dans une composition destinée à la teinture directe éclaircissante, alors la composition tinctoriale conforme à l'invention renferme en outre au moins un agent oxydant, choisi par exemple parmi le peroxyde d'hydrogène, le peroxyde d'urée, les bromates de métaux alcalins, les persels tels que les perborates et persulfates, et les enzymes telles que les peroxydases, les laccases et les oxydo-réductases à deux électrons. L'utilisation du peroxyde d'hydrogène ou des enzymes est particulièrement préférée.

Un autre objet de l'invention est un procédé de teinture des fibres kératiniques et en particulier des fibres kératiniques humaines telles que les cheveux mettant en oeuvre la composition tinctoriale telle que définie précédemment.

Selon une première variante de ce procédé de teinture conforme à l'invention, on applique sur les fibres au moins une composition tinctoriale telle que définie précédemment, pendant un temps suffisant pour développer la coloration désirée, après quoi on rince, on lave éventuellement au shampooing, on rince à nouveau et on sèche.

Le temps nécessaire au développement de la coloration sur les fibres kératiniques est généralement compris entre 3 et 60 minutes et encore plus précisément 5 et 40 minutes.

Selon une deuxième variante de ce procédé de teinture conforme à l'invention, on applique sur les fibres au moins une composition tinctoriale telle que définie précédemment, pendant un temps suffisant pour développer la coloration désirée, sans rinçage final.

Selon une forme de réalisation particulière de ce procédé de teinture, et lorsque la composition tinctoriale conforme à l'invention renferme au moins une base d'oxydation et au moins un agent oxydant, le procédé de teinture comporte une étape préliminaire consistant à stocker sous forme séparée, d'une part, une composition (A1) comprenant, dans un milieu approprié pour la teinture, au moins un colorant direct cationique et au moins de la glycérine et/ou un polyol particulier et/ou un éther de polyol particulier tels que définis précédemment et au moins une base d'oxydation et, d'autre part, une composition (B1) renfermant, dans un milieu approprié pour la teinture, au moins un agent oxydant, puis à procéder à leur mélange au moment de l'emploi avant d'appliquer ce mélange sur les fibres kératiniques.

Selon une autre forme de réalisation particulière de ce procédé de teinture, et lorsque la composition tinctoriale conforme à l'invention renferme au moins un agent oxydant, le procédé de teinture comporte une étape préliminaire consistant à stocker sous forme séparée, d'une part, une composition (A2) comprenant, dans un milieu approprié pour la teinture, au moins un colorant direct cationique et au moins de la glycérine et/ou un polyol particulier et/ou un éther de polyol particulier tels que définis précédemment et, d'autre part, une composition (B1) renfermant, dans un milieu approprié pour la teinture, au moins un agent oxydant, puis à procéder à leur mélange au moment de l'emploi avant d'appliquer ce mélange sur les fibres kératiniques.

Un autre objet de l'invention est un dispositif à plusieurs compartiments ou "kit" de teinture ou tout autre système de conditionnement à plusieurs compartiments dont un premier compartiment renferme la composition (A1) ou (A2) telle que définie ci-dessus et un second compartiment renferme la composition (B1) telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

### EXEMPLES

### EXEMPLE 1 :

On a préparé la composition de teinture suivante :

| | |
|---|---|
| Colorant direct cationique de formule (I)₂₇ | 0,1 g |
| Glycérine | 10, 0 g |
| 2-amino-2-méthyl-propanol q.s | pH 9 |
| Eau déminéralisée q.s.p | 100 g |

| | |
|---|---|
| M.A.* : Matière Active | |

La composition ci-dessus a été appliquée pendant 30 minutes sur des mèches de cheveux gris naturels à 90 % de blancs. Les mèches de cheveux ont ensuite été rincées, lavées avec un shampooing standard puis séchées.

Elles ont été teintes dans une nuance pourpre puissant.

### EXEMPLE 2 :

On a préparé la composition de teinture suivante :

| | |
|---|---|
| Colorant direct cationique de formule (I)₁₀ | 0,12 g |
| Monométhyléther de propylène glycol | 10, 0 g |
| 2-amino-2-méthyl-propanol q.s | pH 9 |
| Eau déminéralisée q.s.p | 100 g |

| | |
|---|---|
| M.A.* : Matière Active | |

La composition ci-dessus a été appliquée pendant 30 minutes sur des mèches de cheveux gris naturels à 90 % de blancs. Les mèches de cheveux ont ensuite été rincées, lavées avec un shampooing standard puis séchées.

Elles ont été teintes dans une nuance rouge puissant.

## Revendications

1. Composition pour la teinture des fibres kératiniques et en particulier des fibres kératiniques humaines telles que les cheveux, renfermant dans un milieu approprié pour la teinture, (i)au moins un colorant direct cationique de formule (I) suivante :
A―N=N―B (I)
dans laquelle :
**le symbole A** représente un groupement choisi parmi les structures A1 à A3 suivantes : 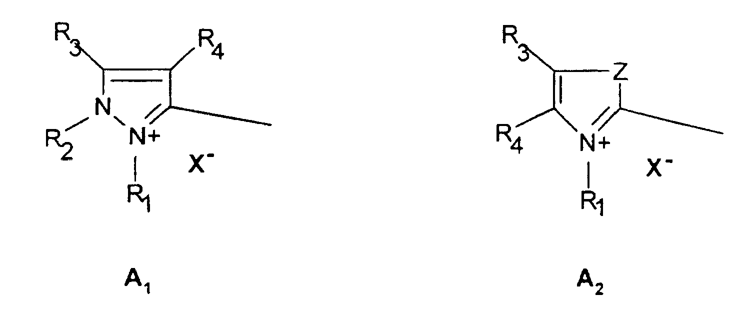 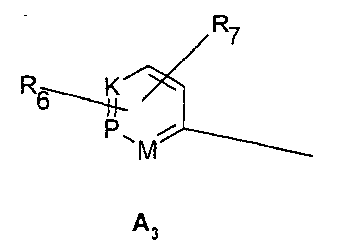
structures A1 à A3 dans lesquelles,
R₁ désigne un radical alkyle en C₁-C₄, un radical phényle pouvant être substitué par un radical alkyle en C₁-C₄ ou un atome d'halogène choisi parmi le chlore, le brome, l'iode et le fluor ;
R₂ désigne un radical alkyle en C₁-C₄ ou un radical phényle;
R₃ et R₄, identiques ou différents, représentent un radical alkyle en C₁-C₄, un radical phényle, ou bien, dans le cas de la structure A1, peuvent former ensemble un cycle benzénique substitué, et dans le cas de la structure A2, peuvent former ensemble un cycle benzénique éventuellement substitué par un ou plusieurs radicaux alkyle en C₁-C₄, alcoxy en C₁-C₄, ou NO₂;
R₃ peut en outre désigner un atome d'hydrogène;
Z désigne un atome d'oxygène, de soufre ou un groupement -NR₂;
M représente un groupement -CH, -CR (R désignant alkyle en C₁-C₄), ou -NR₅(X⁻)ᵣ;
K représente un groupement -CH, -CR (R désignant alkyle en C₁-C₄), ou -NR₅(X⁻)ᵣ;
P représente un groupement -CH, -CR (R désignant alkyle en C₁-C₄),
ou -NR₅(X⁻)ᵣ; r désigne zéro ou 1;
R₅ représente un atome O⁻, un radical alcoxy en C₁-C₄, ou un radical alkyle en C₁-C₄;
R₆ et R₇, identiques ou différents, représentent un atome d'hydrogène ou d'halogène choisi parmi le chlore, le brome, l'iode et le fluor, un radical alkyle en C₁-C₄, alcoxy en C₁-C₄ un radical -NO₂;
X⁻ représente un anion de préférence choisi parmi le chlorure, l'iodure, le méthyl sulfate, l'éthyl sulfate, l'acétate et le perchlorate;
sous réserve que,
si R₄ désigne un radical alkyle en C₁-C₄ et Z désigne un atome de soufre, R₃ ne désigne pas un atome d'hydrogène;
si R₅ désigne O⁻, alors r désigne zéro;
si K ou P ou M désignent -N-alkyle C₁-C₄ X⁻ , alors R₆ ou R₇ est différent d'un atome d'hydrogène;
si K désigne -NR₅(X⁻)ᵣ, alors M= P= -CH; -CR;
si M désigne -NR₅(X⁻)ᵣ, alors K= P= -CH; -CR;
si P désigne -NR₅(X⁻)ᵣ, alors K= M et désignent -CH ou -CR;
si Z désigne -NR₂ et R₂ désigne un radical alkyle en C₁-C₄, alors au moins un des radicaux R₁, R₃ ou R₄ de **A**_{**2**} est différent d'un radical alkyle en C₁-C₄;
**le symbole B** représente :
- **(a)** un groupement de structure B1 suivante : 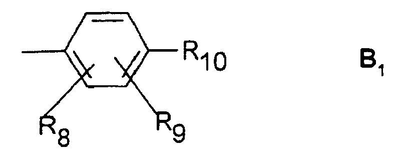 structure B1 dans laquelle,
R₈ représente un atome d'hydrogène, un atome d'halogène choisi parmi le chlore, le brome, l'iode et le fluor, un radical alkyle en C₁-C₄, alcoxy en C₁-C₄, un radical - OH, -NO₂, -NHR₁₁, -NR₁₂R₁₃, -NHCOalkyle en C₁-C₄, ou forme avec R₉ un cycle à 5 ou 6 chaînons contenant ou non un ou plusieurs hétéroatomes choisis parmi l'azote, l'oxygène ou le soufre;
R₉ représente un atome d'hydrogène, un atome d'halogène choisi parmi le chlore, le brome, l'iode et le fluor, un radical alkyle en C₁-C₄, alcoxy en C₁-C₄,
ou forme avec R₁₀ ou R₁₁ un cycle à 5 ou 6 chaînons contenant ou non un ou plusieurs hétéroatomes choisis parmi l'azote, l'oxygène ou le soufre;
R₁₀ représente un atome d'hydrogène, un radical -OH, un radical -NHR₁₁, un radical -NR₁₂R₁₃;
R₁₁ représente un atome d'hydrogène, un radical alkyle en C₁-C₄, un radical monohydroxyalkyle en C₁-C₄, polyhydroxyalkyle en C₂-C₄, un radical phényle;
R₁₂ et R₁₃, identiques ou différents, représentent un radical alkyle en C₁-C₄, un radical monohydroxyalkyle en C₁-C₄, polyhydroxyalkyle en C₂-C₄;
- **(b)** un groupement hétérocyclique azoté à 5 ou 6 chaînons susceptible de renfermer d'autres hétéroatomes et/ou des groupements carbonylés et pouvant être substitué par un ou plusieurs radicaux alkyle en C₁-C₄, amino ou phényle, et notamment un groupement de structure B2 suivante : 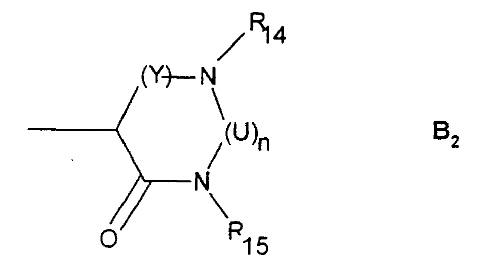 structure B2 dans laquelle,
R₁₄ et R₁₅, identiques ou différents, représentent un atome d'hydrogène , un radical alkyle en C₁-C₄, un radical phényle;
Y désigne le radical -CO- ou le radical 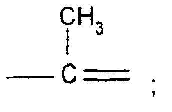 n = 0 ou 1, avec, lorsque n désigne 1, U désigne le radical -CO- ladite composition étant **caractérisée par le fait qu'**elle contient en outre :
(ii)au moins de la glycérine et/ou un polyol contenant au moins 4 atomes de carbone et/ou un éther aliphatique en C₁-C₈ de polyol en C₃-C₉ et/ou un éther aromatique en C₆-C₈ de polyol en C₂-C₉.

2. Composition selon la revendication 1, **caractérisée par le fait que** dans la formule (I), les radicaux alkyles en C₁-C₄ et les radicaux alcoxy en C₁-C₄ sont des radicaux méthyle, éthyle, butyle, méthoxy et éthoxy.

3. Composition selon la revendication 2, **caractérisée par le fait que** les colorants directs cationiques répondent aux structures (I)₁ à (I)₇₇ suivantes :

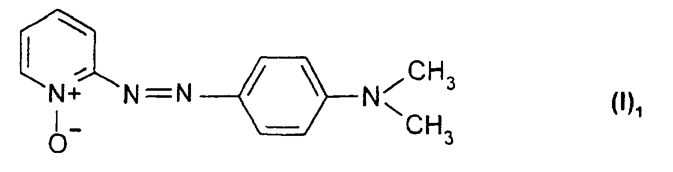

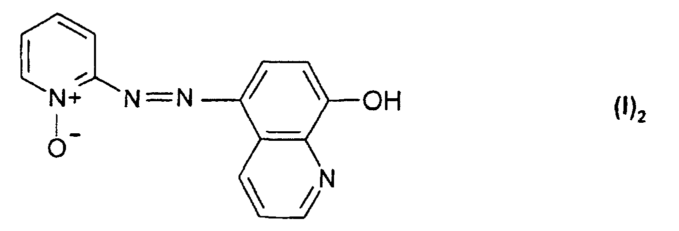

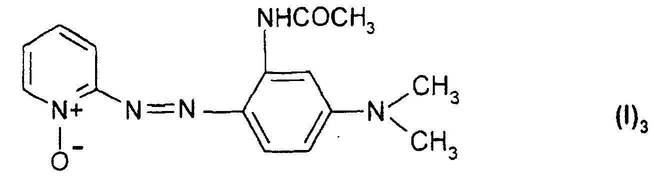

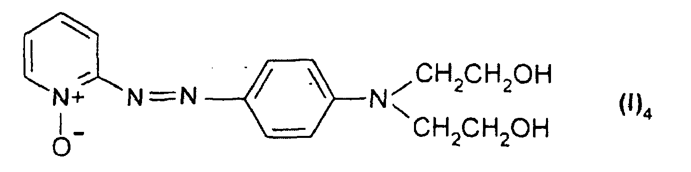

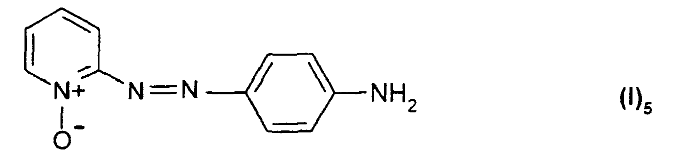

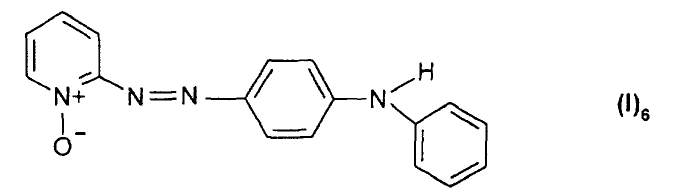

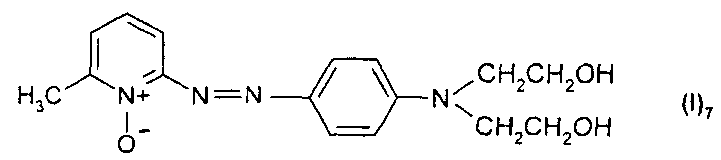

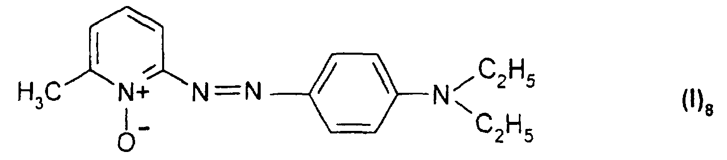

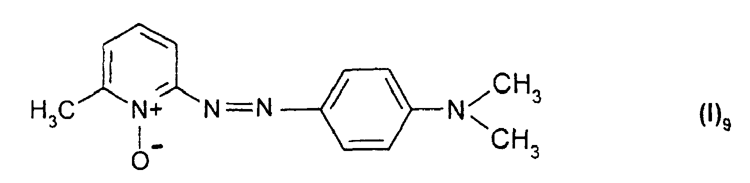

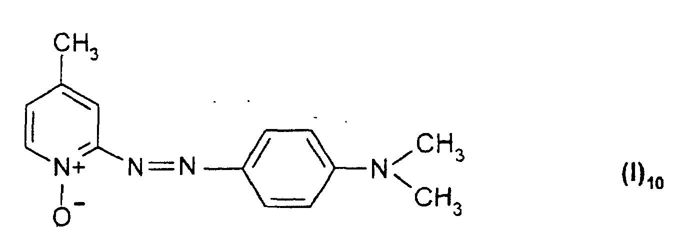

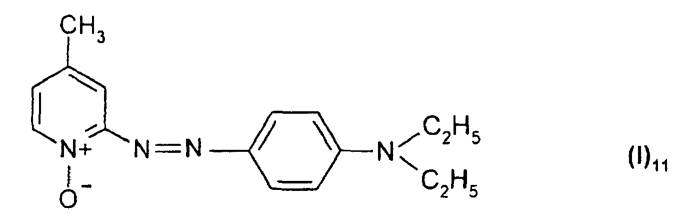

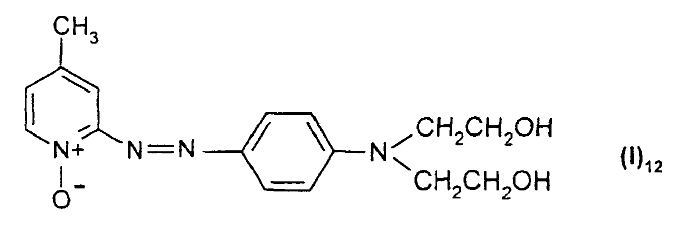

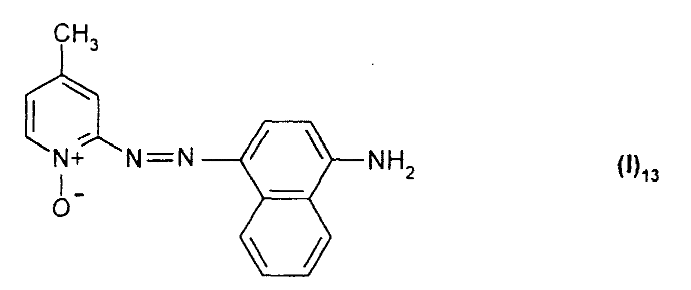

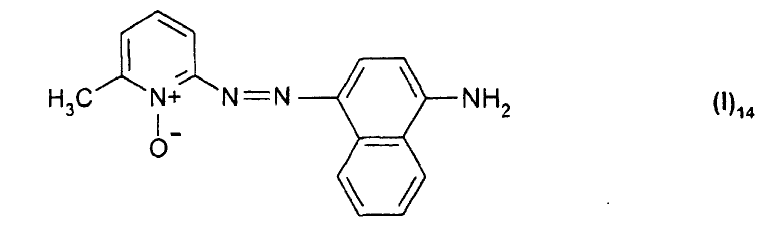

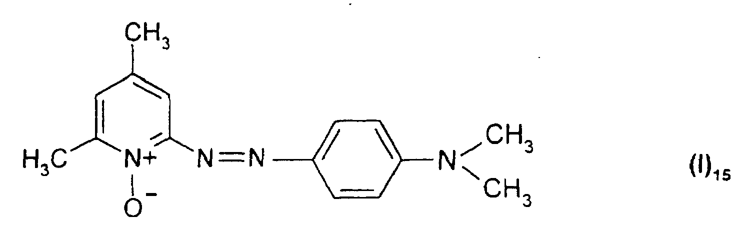

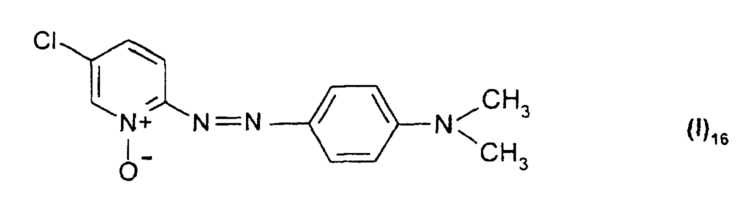

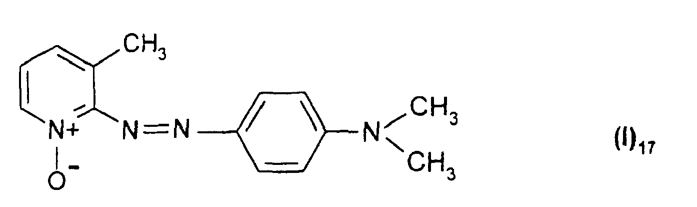

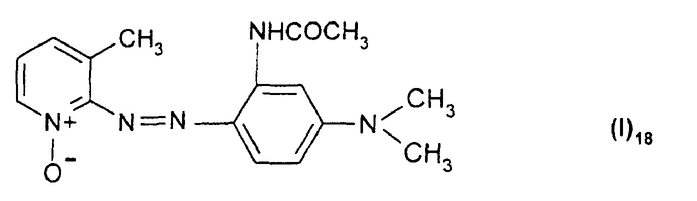

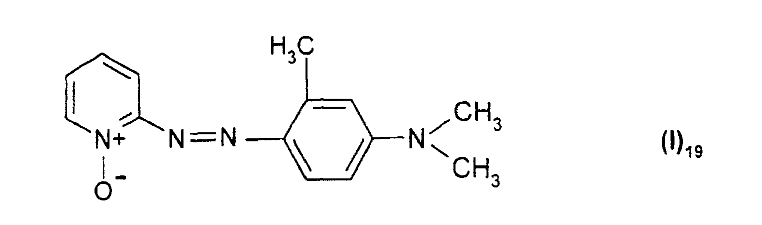

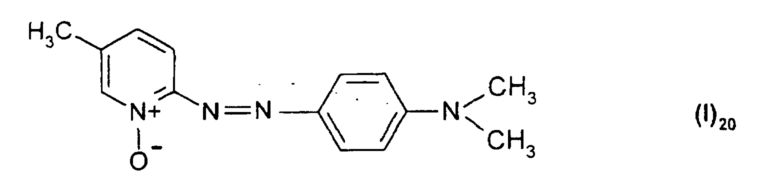

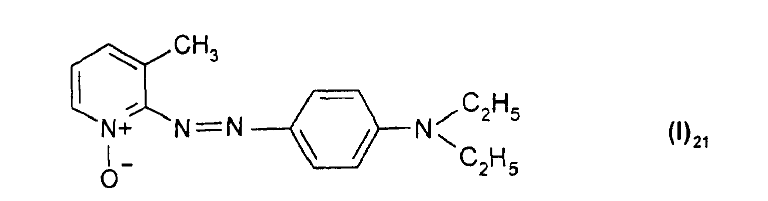

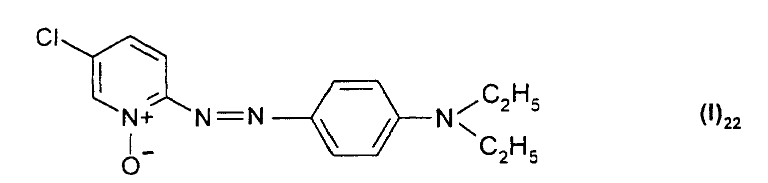

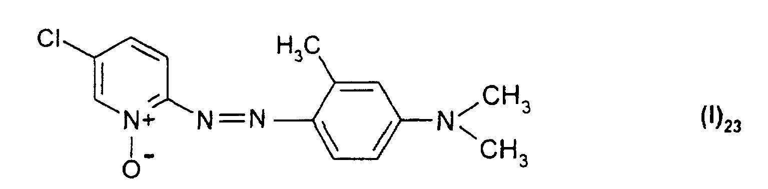

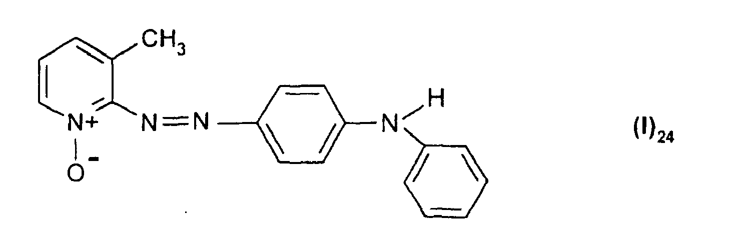

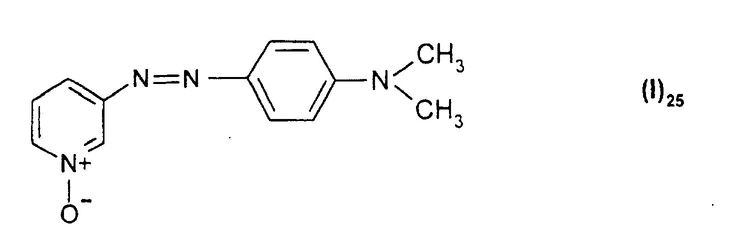

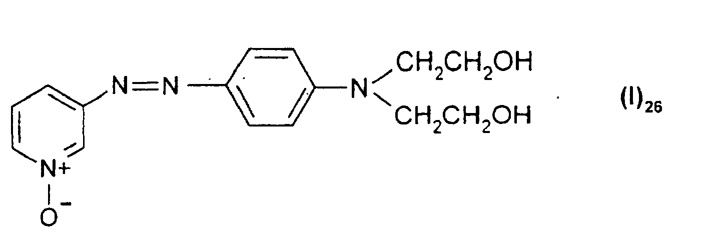

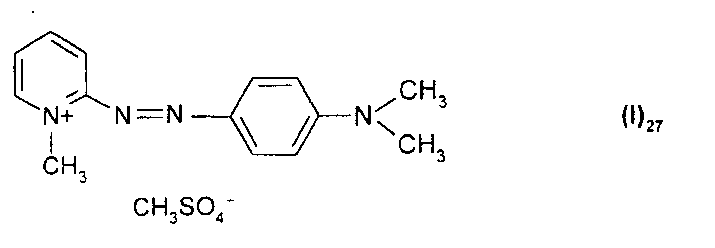

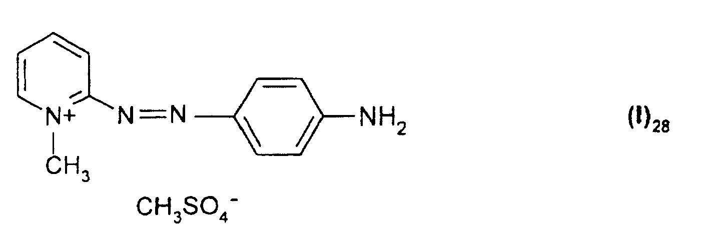

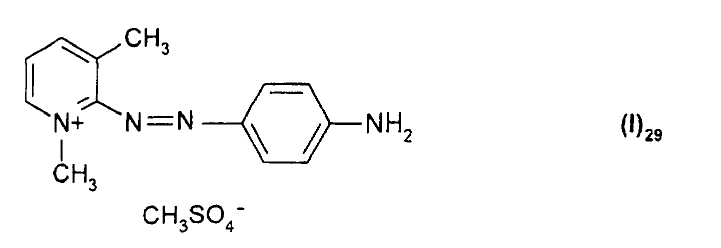

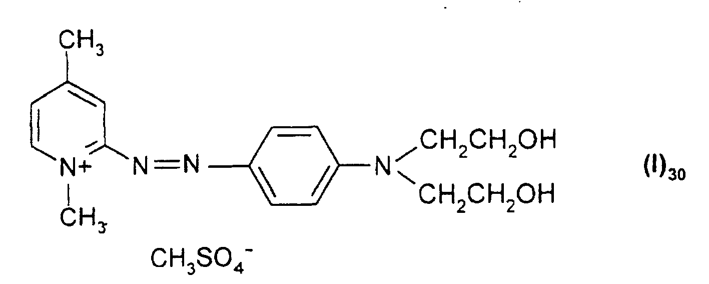

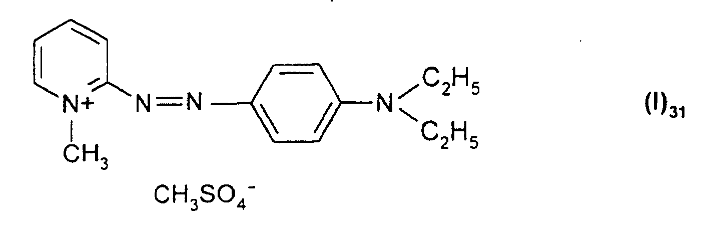

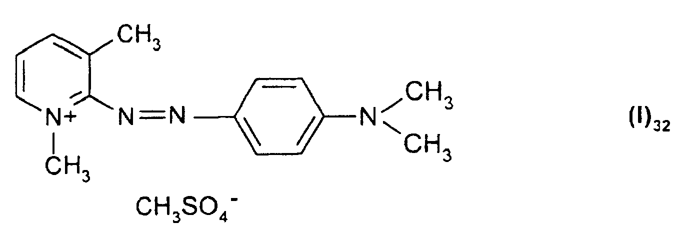

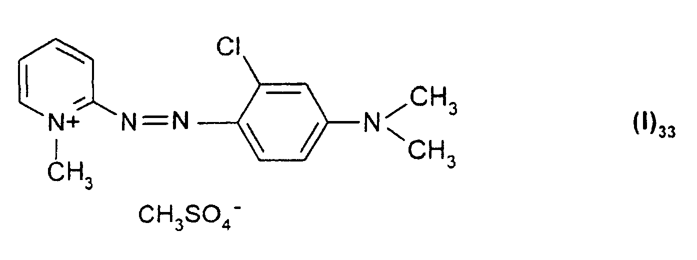

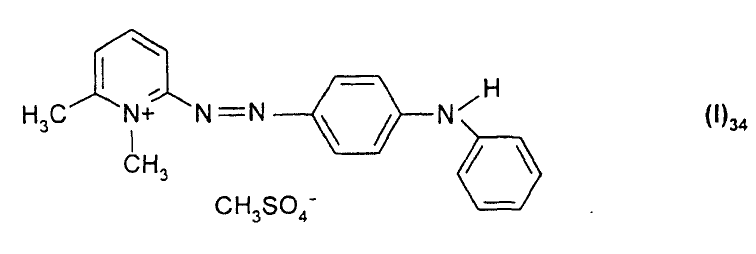

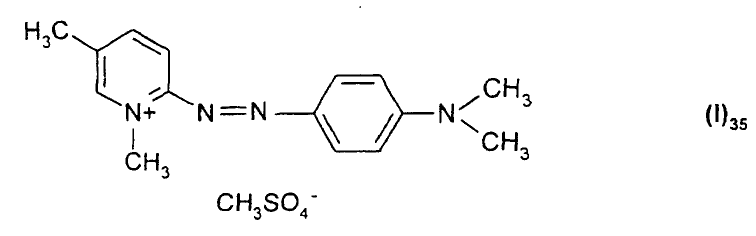

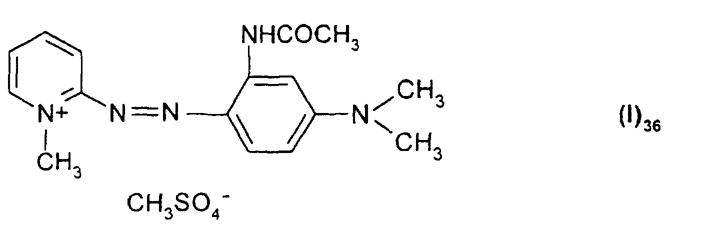

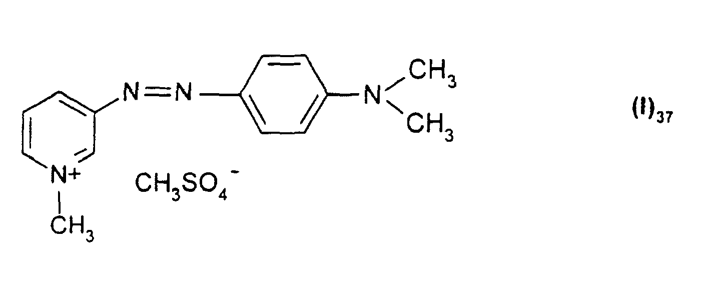

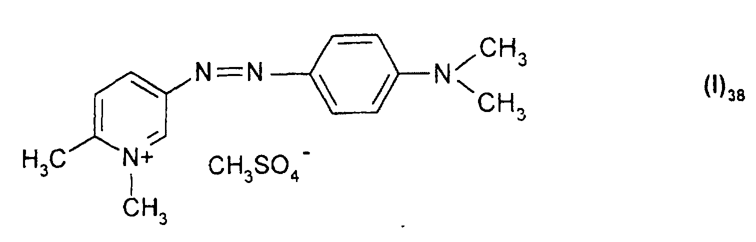

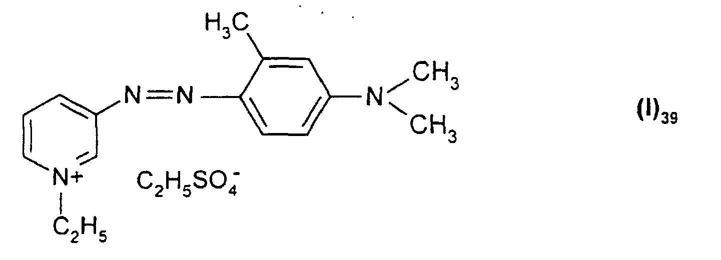

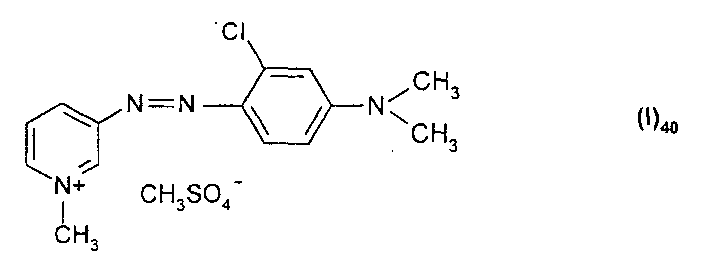

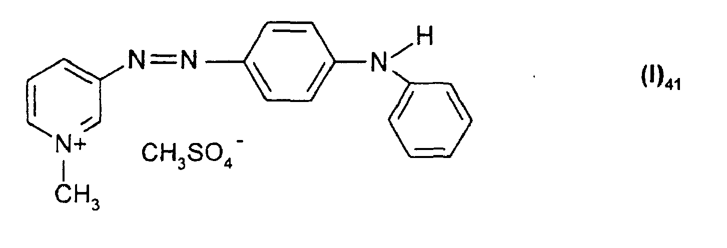

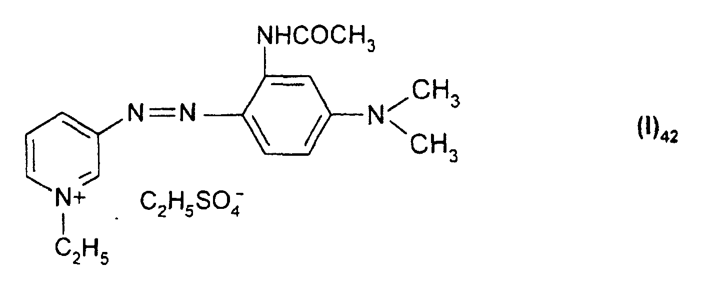

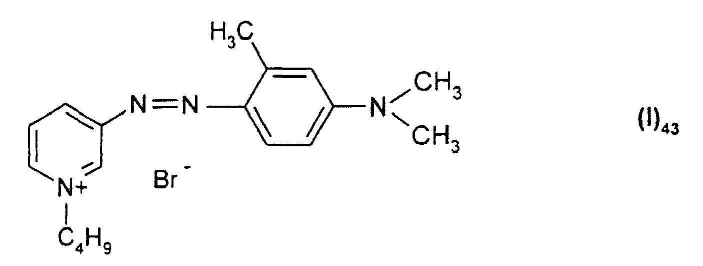

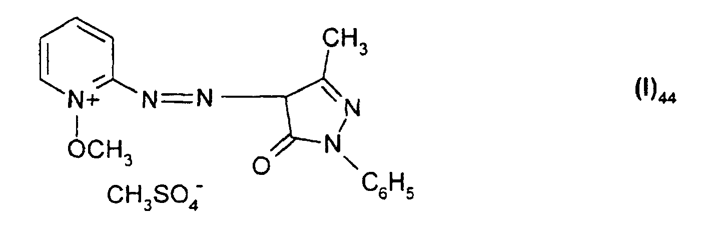

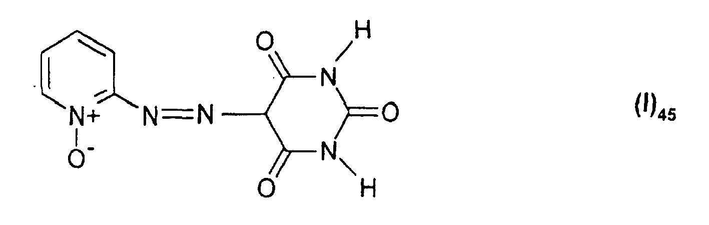

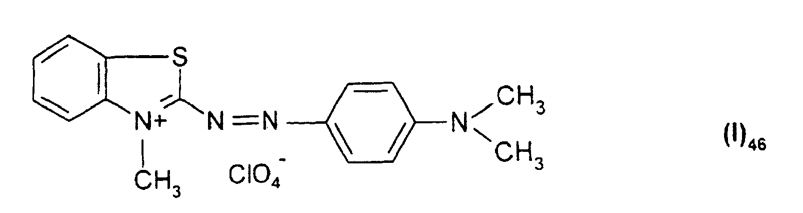

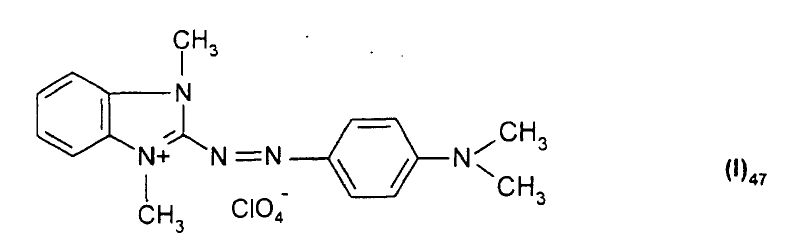

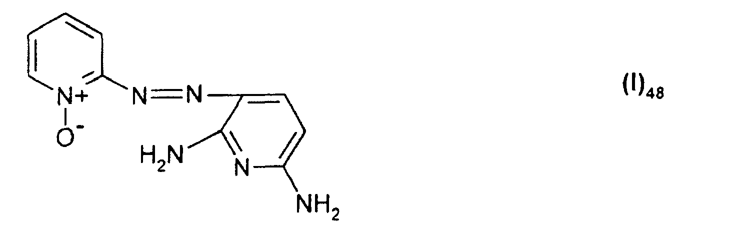

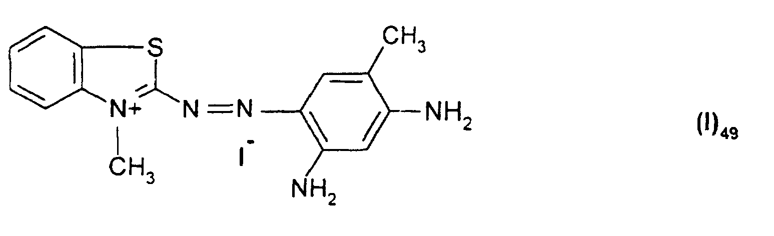

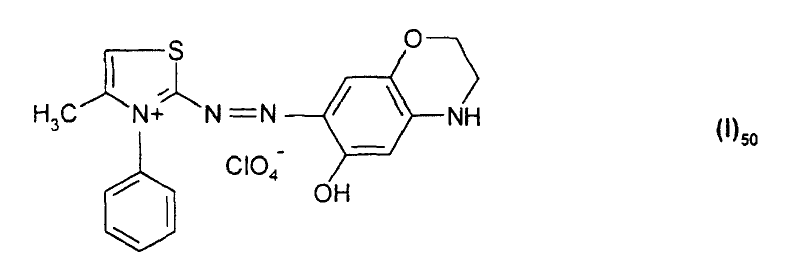

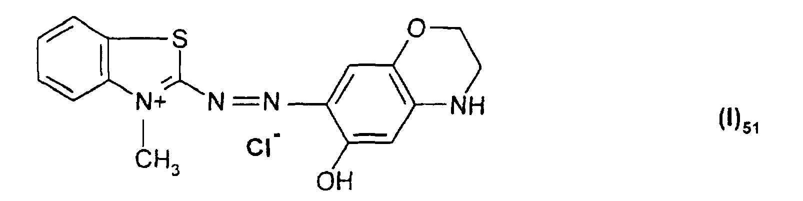

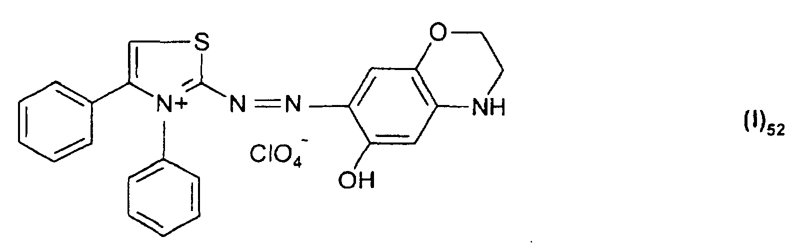

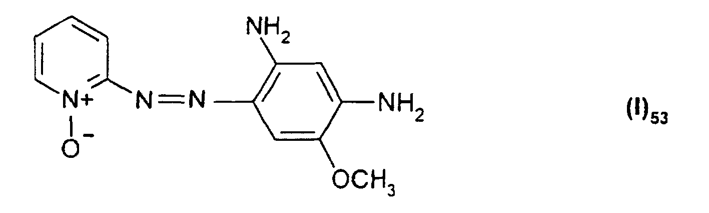

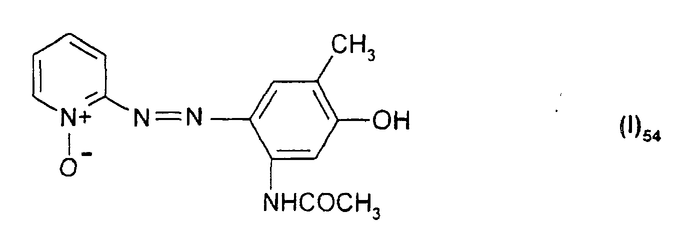

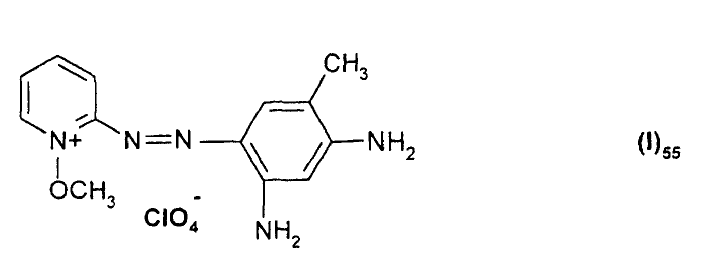

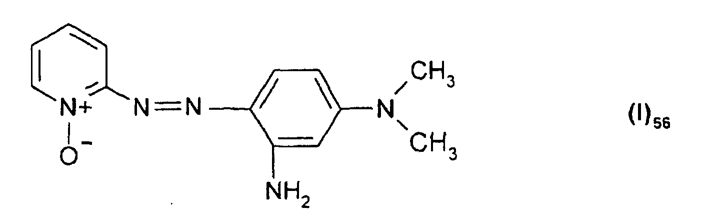

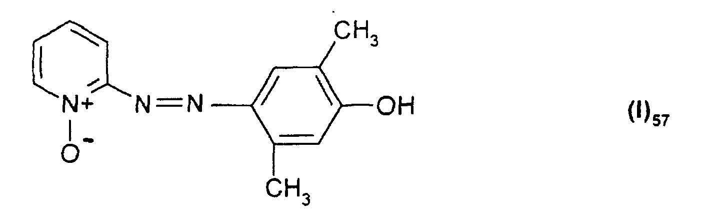

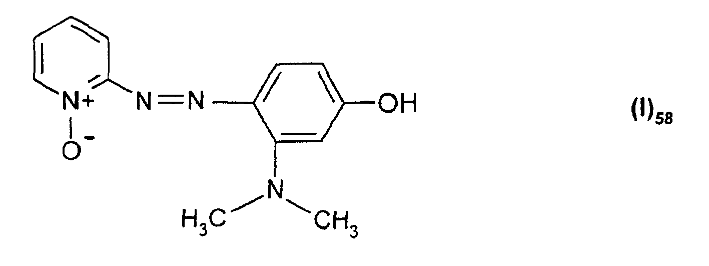

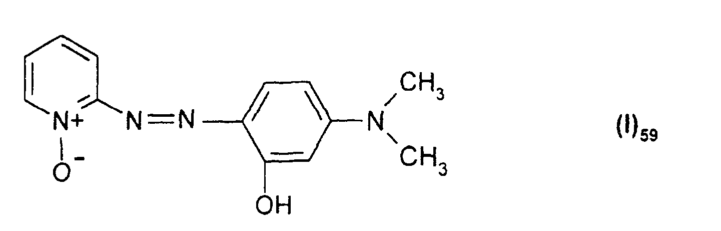

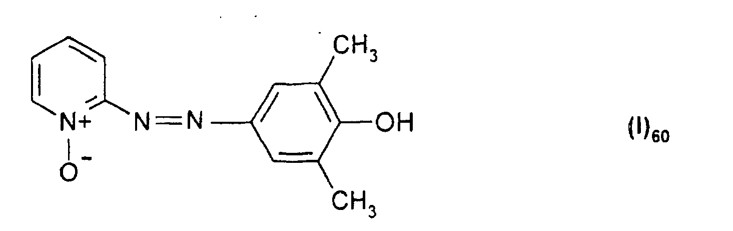

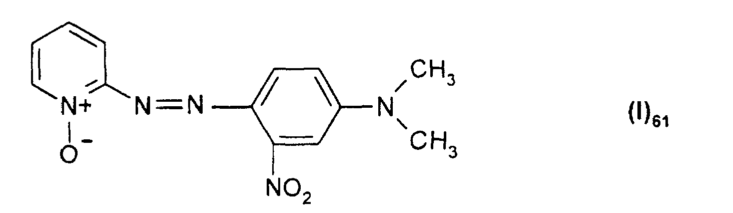

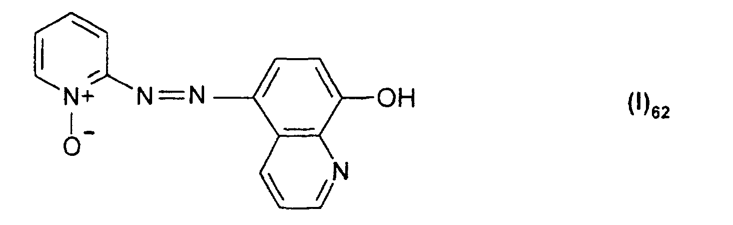

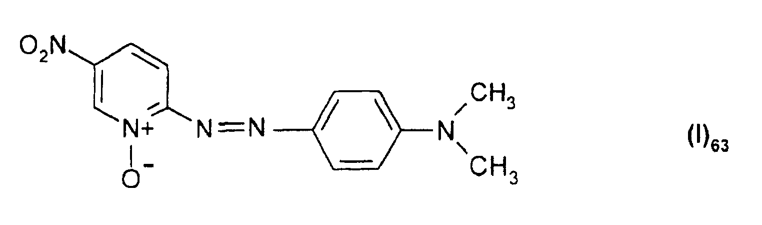

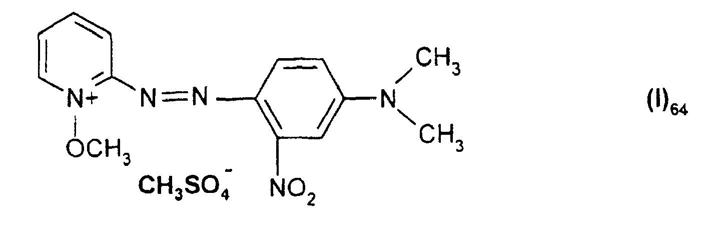

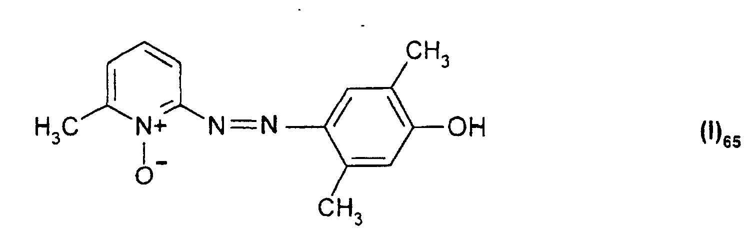

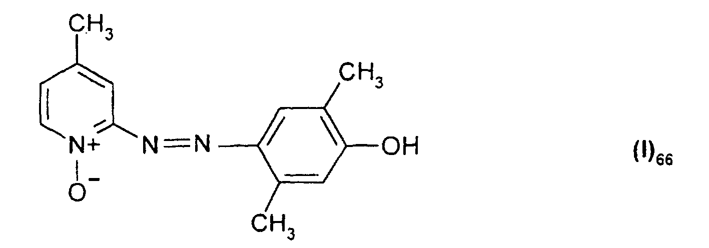

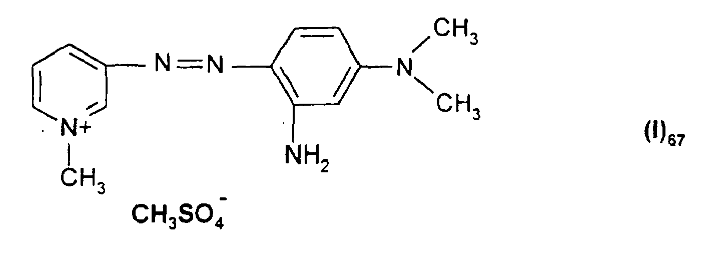

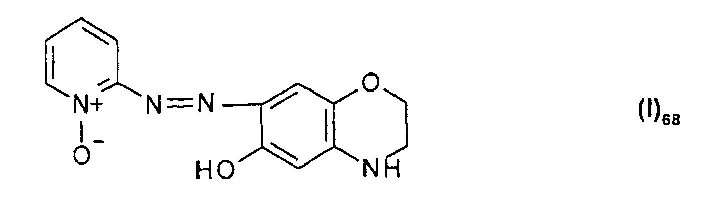

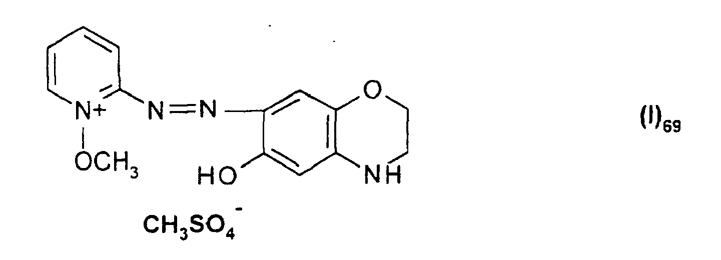

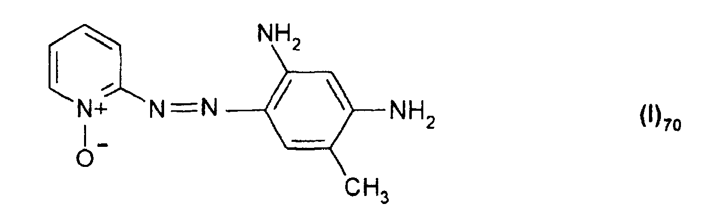

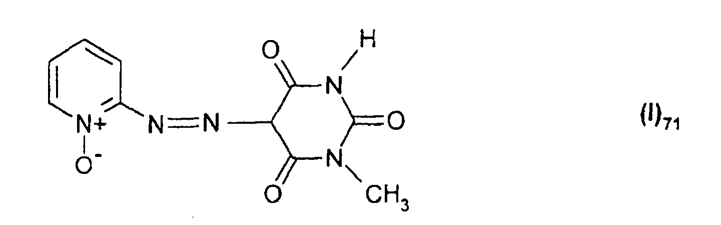

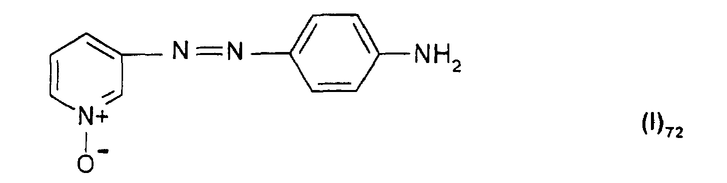

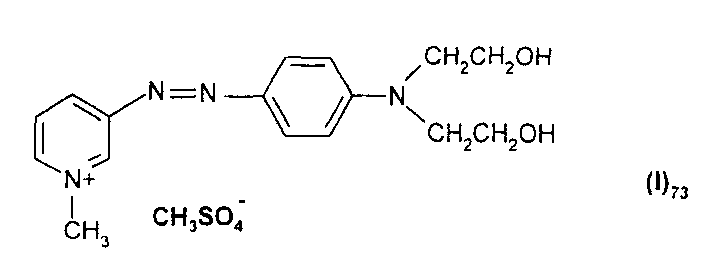

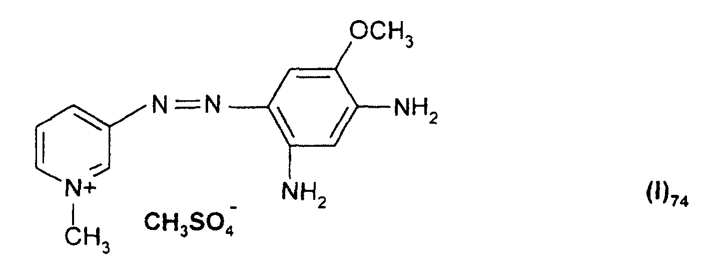

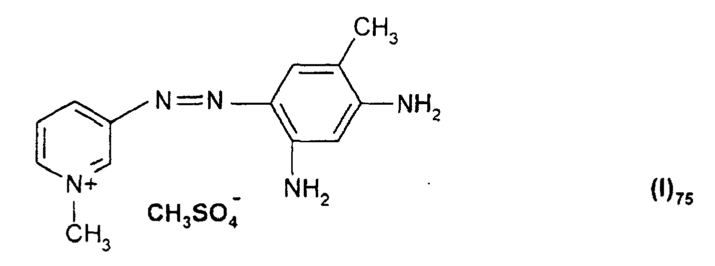

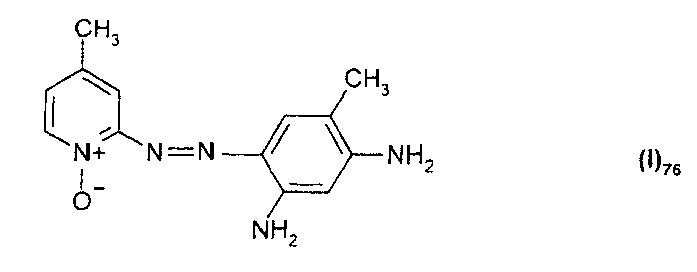

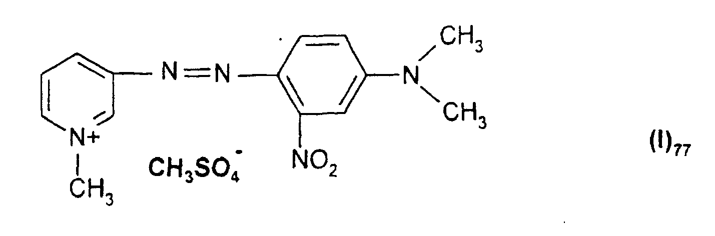

4. Composition selon l'une quelconque des revendications précédentes, **caractérisée par le fait que** le ou les colorants directs cationiques de formule (I), représentent de 0,001 à 10 % en poids du poids total de la composition.

5. Composition selon la revendication 4, **caractérisée par le fait que** le ou les colorants directs cationiques de formule (I), représentent de 0,005 à 5 % en poids du poids total de la composition.

6. Composition selon l'une quelconque des revendications précédentes, **caractérisée par le fait que** la glycérine et/ou le polyol contenant au moins 4 atomes de carbone et/ou l'éther aliphatique en C₁-C₈ de polyol en C₃-C₉ et/Ou l'éther aromatique en C₆-C₈ de polyol en C₂-C₉ représentent de 0,1 à 40% en poids du poids total de la composition.

7. Composition selon la revendication 6, **caractérisée par le fait que** la glycérine et/ou le polyol contenant au moins 4 atomes de carbone et/ou l'éther aliphatique en C₁-C₈ de polyol en C₃-C₉ et/ou l'éther aromatique en C₆-C₈ de polyol en C₂-C₉ représentent de 0,5 à 20% en poids du poids total de la composition.

8. Composition selon l'une quelconque des revendications précédentes, **caractérisée par le fait qu'**elle contient en outre des colorants directs additionnels.

9. Composition selon l'une quelconque des revendications précédentes, **caractérisée par le fait que** le milieu approprié pour les fibres kératiniques (ou support) est constitué par un mélange d'eau et d'au moins un solvant choisi parmi la glycérine et/ou les polyols et/ou les éthers de polyols définis dans la revendication 1.

10. Composition selon la revendication 9, **caractérisée par le fait que** le milieu comprend en outre au moins un solvant organique.

11. Composition selon l'une quelconque des revendications précédentes, **caractérisée par le fait qu'**elle présente un pH compris entre 2 et 11, et de préférence entre 5 et 10.

12. Composition selon l'une quelconque des revendications précédentes, **caractérisée par le fait qu'**elle est destinée à la teinture d'oxydation et qu'elle contient une ou plusieurs bases d'oxydation choisie parmi les paraphénylènediamines, les bis-phénylalkylènediamines, les para-aminophénols, les ortho-aminophénols et les bases hétérocycliques.

13. Composition selon la revendication 12, **caractérisée par le fait que** la ou les bases d'oxydation représentent 0,0005 à 12 % en poids du poids total de la composition tinctoriale.

14. Composition selon la revendication 13, **caractérisée par le fait que** la ou les bases d'oxydation représentent 0,005 à 6 % en poids du poids total de la composition tinctoriale.

15. Composition selon l'une quelconque des revendications 12 à 14, **caractérisée par le fait qu'**elle renferme un ou plusieurs coupleurs choisis parmi les métaphénylènediamines, les méta-aminophénols, les métadiphénols et les coupleurs hétérocycliques.

16. Composition selon la revendication 15, **caractérisée par le fait que** le ou les coupleurs représentent de 0,0001 à 10 % en poids du poids total de la composition tinctoriale.

17. Composition selon la revendication 16, **caractérisée par le fait que** le ou les coupleurs représentent de 0,005 à 5 % en poids du poids total de la composition tinctoriale.

18. Composition selon l'une quelconque des revendications précédentes,
**caractérisée par le fait qu'**elle est destinée à la teinture d'oxydation ou la teinture directe éclaircissante et qu'elle renferme au moins un agent oxydant.

19. Procédé de teinture des fibres kératiniques et en particulier des fibres kératiniques humaines telles que les cheveux, **caractérisé par le fait que** qu'on applique sur les fibres au moins une composition tinctoriale telle que définie à l'une quelconque des revendications 1 à 11, pendant un temps suffisant pour développer la coloration désirée, après quoi on rince, on lave éventuellement au shampooing, on rince à nouveau et on sèche.

20. Procédé de teinture des fibres kératiniques et en particulier des fibres kératiniques humaines telles que les cheveux, **caractérisé par le fait que** qu'on applique sur les fibres au moins une composition tinctoriale telle que définie à l'une quelconque des revendications 1 à 11, pendant un temps suffisant pour développer la coloration désirée, sans rinçage final.

21. Procédé de teinture des fibres kératiniques et en particulier des fibres kératiniques humaines telles que les cheveux, **caractérisé par le fait qu'**il comporte une étape préliminaire consistant à stocker sous forme séparée, d'une part, une composition (A1) comprenant, dans un milieu approprié pour la teinture, au moins un colorant direct cationique et au moins de la glycérine et/ou un polyol et/ou un éther de polyol tels que définis dans les revendications précédentes et au moins une base d'oxydation et, d'autre part, une composition (B1) renfermant, dans un milieu approprié pour la teinture, au moins un agent oxydant, puis à procéder à leur mélange au moment de l'emploi avant d'appliquer ce mélange sur les fibres kératiniques.

22. Procédé de teinture des fibres kératiniques et en particulier des fibres kératiniques humaines telles que les cheveux, **caractérisé par le fait qu'**il comporte une étape préliminaire consistant à stocker sous forme séparée, d'une part, une composition (A2) comprenant, dans un milieu approprié pour la teinture, au moins un colorant direct cationique et au moins de la glycérine et/ou un polyol et/ou un éther de polyol tels que définis dans les revendications précédentes et, d'autre part, une composition (B1) renfermant, dans un milieu approprié pour la teinture, au moins un agent oxydant, puis à procéder à leur mélange au moment de l'emploi avant d'appliquer ce mélange sur les fibres kératiniques.

23. Dispositif à plusieurs compartiments ou "kit" de teinture à plusieurs compartiments, **caractérisé par le fait qu'**un premier compartiment renferme la composition (A1) ou (A2) telle que définie à la revendication 21 ou 22 et un second compartiment renferme la composition (B1) telle que définie à la revendication 21 ou 22.

## Patentansprüche

1. Zusammensetzung zum Färben von Keratinfasern und insbesondere menschlichen Keratinfasern, wie Haaren, die in einem zum Färben geeigneten Medium enthält: (i) mindestens einen kationischen Direktfarbstoff der folgenden Formel (I):
A-N=N-B (I)
worin bedeuten:
das Symbol A eine Gruppe, die unter den folgenden Strukturen A₁ bis A₃ ausgewählt ist: 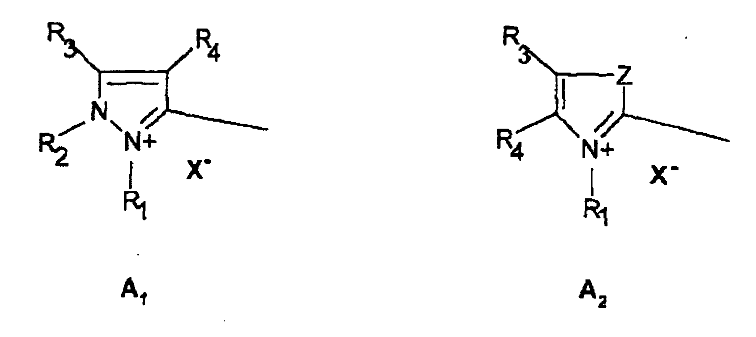 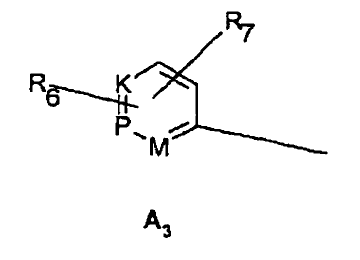
wobei in den Strukturen A₁ bis A₃ bedeuten:
R₁ eine C₁₋₄-Alkylgruppe, eine Phenylgruppe, die mit einer C₁₋₄₋Alkylgruppe oder einem Halogenatom substituiert sein kann, das unter Chlor, Brom, Iod und Fluor ausgewählt ist;
R₂ eine C₁₋₄-Alkylgruppe oder eine Phenylgruppe,
R₃ und R₄, die gleich oder verschieden sind, eine C₁₋₄-Alkylgruppe oder eine Phenylgruppe, oder sie können in A₁ gemeinsam einen substituierten Benzolring bilden oder sie können in A₂ gemeinsam einen Benzolring bilden, der gegebenenfalls mit einer oder mehreren Gruppen C₁₋₄-Alkyl, C₁₋₄-Alkoxy oder NO₂ substituiert ist, R₃ kann ferner auch ein Wasserstoffatom bedeuten,
Z ein Sauerstoffatom, ein Schwefelatom oder eine Gruppe -NR₂;
M eine Gruppe -CH, -CR (wobei R eine C₁₋₄-Alkylgruppe ist) oder -NR₅(X-)ᵣ;
K eine Gruppe -CH, -CR (wobei R eine C₁₋₄-Alkylgruppe ist) oder -NR₅(X⁻)ᵣ;
P eine Gruppe -CH, -CR (wobei R eine C₁₋₄-Alkylgruppe ist) oder -NR₅(X⁻)ᵣ; wobei r Null oder 1 bedeutet;
R₅ ein Atom O-, eine C₁₋₄-Alkoxygruppe oder eine C₁₋₄-Alkylgruppe,
R₆ und R₇, die gleich oder verschieden sind, ein Wasserstoffatom, ein Halogenatom, das unter Chlor, Brom, Iod und Fluor ausgewählt ist, eine C₁₋₄-Alkylgruppe, eine C₁₋₄-Alkoxygruppe oder die Gruppe -NO₂;
X⁻ ein Anion, das vorzugsweise unter Chlorid, Iodid, Methylsulfat, Ethylsulfat, Acetat und Perchlorat ausgewählt ist;
mit der Maßgabe, dass
· wenn R₄ C₁₋₄-Alkyl ist und Z ein Schwefelatom bedeutet, R₃ von Wasserstoff verschieden ist;
· wenn R₅ O⁻ bedeutet, r Null ist;
· wenn K oder P oder M -N-alkyl(C₁₋₄) X⁻ bedeutet, R₆ oder R₇ von Wasserstoff verschieden ist;
· wenn K -NR₅(X⁻)ᵣ bedeutet, M = P = -CH; -CR;
· wenn M -NR₅(X⁻)ᵣ bedeutet, K = P = -CH; -CR;
· wenn P -NR₅(X⁻)ᵣ bedeutet, K = M = -CH oder -CR;
· wenn Z -NR₂ bedeutet und R₂ eine C₁₋₄-Alkylgruppe ist, mindestens eine der Gruppen R₁, R₃ oder R₄ in A₂ von C₁₋₄-Alkyl verschieden ist;
das Symbol B:
(a) eine Gruppe der folgenden Struktur B₁: 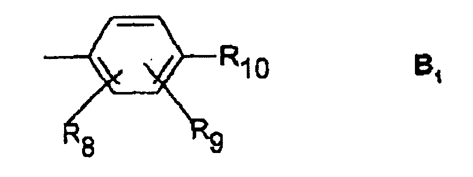
wobei in der Struktur B₁ bedeuten:
R₈ ein Wasserstoffatom, ein Halogenatom, das unter Chlor, Brom, Iod und Fluor ausgewählt ist, C₁₋₄-Alkyl, C₁₋₄-Alkoxy, -OH, -NO₂, -NHR₁₁, -NR₁₂R₁₃, -NHCOalkyl(C₁₋₄) oder R₈ bildet mit R₉ einen 5-oder 6-gliedrigen Ring, der gegebenenfalls ein oder mehrere Heteroatome enthält, die unter Stickstoff, Sauerstoff oder Schwefel ausgewählt sind;
R₉ ein Wasserstoffatom, ein Halogenatom, das unter Chlor, Brom, Iod und Fluor ausgewählt ist, C₁₋₄-Alkyl, C₁₋₄-Alkoxy oder R₉ bildet mit Rio oder R₁₁ einen 5- oder 6-gliedrigen Ring, der gegebenenfalls ein oder mehrere Heteroatome enthält, die unter Stickstoff, Sauerstoff oder Schwefel ausgewählt sind;
R₁₀ Wasserstoff, -OH, -NHR₁₁ oder -NR₁₂R₁₃;
R₁₁ Wasserstoff, C₁₋₄-Alkyl, C₁₋₄-Monohydroxyalkyl, C₂₋₄-Polyhydroxyalkyl oder Phenyl;
R₁₂ und R₁₃, die gleich oder verschieden sind, C₁₋₄-Alkyl, C₁₋₄-Monohydroxyalkyl oder C₂₋₄-Polyhydroxyalkyl;
(b) eine stickstoffhaltige, 5- oder 6-gliedrige heterocyclische Gruppe, die weitere Heteroatome und/ oder carbonylhaltige Gruppen enthalten und mit einer oder mehreren Gruppen C₁₋₄-Alkyl, Amino oder Phenyl substituiert sein kann, insbesondere eine Gruppe der folgenden Struktur B₂: 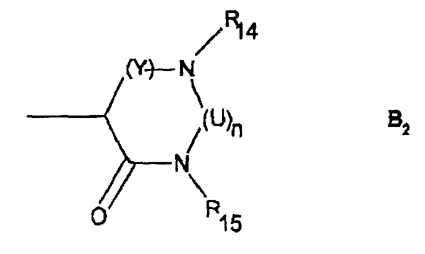
wobei in der Struktur B₂ bedeuten:
R₁₄ und R₁₅, die gleich oder verschieden sind, Wasserstoff, C₁₋₄₋Alkyl oder Phenyl;
Y die Gruppe -CO- oder die Gruppe 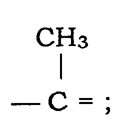 n = 0 oder 1, wobei U die Gruppe -CO- bedeutet, wenn n 1 ist;
wobei die Zusammensetzung **dadurch gekennzeichnet ist, dass** sie ferner (ii) zumindest Glycerin und/oder ein Polyol, das mindestens 4 Kohlenstoffatome aufweist, und/oder einen aliphatischen C₁₋₈₋Ether eines C₃₋₉-Polyols und/oder aromatischen C₆₋₈-Ether eines C₂₋₉-Polyols enthält.

2. Zusammensetzung nach Anspruch 1, **dadurch gekennzeichnet, dass** es sich in der Formel (I) bei den C₁₋₄-Alkylgruppen und C₁₋₄₋Alkoxylgruppen um die Gruppen Methyl, Ethyl, Butyl, Methoxy und Ethoxy handelt.

3. Zusammensetzung nach Anspruch 2, **dadurch gekennzeichnet, dass** die kationischen Direktfarbstoffe der Formel (I) den folgenden Strukturen (I)₁ bis (I)₇₇ entsprechen:

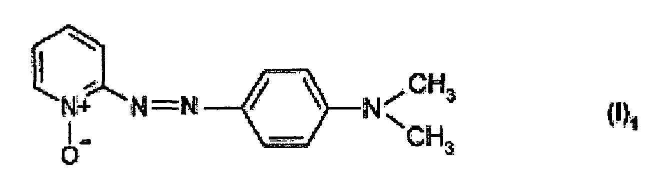

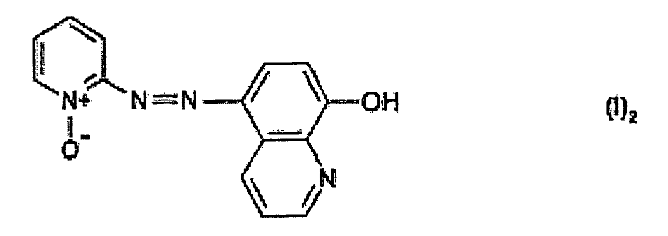

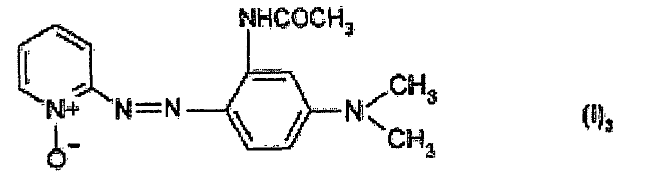

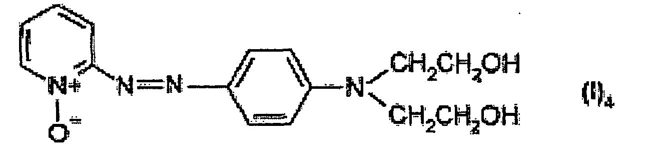

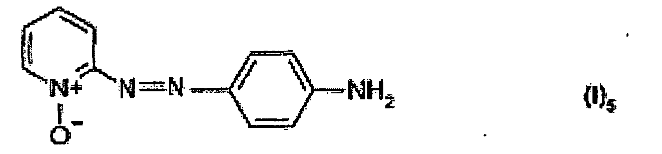

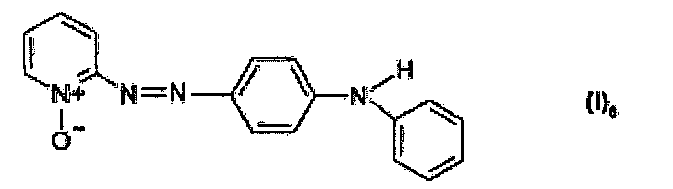

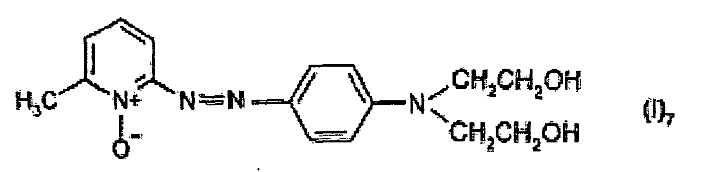

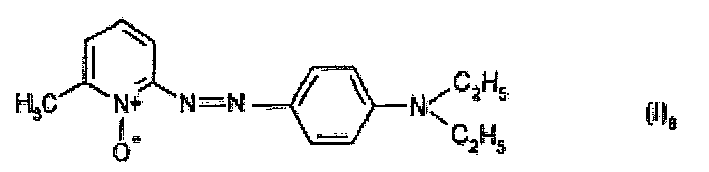

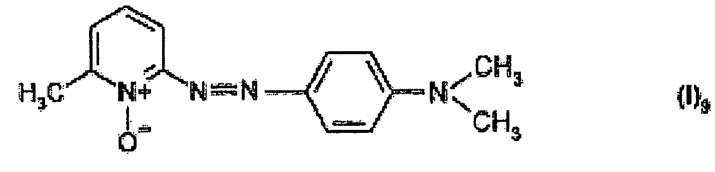

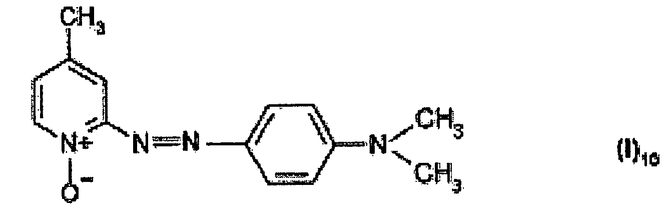

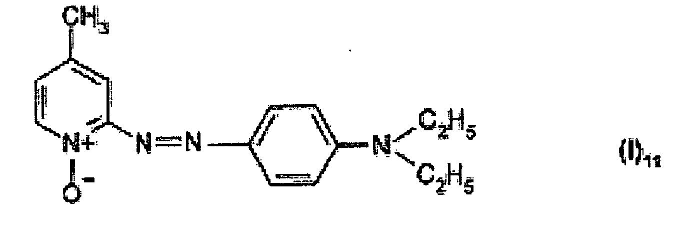

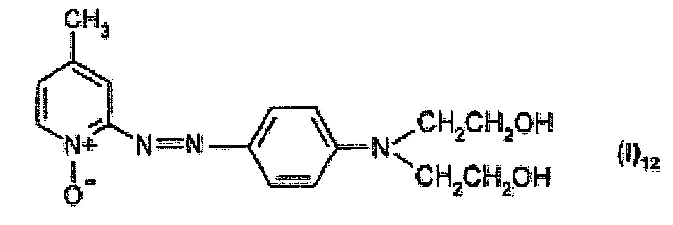

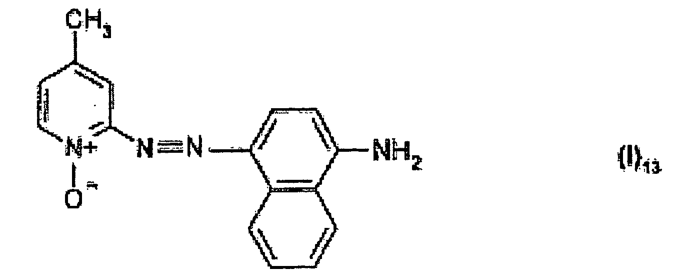

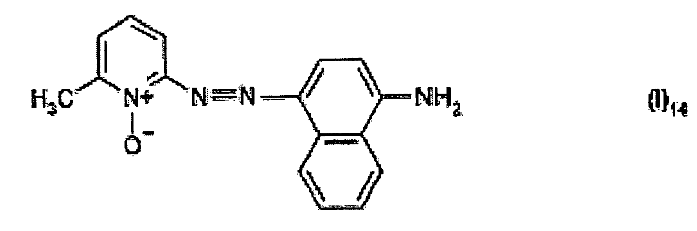

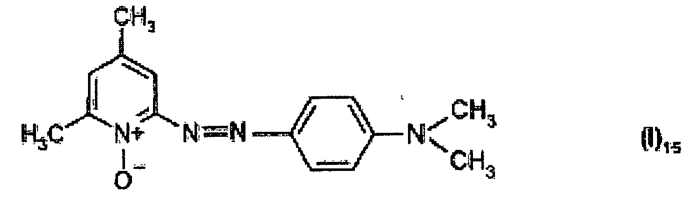

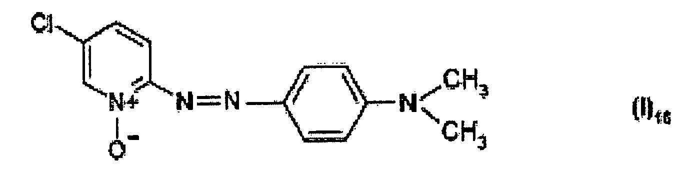

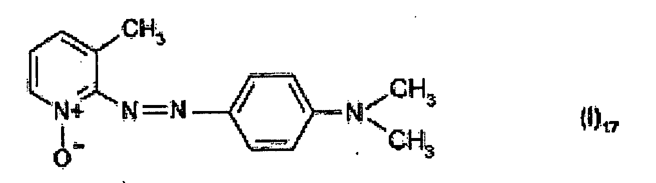

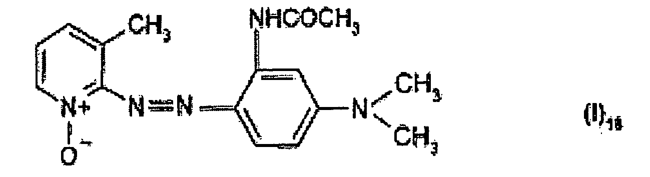

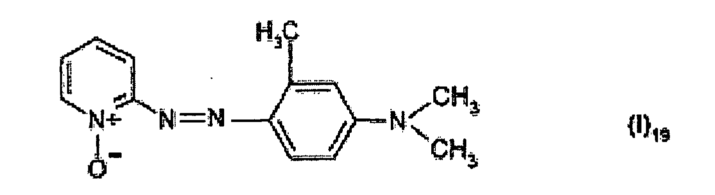

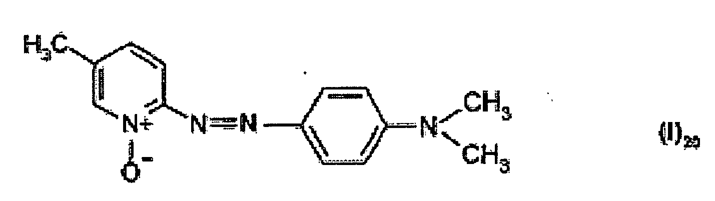

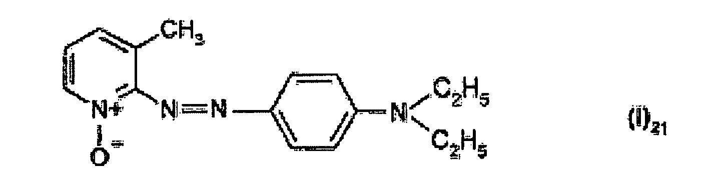

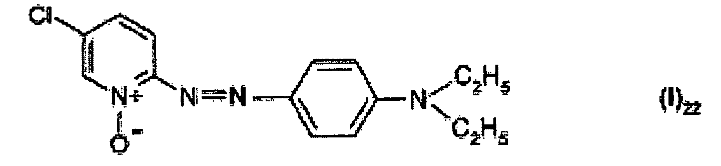

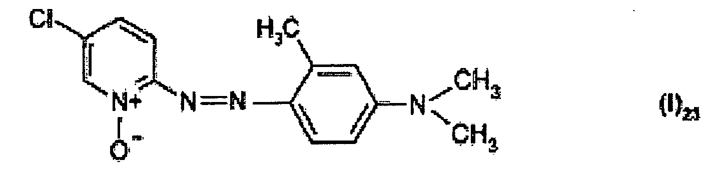

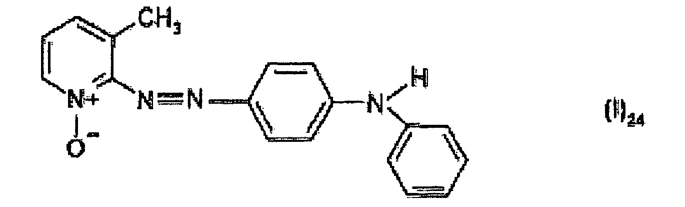

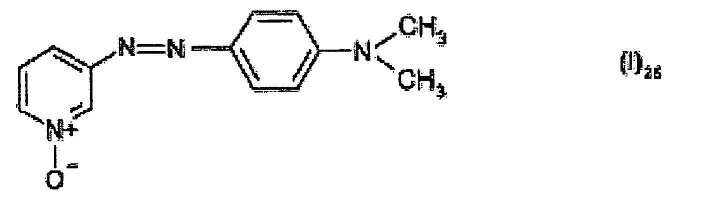

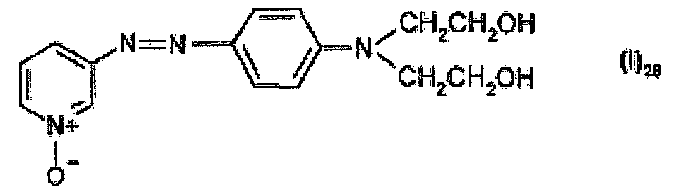

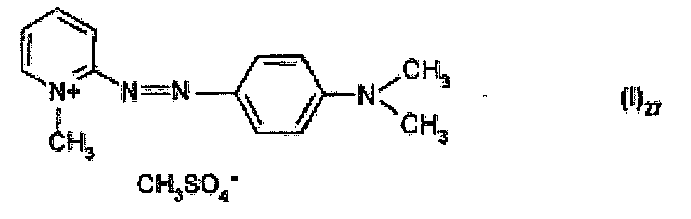

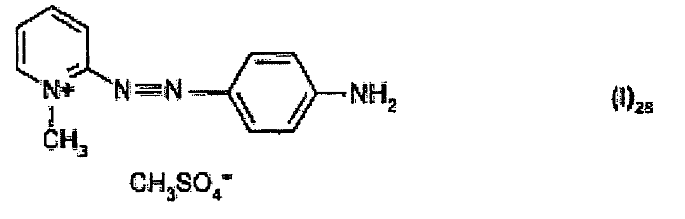

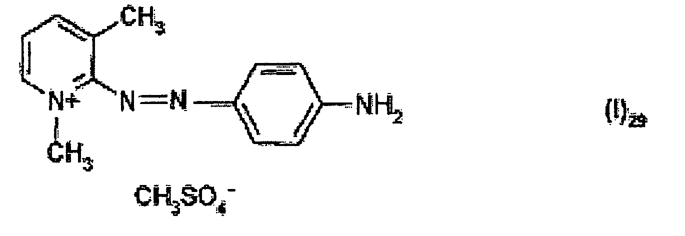

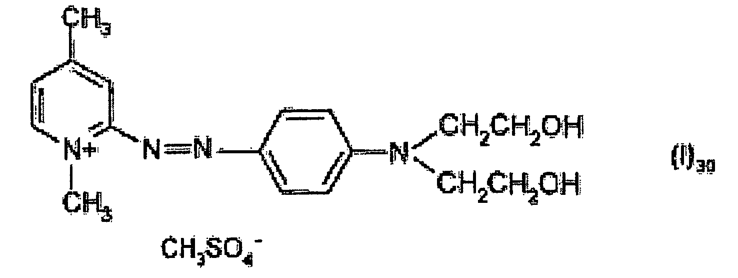

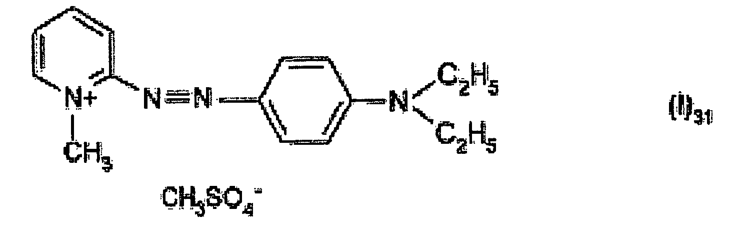

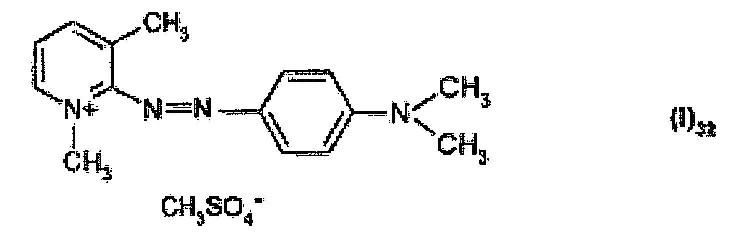

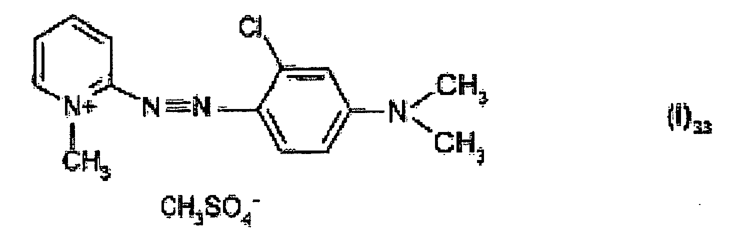

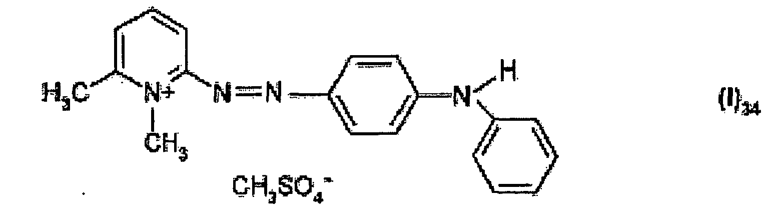

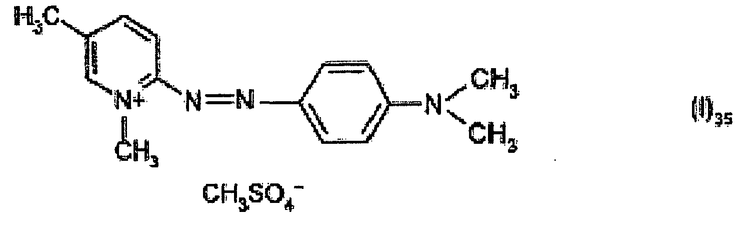

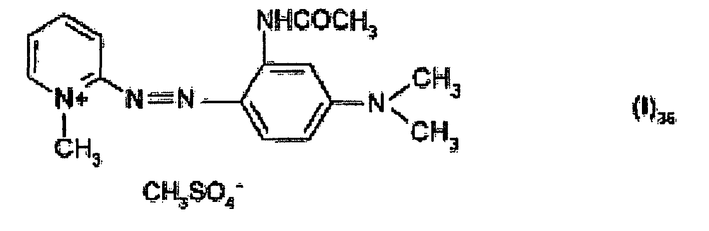

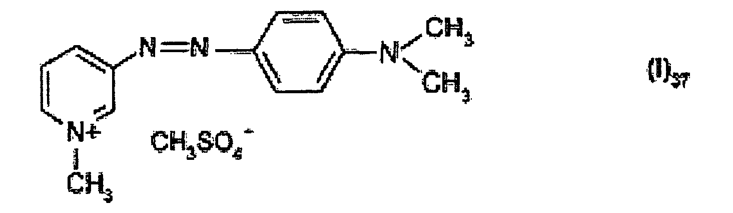

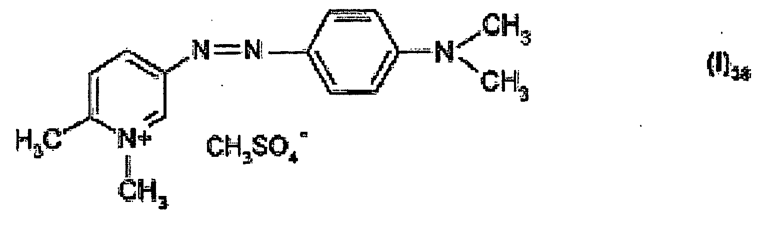

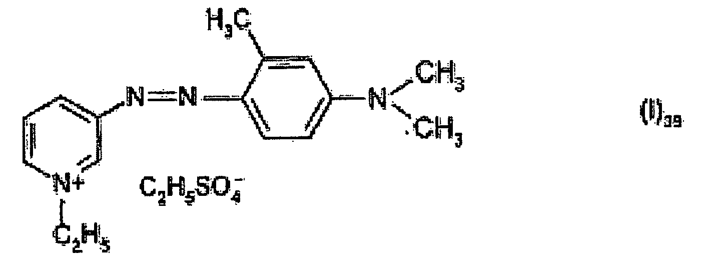

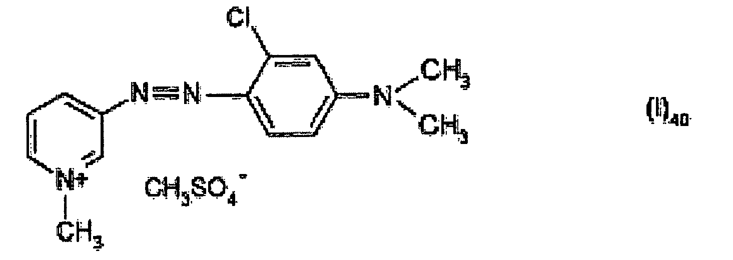

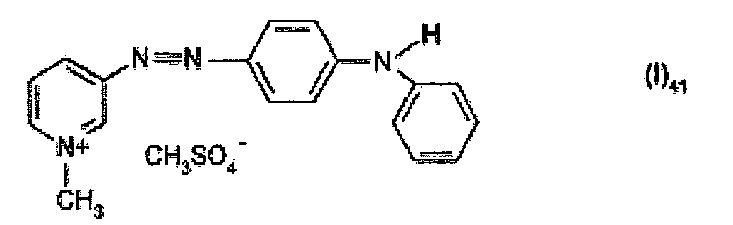

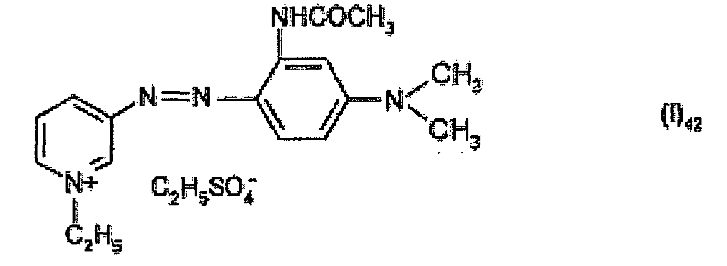

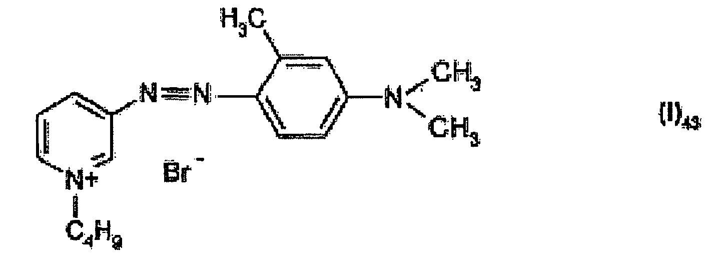

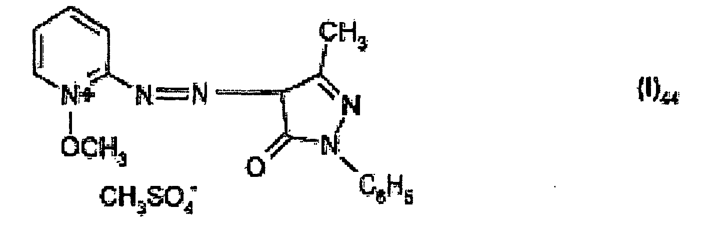

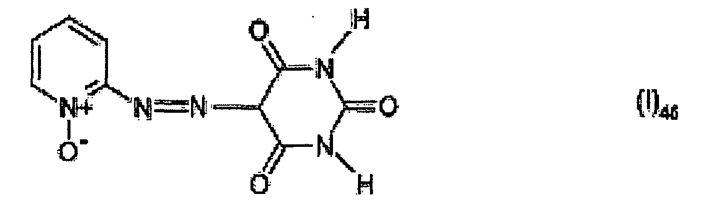

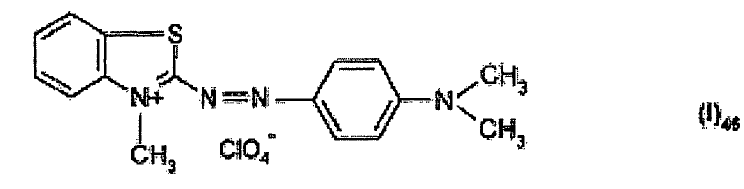

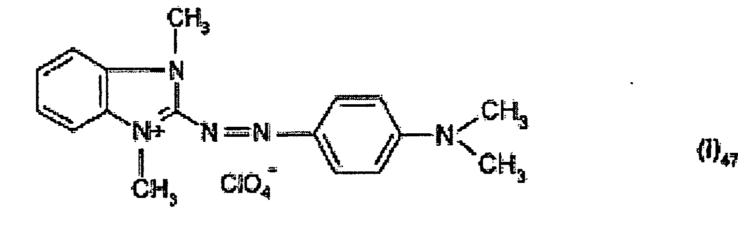

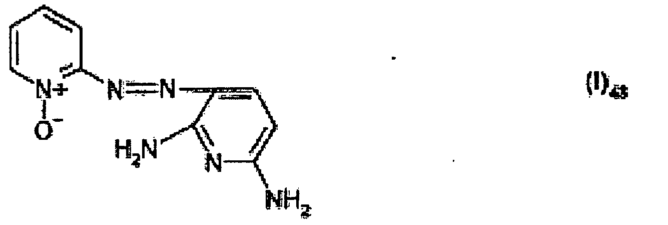

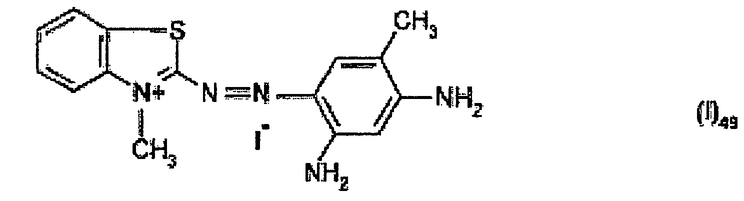

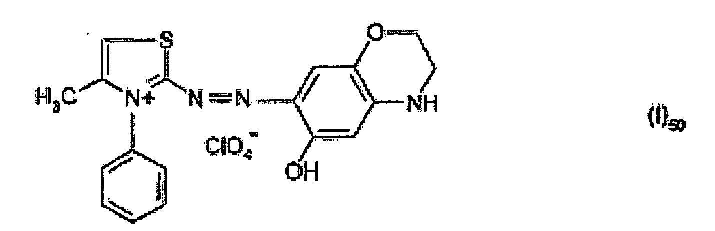

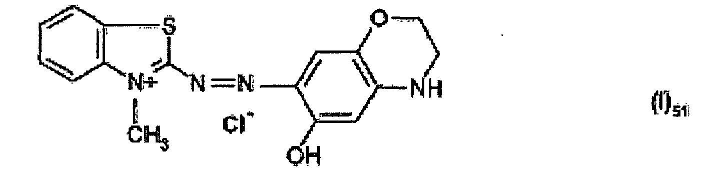

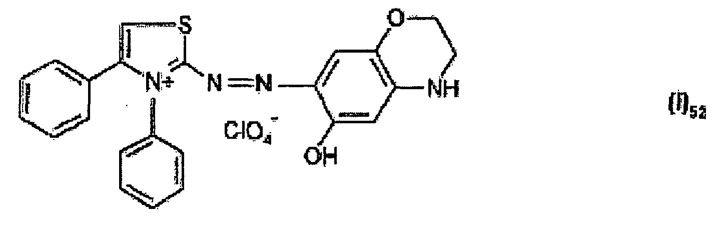

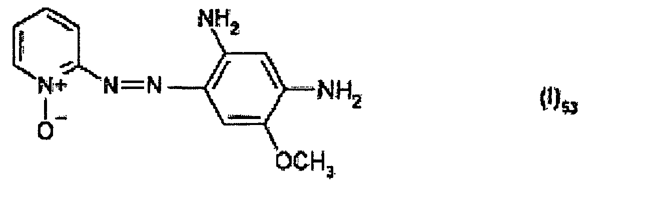

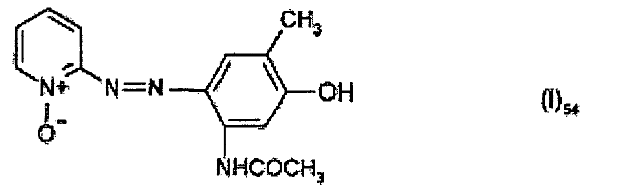

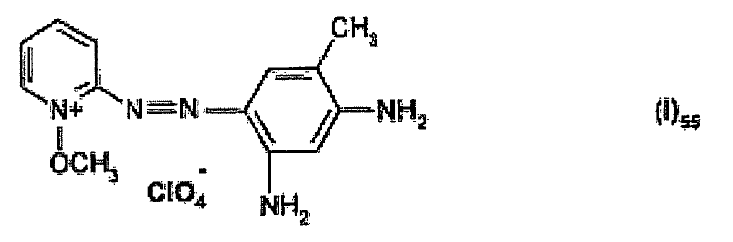

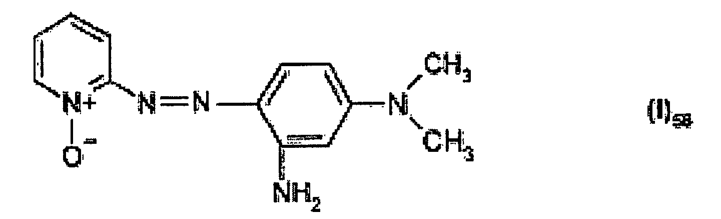

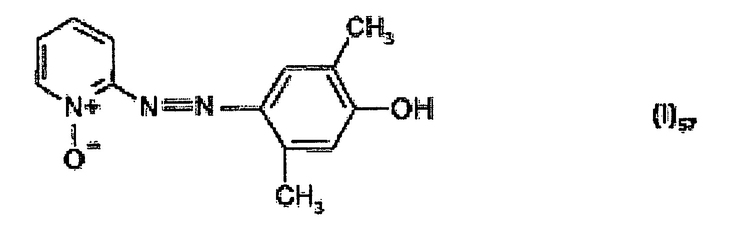

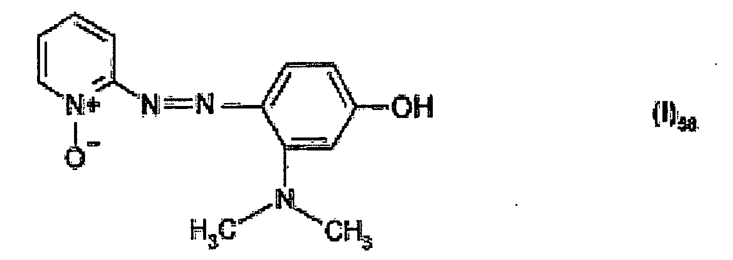

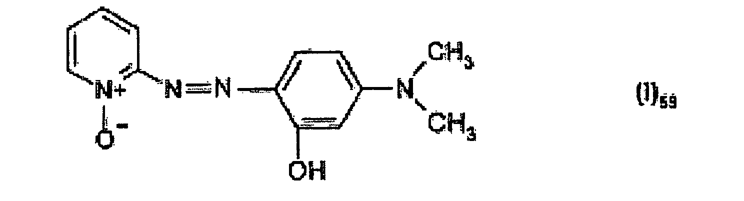

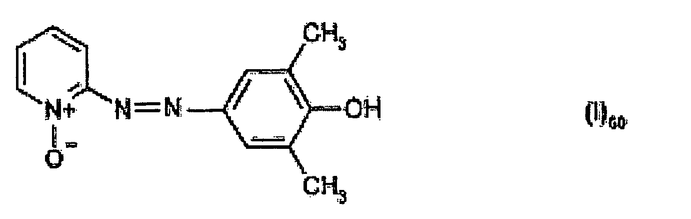

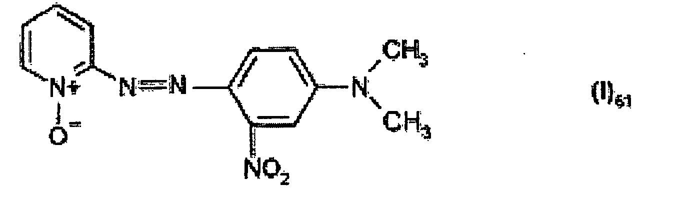

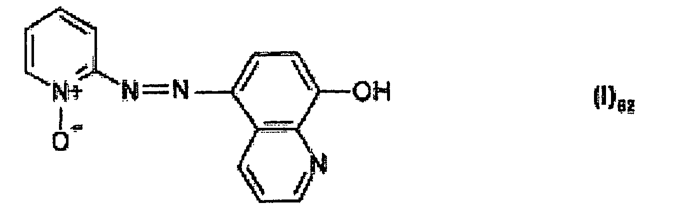

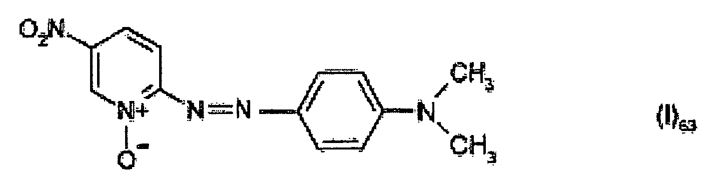

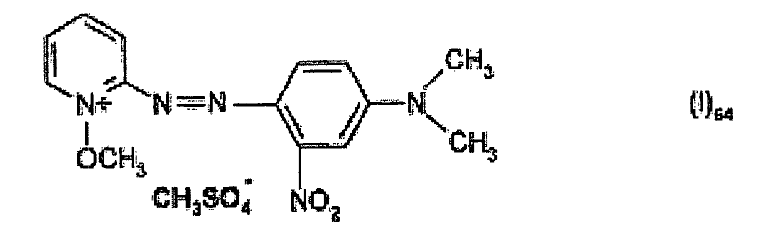

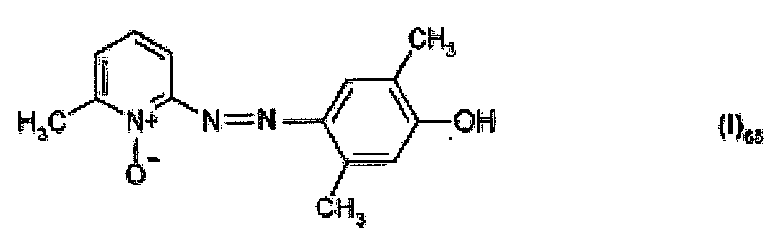

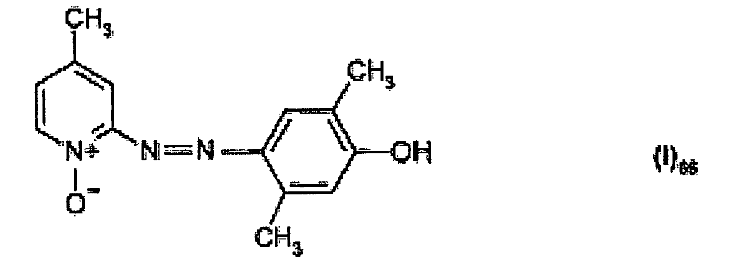

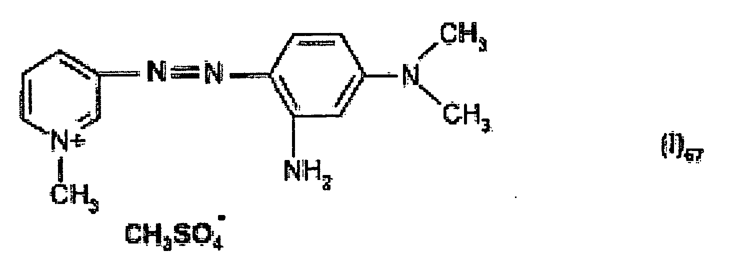

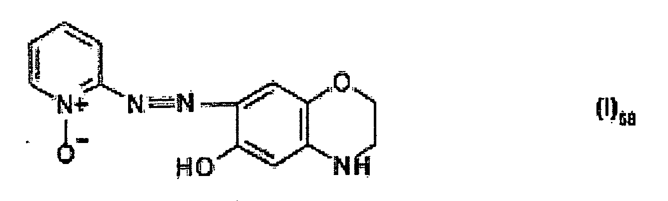

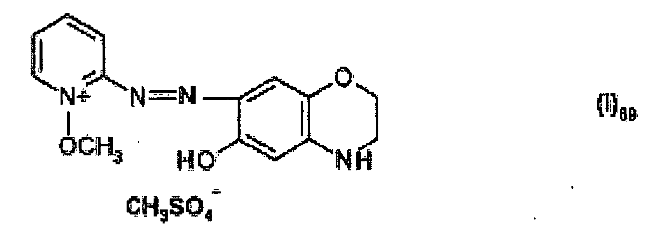

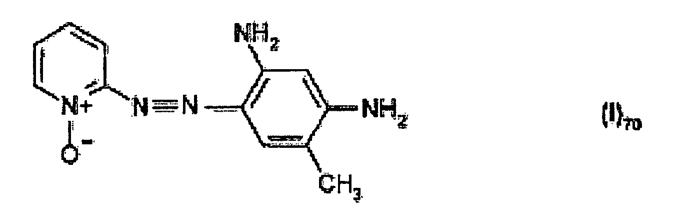

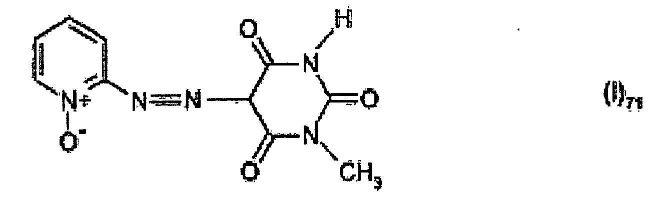

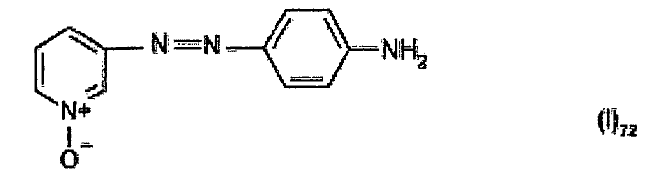

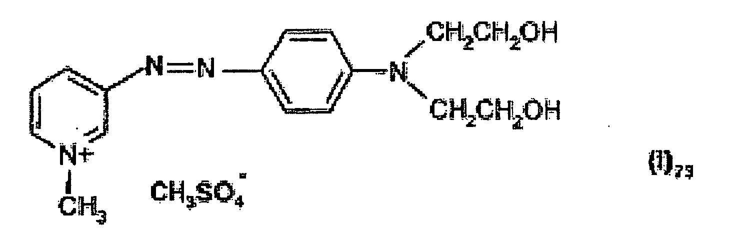

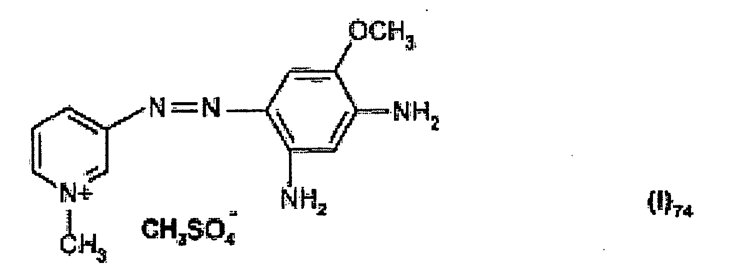

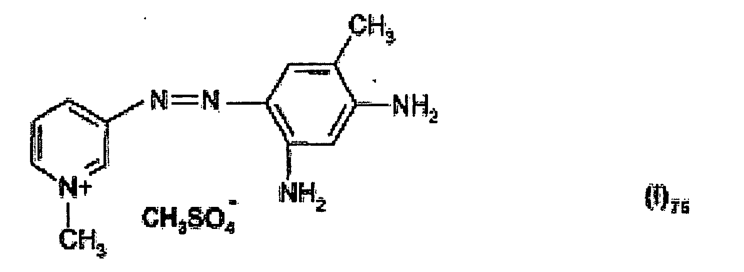

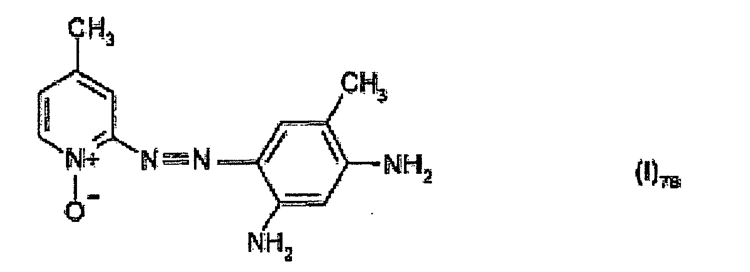

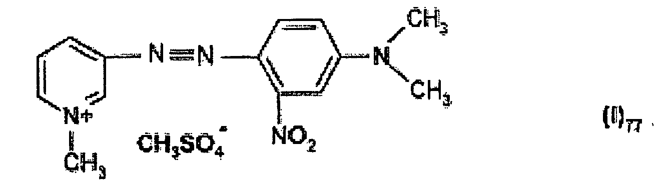

4. Zusammensetzung nach einem der vorhergehenden Ansprüche, **dadurch gekennzeichnet, dass** der oder die kationische(n) Direktfarbstoff(e) der Formel (I) 0,001 bis 10 Gew.-% des Gesamtgewichts der Zusammensetzung ausmachen.

5. Zusammensetzung nach Anspruch 4, **dadurch gekennzeichnet, dass** der oder die kationische(n) Direktfarbstoff(e) der Formel (I) 0,005 bis 5 Gew.-% des Gesamtgewichts der Zusammensetzung ausmachen.

6. Zusammensetzung nach einem der vorhergehenden Ansprüche, **dadurch gekennzeichnet, dass** das Glycerin und/oder das Polyol mit mindestens 4 Kohlenstoffatomen und/ oder der aliphatische C₁₋₈-Ether eines C₃₋₉-Polyols und/oder der aromatische C₆₋₈-Ether eines C₂₋₉-Polyols 0,1 bis 40 Gew.-% des Gesamtgewichts der Zusammensetzung ausmachen.

7. Zusammensetzung nach Anspruch 6 **dadurch gekennzeichnet, dass** das Glycerin und/ oder das Polyol mit mindestens 4 Kohlenstoffatomen und/oder der aliphatische C₁₋₈-Ether eines C₃₋₉₋Polyols und/oder der aromatische C₆₋₈-Ether eines C₂₋₉-Polyols 0,5 bis 20 Gew.-% des Gesamtgewichts der Zusammensetzung ausmachen.

8. Zusammensetzung nach einem der vorhergehenden Ansprüche, **dadurch gekennzeichnet, dass** sie ferner weitere Direktfarbstoffe enthält.

9. Zusammensetzung nach einem der vorhergehenden Ansprüche, **dadurch gekennzeichnet, dass** das für Keratinfasern geeignete Medium (oder der Träger) aus einem Gemisch von Wasser und mindestens einem Lösungsmittel besteht, welches unter Glycerin und/oder den Polyolen und/oder Polyolethern nach Anspruch 1 ausgewählt ist.

10. Zusammensetzung nach Anspruch 9, **dadurch gekennzeichnet, dass** das Medium ferner mindestens ein organisches Lösemittel enthält.

11. Zusammensetzung nach einem der vorhergehenden Ansprüche, **dadurch gekennzeichnet, dass** sie einen pH-Wert im Bereich von 2 bis 11 und vorzugsweise 5 bis 10 aufweist.

12. Zusammensetzung nach einem der vorhergehenden Ansprüche, **dadurch gekennzeichnet, dass** sie zum oxidativen Färben vorgesehen ist und eine oder mehrere Oxidationsbasen enthält, die unter den p-Phenylendiaminen, Bisphenylalkylendiaminen, p-Aminophenolen, o-Aminophenolen und heterocyclischen Basen ausgewählt sind.

13. Zusammensetzung nach Anspruch 12, **dadurch gekennzeichnet, dass** die Oxidationsbase(n) 0,0005 bis 12 Gew.-% des Gesamtgewichts der Farbmittelzusammensetzung ausmachen.

14. Zusammensetzung nach Anspruch 13, **dadurch gekennzeichnet, dass** die Oxidationsbase(n) 0,005 bis 6 Gew.-% des Gesamtgewichts der Farbmittelzusammensetzung ausmachen.

15. Zusammensetzung nach einem der Ansprüche 12 bis 14, **dadurch gekennzeichnet, dass** sie einen oder mehrere Kuppler enthält, die unter den m-Phenylendiaminen, m-Aminophenolen, m-Dihydroxybenzolen und heterocyclischen Kupplern ausgewählt sind.

16. Zusammensetzung nach Anspruch 15, **dadurch gekennzeichnet, dass** der oder die Kuppler 0,0001 bis 10 Gew.-% des Gesamtgewichts der Farbmittelzusammensetzung ausmachen.

17. Zusammensetzung nach Anspruch 16, **dadurch gekennzeichnet, dass** der oder die Kuppler 0,005 bis 5 Gew.-% des Gesamtgewichts der Farbmittelzusammensetzung ausmachen.

18. Zusammensetzung nach einem der vorhergehenden Ansprüche, **dadurch gekennzeichnet, dass** sie zum oxidativen Färben oder für die aufhellende Direktfärbung vorgesehen ist und mindestens ein Oxidationsmittel enthält.

19. Verfahren zum Färben von Keratinfasern und insbesondere menschlichen Keratinfasern, wie Haaren, **dadurch gekennzeichnet, dass** auf die Fasern mindestens eine Farbmittelzusammensetzung nach einem der Ansprüche 1 bis 11 während einer Zeitspanne aufgebracht wird, die zur Bildung der gewünschten Färbung ausreichend ist, und dann gespült, gegebenenfalls mit Haarwaschmittel gewaschen, nochmals gespült und getrocknet wird.

20. Verfahren zum Färben von Keratinfasern und insbesondere menschlichen Keratinfasern, wie Haaren, **dadurch gekennzeichnet, dass** auf die Fasern mindestens eine Farbmittelzusammensetzung nach den Ansprüchen 1 bis 11 während einer Zeitspanne aufgebracht wird, die zur Bildung der gewünschten Färbung ausreichend ist, ohne dass die Haare danach ausgespült werden.

21. Verfahren zum Färben von Keratinfasern und insbesondere menschlichen Keratinfasern, wie Haaren, **dadurch gekennzeichnet, dass** es einen vorbereitenden Schritt umfasst, der darin besteht, einerseits eine Zusammensetzung (A1), die in einem zum Färben geeigneten Medium mindestens einen kationischen Direktfarbstoff und zumindest Glycerin und/oder ein Polyol und/oder einen Polyolether nach einem der vorhergehenden Ansprüche und mindestens eine Oxidationsbase enthält, und andererseits eine Zusammensetzung (B 1) getrennt voneinander aufzubewahren, die in einem zum Färben geeigneten Medium mindestens ein Oxidationsmittel enthält, und diese bei der Anwendung zu vermischen, bevor das Gemisch auf die Fasern aufgebracht wird.

22. Verfahren zum Färben von Keratinfasern und insbesondere menschlichen Keratinfasern, wie Haaren, **dadurch gekennzeichnet, dass** es einen vorbereitenden Schritt umfasst, der darin besteht, einerseits eine Zusammensetzung (A2), die in einem zum Färben geeigneten Medium mindestens einen kationischen Direktfarbstoff und zumindest Glycerin und/oder ein Polyol und/oder einen Polyolether nach einem der vorhergehenden Ansprüche enthält, und andererseits eine Zusammensetzung (B1) getrennt voneinander aufzubewahren, die in einem zum Färben geeigneten Medium mindestens ein Oxidationsmittel enthält, und diese bei der Anwendung zu vermischen, bevor das Gemisch auf die Fasern aufgebracht wird.

23. Vorrichtung mit mehreren Abteilungen oder "Kit" zum Färben mit mehreren Abteilungen, **dadurch gekennzeichnet, dass** eine Abteilung die in Anspruch 21 oder 22 definierte Zusammensetzung (A1) oder (A2) und eine andere Abteilung die in Anspruch 21 oder 22 definierte Zusammensetzung (B1) enthält.

## Claims

1. Composition for dyeing keratin fibres, and in particular human keratin fibres such as the hair, containing, in a medium which is suitable for dyeing, **(i)** at least one cationic direct dye of formula (I) below:
**A―N=N―B** (I)
in which:
**the symbol A** represents a group chosen from structures A1 to A3 below: 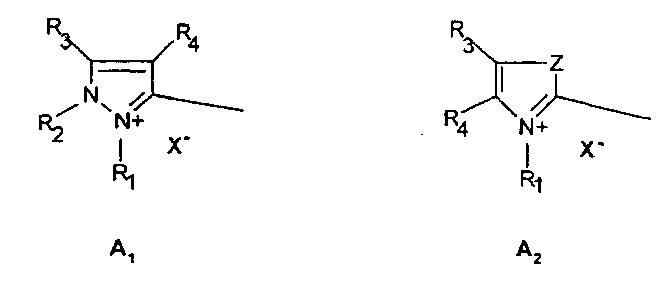 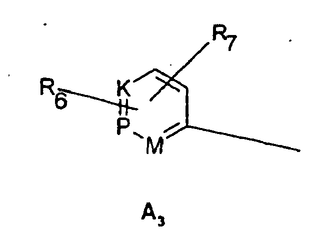
in which structures A1 to A3,
R₁ denotes a C₁-C₄ alkyl radical, a phenyl radical which can be substituted with a C₁-C₄ alkyl radical or a halogen atom chosen from chlorine, bromine, iodine and fluorine;
R₂ denotes a C₁-C₄ alkyl radical or a phenyl radical;
R₃ and R₄, which may be identical or different, represent a C₁-C₄ alkyl radical, a phenyl radical or, in the case of structure A1, can together form a substituted benzene ring, and in the case of structure A2, can together form a benzene ring optionally substituted with one or more C₁-C₄ alkyl, C₁-C₄ alkoxy or NO₂ radicals;
R₃ can also denote a hydrogen atom;
Z denotes an oxygen or sulphur atom or a group -NR₂;
M represents a group -CH, -CR (R denoting C₁-C₄ alkyl) or -NR₅ (X⁻) r;
K represents a group -CH, -CR (R denoting C₁-C₄ alkyl) or -NR₅ (X⁻) r;
P represents a group -CH, -CR (R denoting C₁-C₄ alkyl) or -NR₅(X⁻)ᵣ; r denotes 0 or 1;
R₅ represents an atom O⁻, a C₁-C₄ alkoxy radical or a C₁-C₄ alkyl radical;
R₆ and R₇, which may be identical or different, represent a hydrogen atom or a halogen atom chosen from chlorine, bromine, iodine and fluorine, a C₁-C₄ alkyl or C₁-C₄ alkoxy radical or an -NO₂ radical;
X⁻ represents an anion preferably chosen from chloride, iodide, methyl sulphate, ethyl sulphate, acetate and perchlorate;
with the proviso that,
if R₄ denotes a C₁-C₄ alkyl radical and Z denotes a sulphur atom, R₃ does not denote a hydrogen atom;
if R₅ denotes O⁻, then r denotes zero;
if K or P or M denote C₁-C₄ -N-alkyl X⁻, then R₆ or R₇ is other than a hydrogen atom;
if K denotes -NR₅ (X⁻)ᵣ, then M=P=-CH; -CR;
if M denotes -NR₅ (X⁻)ᵣ, then K=P=-CH; -CR;
if P denotes -NR₅ (X⁻)ᵣ, then K=M and denote -CH or -CR;
if Z denotes -NR₂ and R₂ denotes a C₁-C₄ alkyl radical, then at least one of the radicals R₁, R₃ or R₄ or **A**_{**2**} is other than a C₁-C₄ alkyl radical;
**the symbol B** represents:
- **(a)** a group of structure B1 below: 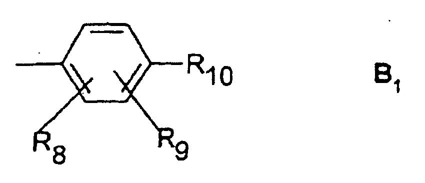
in which structure B1,
R₈ represents a hydrogen atom, a halogen atom chosen from chlorine, bromine, iodine and fluorine, a C₁-C₄ alkyl or C₁-C₄ alkoxy radical, a radical -OH, -NO₂, -NHR₁₁, -NR₁₂R₁₃ or -NHCO(C₁-C₄)alkyl radical or forms, with R₉, a 5- or 6-membered ring which may or may not contain one or more hetero atoms chosen from nitrogen, oxygen and sulphur;
R₉ represents a hydrogen atom, a halogen atom chosen from chlorine, bromine, iodine and fluorine, or a C₁-C₄ alkyl or C₁-C₄ alkoxy radical,
or forms, with R₁₀ or R₁₁, a 5- or 6-membered ring which may or may not contain one or more hetero atoms chosen from nitrogen, oxygen and sulphur;
R₁₀ represents a hydrogen atom, an -OH radical, a radical -NHR₁₁ or a radical -NR₁₂R₁₃;
R₁₁ represents a hydrogen atom, a C₁-C₄ alkyl radical, a C₁-C₄ monohydroxyalkyl or C₂-C₄ polyhydroxyalkyl radical or a phenyl radical;
R₁₂ and R₁₃, which may be identical or different, represent a C₁-C₄ alkyl radical or a C₁-C₄ monohydroxyalkyl or C₂-C₄ polyhydroxyalkyl radical;
-**(b)** a 5- or 6-membered nitrogenous heterocyclic group which can contain other hetero atoms and/or carbonyl groups and which can be substituted with one or more C₁-C₄ alkyl, amino or phenyl radicals, and in particular a group of structure B2 below: 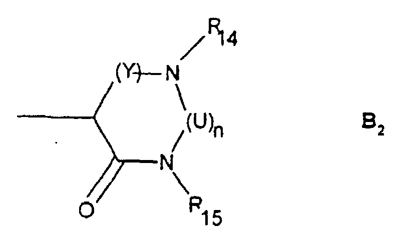
in which structure B2,
R₁₄ and R₁₅, which may be identical or different, represent a hydrogen atom, a C₁-C₄ alkyl radical or a phenyl radical;
Y denotes a -CO- radical or a radical 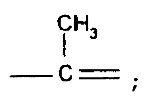
n = 0 or 1, where, when n denotes 1, U denotes a -CO-radical,
the said composition being **characterized in that** it also contains:
**(ii)** at least glycerol and/or a polyol containing at least 4 carbon atoms and/or a C₁-C₈ aliphatic ether of a C₃-C₉ polyol and/or a C₆-C₈ aromatic ether of a C₂-C₉ polyol.

2. Composition according to Claim 1, **characterized in that**, in formula (I), the C₁-C₄ alkyl radicals and the C₁-C₄ alkoxy radicals are methyl, ethyl, butyl, methoxy and ethoxy radicals.

3. Composition according to Claim 2, **characterized in that** the cationic direct dyes correspond to structures (I)₁ to (I)₇₇ below:

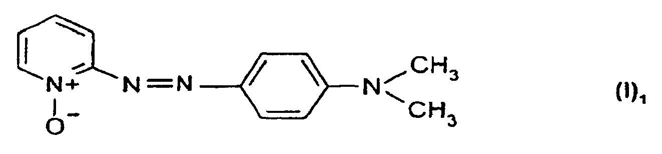

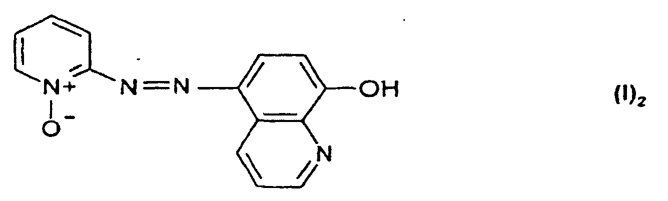

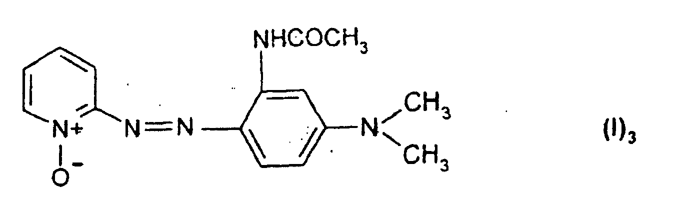

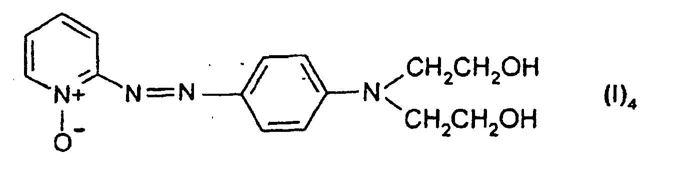

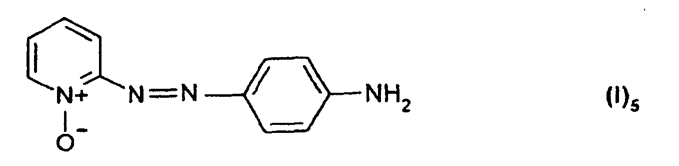

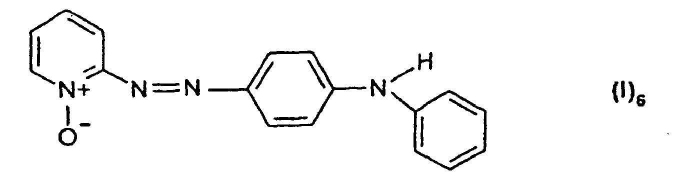

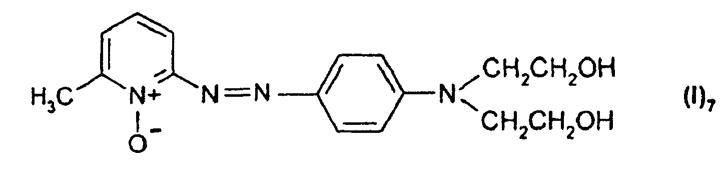

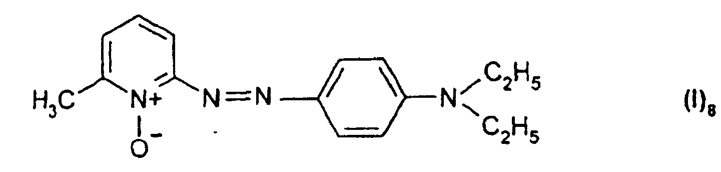

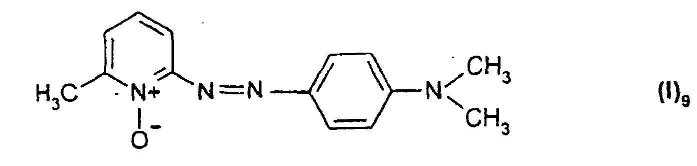

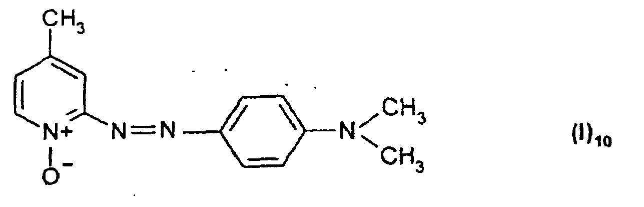

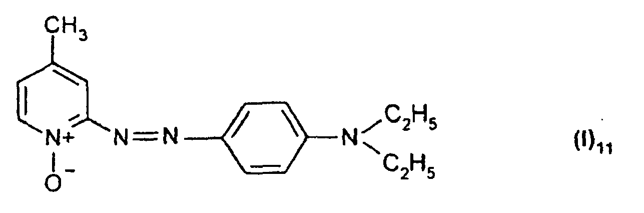

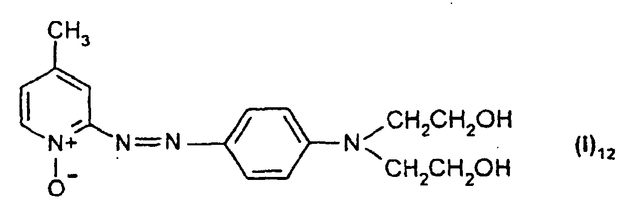

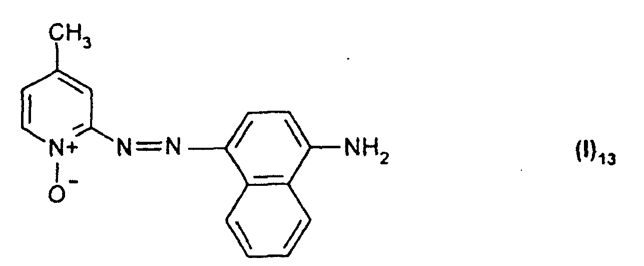

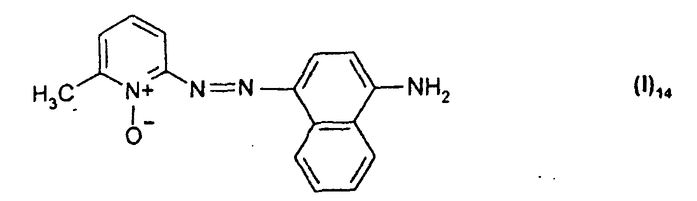

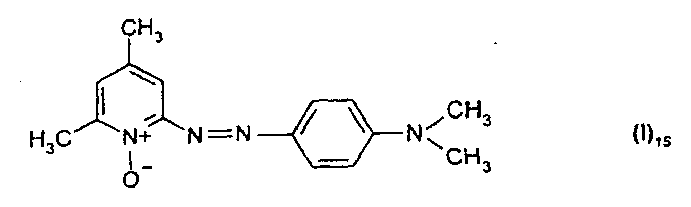

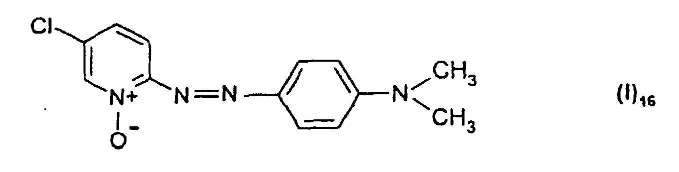

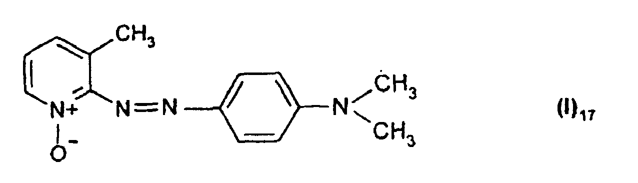

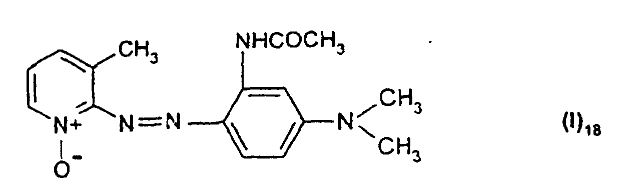

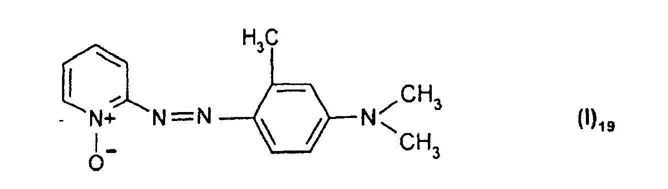

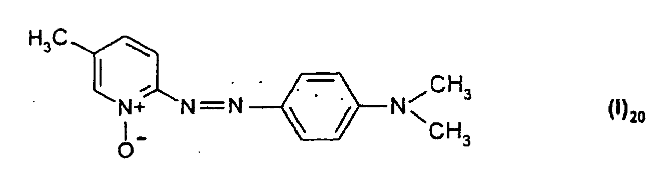

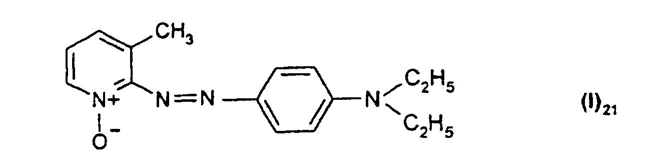

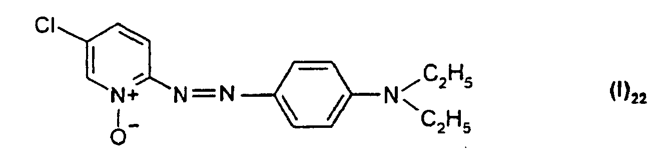

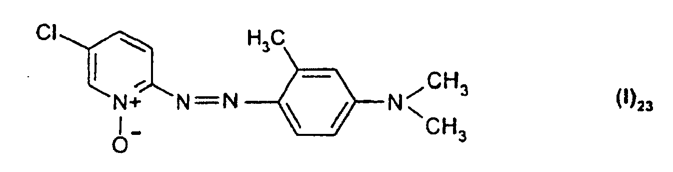

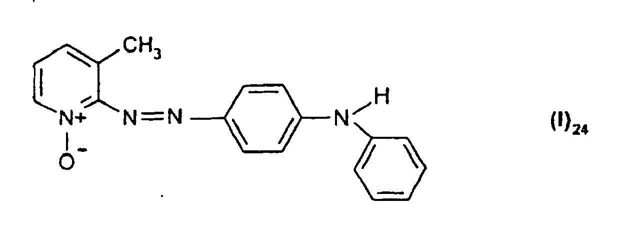

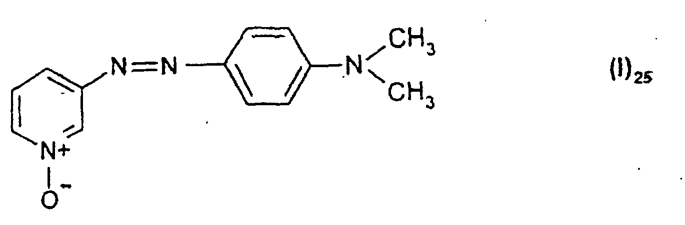

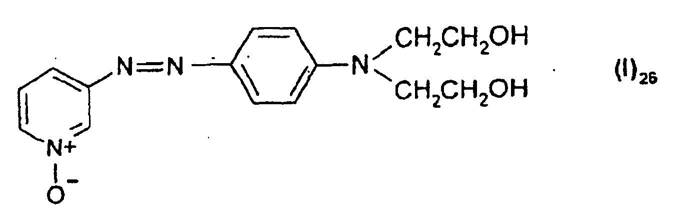

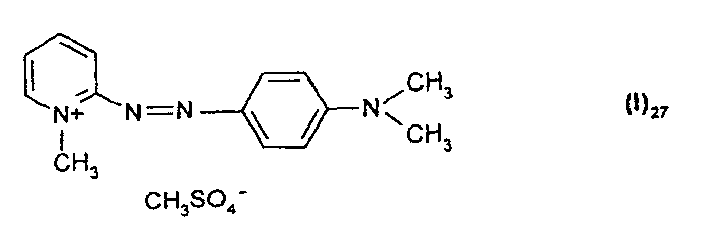

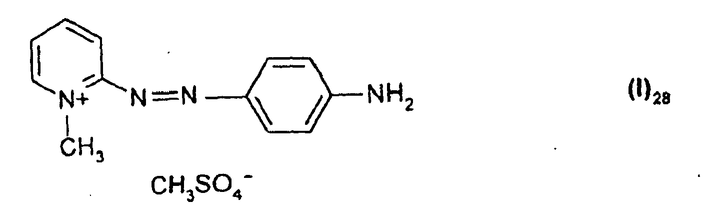

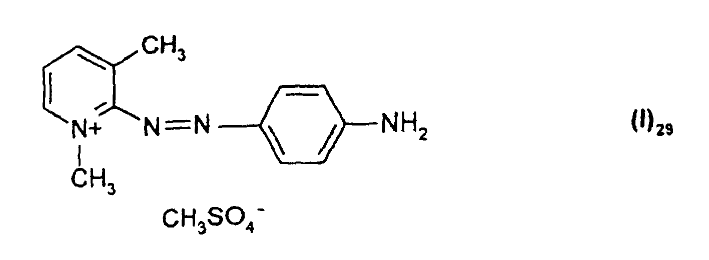

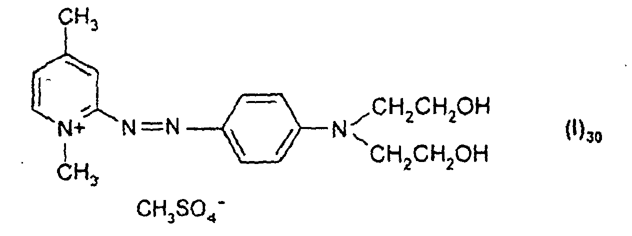

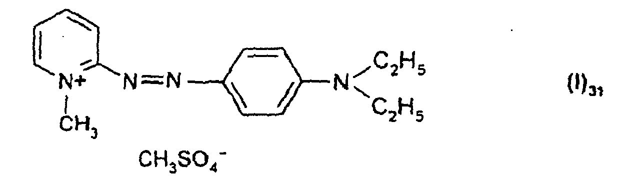

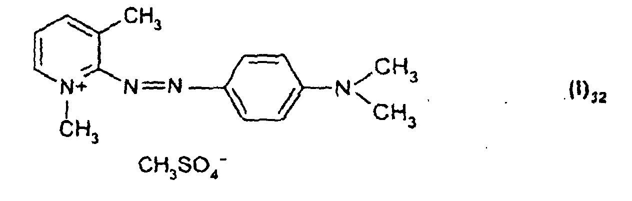

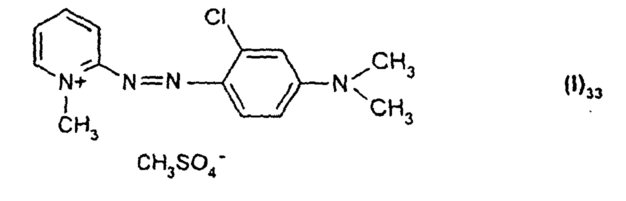

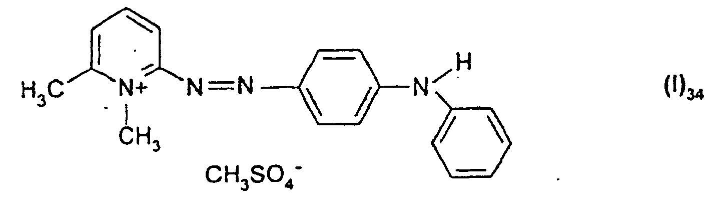

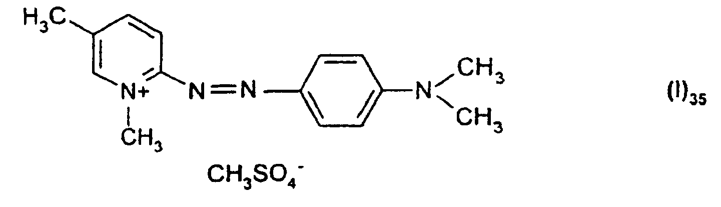

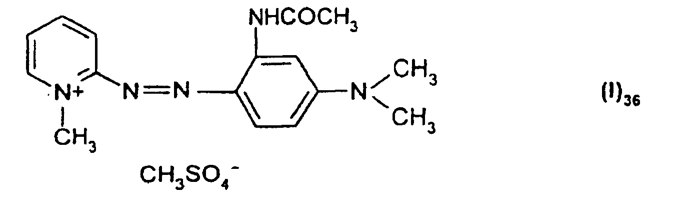

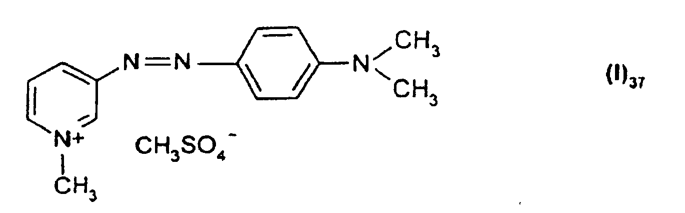

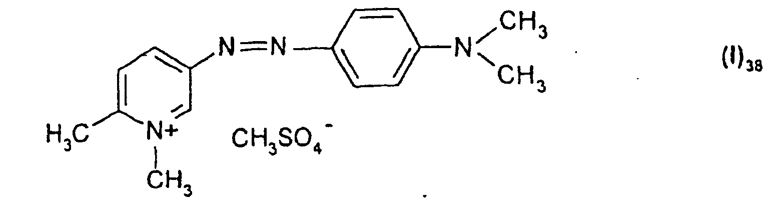

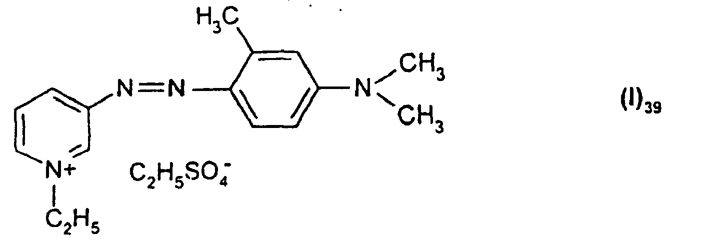

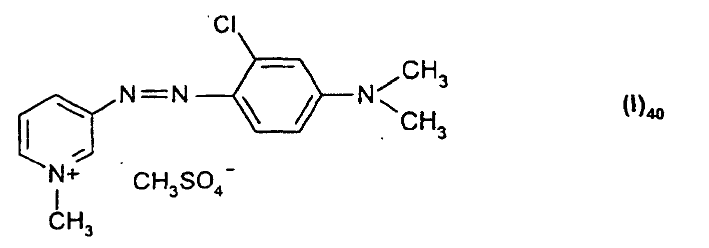

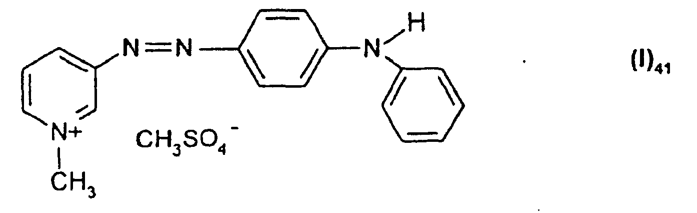

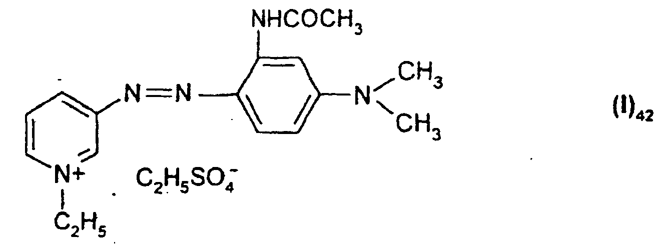

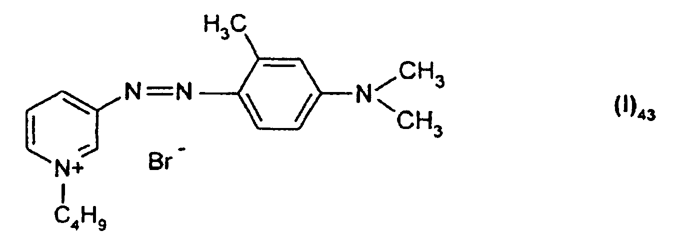

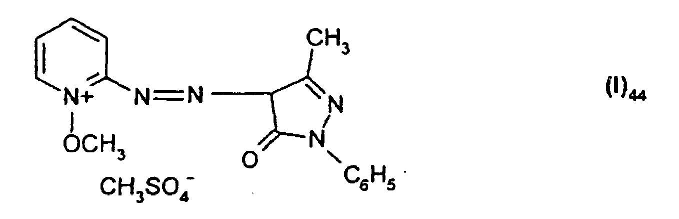

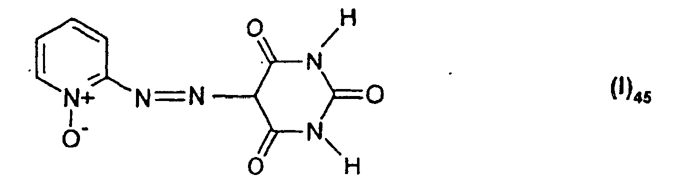

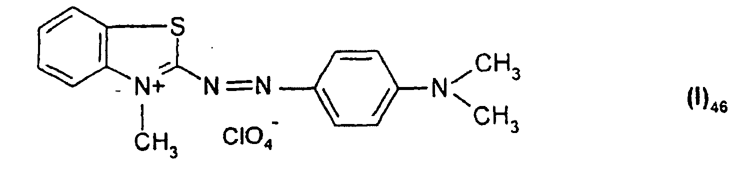

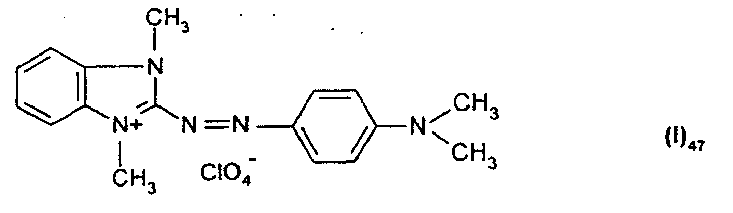

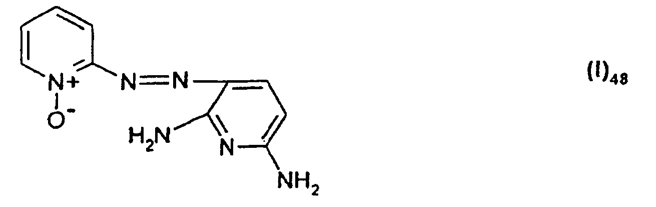

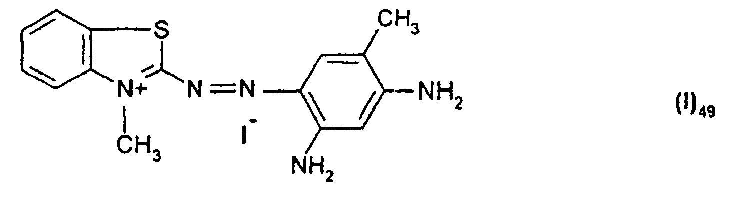

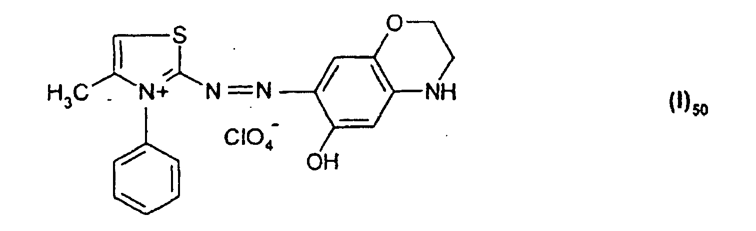

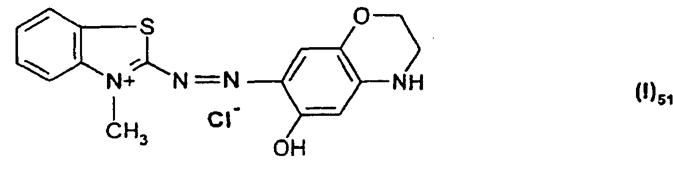

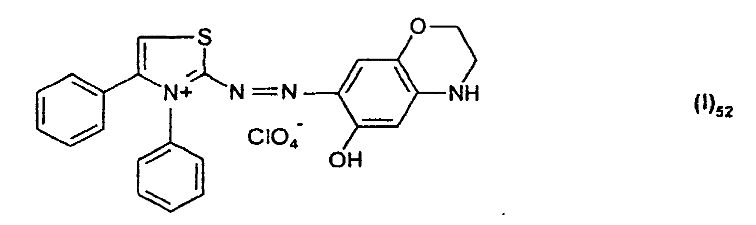

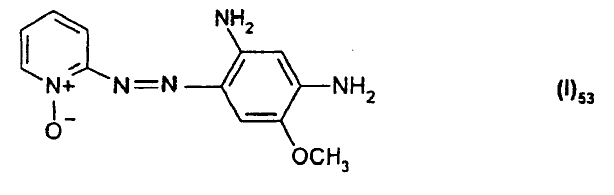

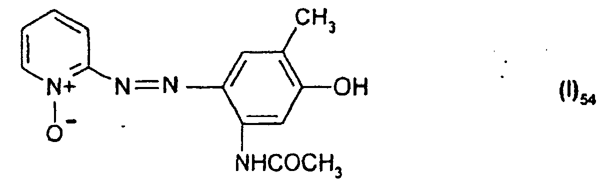

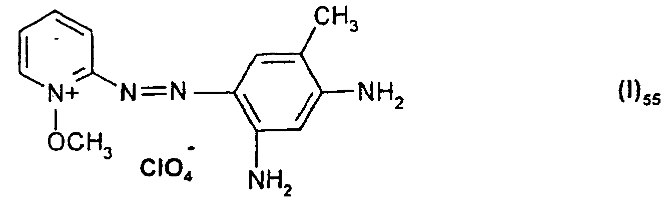

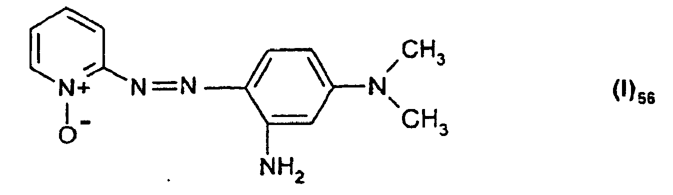

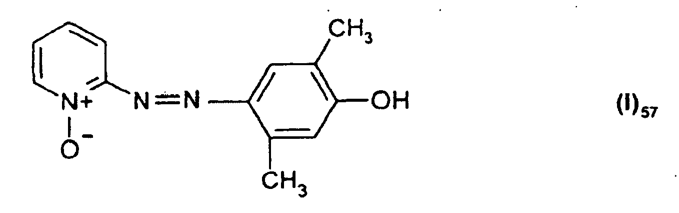

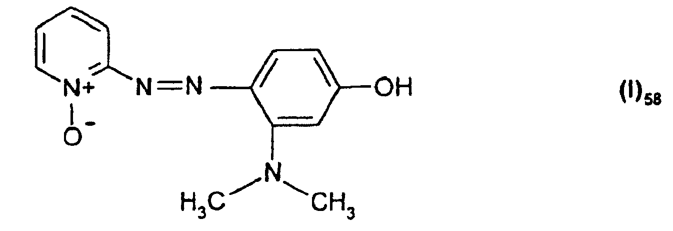

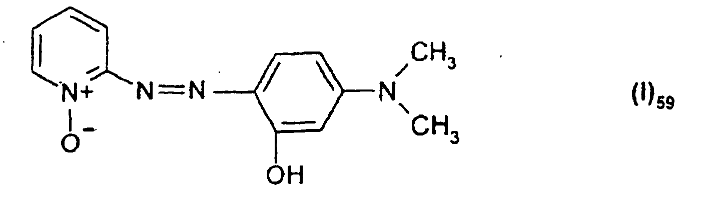

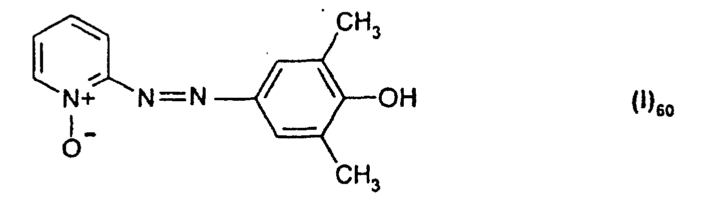

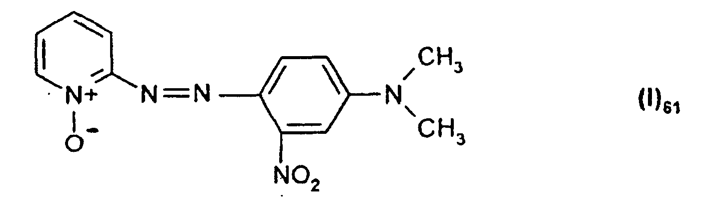

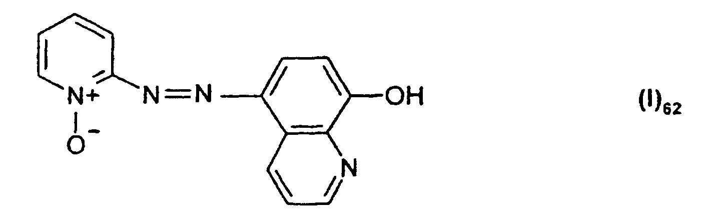

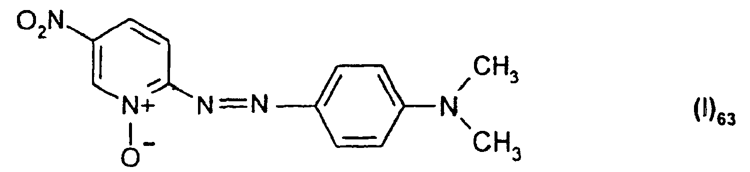

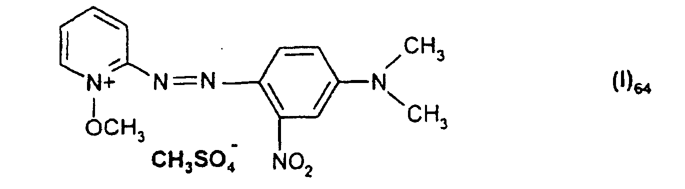

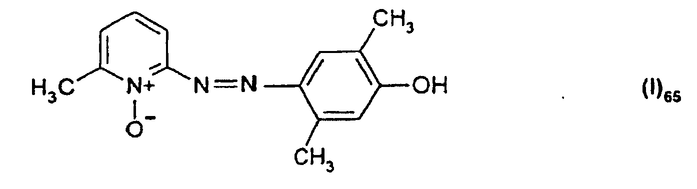

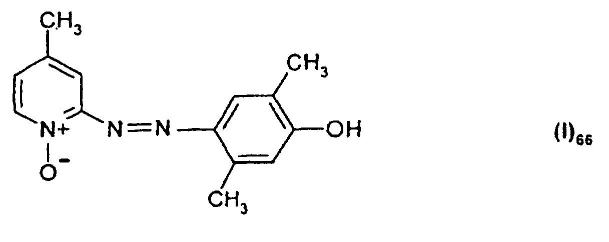

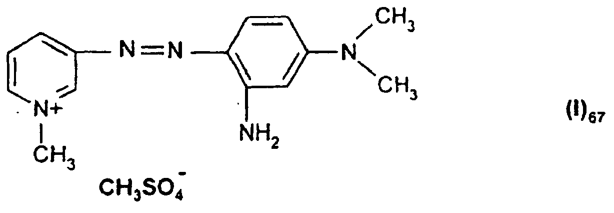

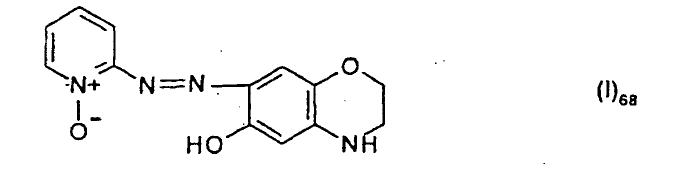

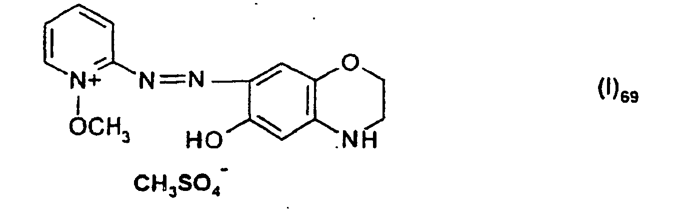

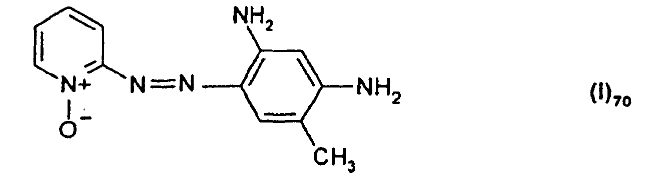

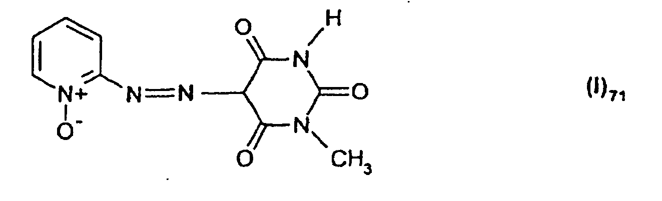

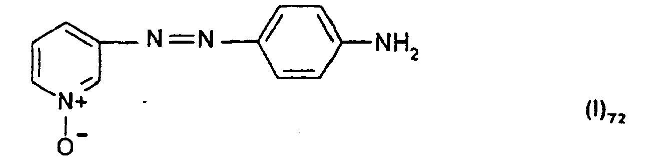

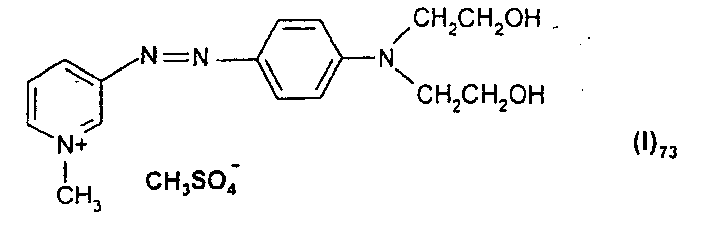

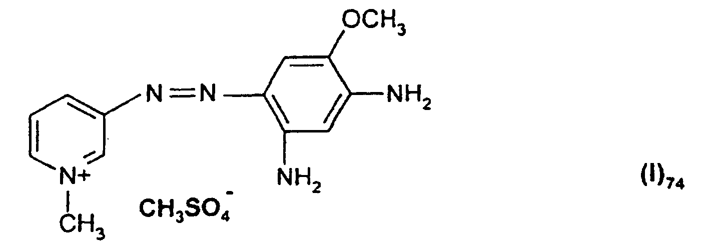

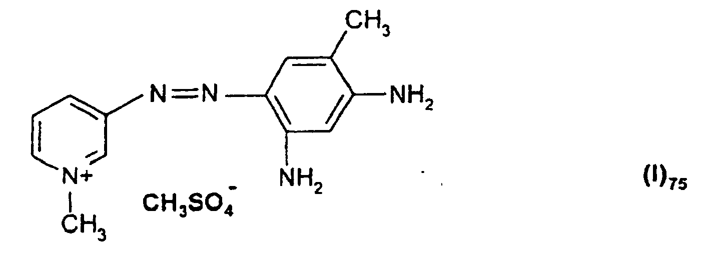

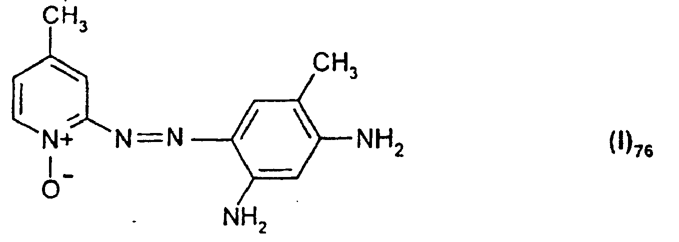

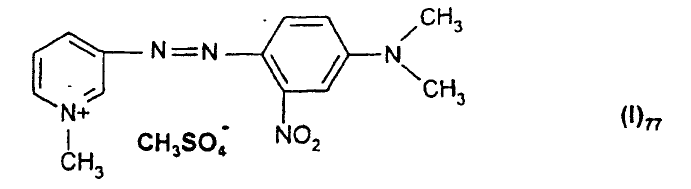

4. Composition according to any one of the preceding claims, **characterized in that** the cationic direct dye(s) of formula (I) represent(s) from 0.001 to 10% by weight relative to the total weight of the composition.

5. Composition according to Claim 4,
**characterized in that** the cationic direct dye(s) of formula (I) represent(s) from 0.005 to 5% by weight relative to the total weight of the composition.

6. Composition according to any one of the preceding claims, **characterized in that** the glycerol and/or the polyol containing at least 4 carbon atoms and/or the C₁-C₈ aliphatic ether of a C₃-C₉ polyol and/or the C₆-C₈ aromatic ether of a C₂-C₉ polyol represent from 0.1 to 40% by weight relative to the total weight of the composition.

7. Composition according to Claim 6,
**characterized in that** the glycerol and/or the polyol containing at least 4 carbon atoms and/or the C₁-C₈ aliphatic ether of a C₃-C₉ polyol and/or the C₆-C₈ aromatic ether of a C₂-C₉ polyol represent from 0.5 to 20% by weight relative to the total weight of the composition.

8. Composition according to any one of the preceding claims, **characterized in that** it also contains additional direct dyes.

9. Composition according to any one of the preceding claims, **characterized in that** the medium which is suitable for keratin fibres (or support) consists of a mixture of water and at least one solvent chosen from glycerol and/or the polyols and/or polyol ethers defined in Claim 1.

10. Composition according to Claim 9,
**characterized in that** the medium also comprises at least one organic solvent.

11. Composition according to any one of the preceding claims, **characterized in that** it has a pH of between 2 and 11 and preferably between 5 and 10.

12. Composition according to any one of the preceding claims, **characterized in that** it is intended for oxidation dyeing and **in that** it contains one or more oxidation bases chosen from paraphenylenediamines, bis(phenyl)alkylenediamines, paraaminophenols, ortho-aminophenols and heterocyclic bases.

13. Composition according to Claim 12,
**characterized in that** the oxidation base(s) represent(s) from 0.0005 to 12% by weight relative to the total weight of the dye composition.

14. Composition according to Claim 13,
**characterized in that** the oxidation base(s) represent(s) from 0.005 to 6% by weight relative to the total weight of the dye composition.

15. Composition according to any one of Claims 12 to 14, **characterized in that** it contains one or more couplers chosen from meta-phenylenediamines, meta-aminophenols, meta-diphenols and heterocyclic couplers.

16. Composition according to Claim 15,
**characterized in that** the coupler(s) represent(s) from 0.0001 to 10% by weight relative to the total weight of the dye composition.

17. Composition according to Claim 16,
**characterized in that** the coupler(s) represent(s) from 0.005 to 5% by weight relative to the total weight of the dye composition.

18. Composition according to any one of the preceding claims, **characterized in that** it is intended for oxidation dyeing or lightening direct dyeing and **in that** it contains at least one oxidizing agent.

19. Process for dyeing keratin fibres, and in particular human keratin fibres such as the hair, **characterized in that** at least one dye composition as defined in any one of Claims 1 to 11 is applied to the fibres, for a period which is sufficient to develop the desired coloration, after which the fibres are rinsed, optionally washed with shampoo, rinsed again and dried.

20. Process for dyeing keratin fibres, and in particular human keratin fibres such as the hair, **characterized in that** at least one dye composition as defined in any one of Claims 1 to 11 is applied to the fibres, for a period which is sufficient to develop the desired coloration, without final rinsing.

21. Process for dyeing keratin fibres, and in particular human keratin fibres such as the hair, **characterized in that** it includes a preliminary step which consists in separately storing, on the one hand, a composition (A1) comprising, in a medium which is suitable for dyeing, at least one cationic direct dye and at least glycerol and/or one polyol and/or polyol ether as defined in the preceding claims and at least one oxidation base, and, on the other hand, a composition (B1) containing, in a medium which is suitable for dyeing, at least one oxidizing agent, and then in mixing them together at the time of use, after which this mixture is applied to the keratin fibres.

22. Process for dyeing keratin fibres, and in particular human keratin fibres such as the hair, **characterized in that** it includes a preliminary step which consists in separately storing, on the one hand, a composition (A2) comprising, in a medium which is suitable for dyeing, at least one cationic direct dye and at least glycerol and/or one polyol and/or polyol ether as defined in the preceding claims, and, on the other hand, a composition (B1) containing, in a medium which is suitable for dyeing, at least one oxidizing agent, and then in mixing them together at the time of use, after which this mixture is applied to the keratin fibres.

23. Multi-compartment device or multi-compartment dyeing kit, **characterized in that** a first compartment contains composition (A1) or (A2) as defined in Claim 21 or 22 and a second compartment contains composition (B1) as defined in Claim 21 or 22.
